(19) Europäisches Patentamt / European Patent Office / Office européen des brevets

(11) EP 2 711 433 B1

(12) **EUROPEAN PATENT SPECIFICATION**

(45) Date of publication and mention of the grant of the patent: **15.02.2017 Bulletin 2017/07**

(21) Application number: **12786619.2**

(22) Date of filing: **16.05.2012**

(51) Int Cl.:
*C12Q 1/68* (2006.01) *A61K 31/47* (2006.01)
*A61P 35/00* (2006.01) *A61P 43/00* (2006.01)
*G01N 33/15* (2006.01) *G01N 33/50* (2006.01)
*C12N 15/09* (2006.01)

(86) International application number: **PCT/JP2012/062509**

(87) International publication number: **WO 2012/157672 (22.11.2012 Gazette 2012/47)**

(54) **METHOD FOR PREDICTING EFFECTIVENESS OF ANGIOGENESIS INHIBITOR**

VERFAHREN ZUR PROGNOSE DER WIRKSAMKEIT EINES ANGIOGENESEHEMMERS

MÉTHODE DE PRÉDICTION DE L'EFFICACITÉ D'UN INHIBITEUR DE L'ANGIOGENÈSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.05.2011 JP 2011110884**

(43) Date of publication of application: **26.03.2014 Bulletin 2014/13**

(73) Proprietor: **Eisai R&D Management Co., Ltd.**
**Tokyo 112-8088 (JP)**

(72) Inventors:
- **SEMBA, Taro** Tsukuba-shi Ibaraki 300-2635 (JP)
- **NARITA, Yusuke** Tsukuba-shi Ibaraki 300-2635 (JP)
- **MINOSHIMA, Yukinori** Tsukuba-shi Ibaraki 300-2635 (JP)
- **YAMAGUCHI, Atsumi** Tsukuba-shi Ibaraki 300-2635 (JP)
- **ADACHI, Yusuke** Tsukuba-shi Ibaraki 300-2635 (JP)
- **YAMADA, Kazuhiko** Tsukuba-shi Ibaraki 300-2635 (JP)
- **MATSUI, Junji** Tsukuba-shi Ibaraki 300-2635 (JP)
- **KADOWAKI, Tadashi** Tsukuba-shi Ibaraki 300-2635 (JP)
- **TAKAHASHI, Kentaro** Tsukuba-shi Ibaraki 300-2635 (JP)
- **FUNAHASHI, Yasuhiro** Tsukuba-shi Ibaraki 300-2635 (JP)

(74) Representative: **Hoffmann Eitle**
**Patent- und Rechtsanwälte PartmbB**
**Arabellastraße 30**
**81925 München (DE)**

(56) References cited:
**EP-A1- 1 925 941** **WO-A1-2007/015569**
**WO-A1-2009/150256** **WO-A2-2012/166899**

- **MATSUI JUNJI. ET AL.: 'Multi-Kinase Inhibitor E7080 Suppresses Lymph Node and Lung Metastases of Human Mammary Breast Tumor MDA-MB-231 via Inhibition of Vascular Endothelial Growth Factor-Receptor (VEGF-R) 2 and VEGF-R3 Kinase' CLINICAL CANCER RESEARCH vol. 14, 2008, pages 5459 - 5465, XP055136717**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 711 433 B1

- TERUHIKO FUJII ET AL.: 'Kekkan Shinsei Sogaiyaku' CLINICAL GASTROENTEROLOGY vol. 19, no. 7, 25 May 2004, pages 220 - 227, XP008172331
- TAKAO YAMORI ET AL.: 'Kokeigan no Saishin Chiryo Chiryo no Aratana Torikumi Yakubutsu Ryoho Kinase Inhibitors' JAPANESE JOURNAL OF CLINICAL MEDICINE vol. 68, 01 June 2010, pages 1059 - 1066, XP008171654
- TOMOYA YOKOTA: 'ASCO Hokoku Shokakigan Ryoiki' GAN BUNSHI HYOTEKI CHIRYO vol. 8, no. 4, 2010, pages 271 - 283, XP008171839
- DANKORT DAVID. ET AL.: 'BrafV600E cooperates with Pten loss to induce metastatic melanoma' NATURE GENETICS vol. 41, 2009, pages 544 - 552, XP055136718
- WANG X. ET AL.: 'KRAS, BRAF, PIK3CA MUTATIONS AND PTEN EXPRESSION IN HUMAN COLORECTAL CANCER- RELATIONSHIP WITH METASTATIC COLORECTAL CANCER' ANN ONCOL vol. 21, no. 6, 2010, page VI64, XP055134306

## Description

### Technical Field

The present invention relates to a novel method for predicting the responsiveness of a subj ect buffering from a cancer to an angiogenesis inhibitor.

### Background Art

Many kinase inhibitors have been developed as anticancer agents. Particularly, a group of substances having an inhibitory activity against a receptor tyrosine kinase such as Vascular Endothelial Growth factor (hereinafter also referred to as "VEGF") receptor have characteristics of inhibiting angiogenesis associated with growth of cancer and draw attention as anew generation of anticancer agents.

However, an anticancer agent effective for all types of cancer has not yet been approved. Particularly, advanced malignant melanoma is highly metastatic and its prognosis is extremely poor. Due to this, it is difficult to develop an anticancer agent for malignant melanoma.

In the meantime, therapy with an anticancer agent generally entrails side effects such as severe nausea and general malaise, Thus, administration of an anticancer agent to a subject, on which the agent is not expected to exert a therapeutic effect, should be avoided. Therefore, it has been desired to develop a biomarker by which a therapeutic effect on a subject can be predicted before an anticancer agent is administered in order to avoid administration of an ineffective medicinal drug and reduce side effects.

Incidentally, 4-(3-chloro-4-(ayclopropylamidocarbonyl)aminophenoxy)-7-methoxy-6-quinolinecarboxamide has been used as a multi-kinase inhibitor having an inhibitory activity against receptor tyrosine kinases such as VEGF receptor, Fibroblast Growth Factor ((hereinafter also referred to as "FGF") receptor, Platelet-Derived Growth Factor (hereinafter also referred to as "poof") receptor, RET kinase and KIT kinase, and exhibits an excellent angiogenesis inhibition effect and an anti-growth effect (Patent Literature 1; Patent Literature 2; Non Patent Literature 1).

Furthermore, B-Raf, a kind of serine/threonine kinase, is known to serve as a cell-proliferation signal, if activated, to activate the MAP kinase pathway important as a cellproliferation signal pathway. In addition, B-Raf has been reported to activate various types of cancer due to its mutation (Non Patent Literature 2).

Furthermore, a cancer repressor gene, PTEN (phosphatase and tensin homolog deleted on chromosome 10), encodes a lipid phosphatase which mainly utilizes PIP3 as a substrate and negatively controls the signal. PTEN has a function of inhibiting activation of Akt kinase, thereby inducing apoptosis to suppress cell-proliferation; however, a mutation and loss of expression of PATEN are known to induce excessive activation of Akt kinase, causing growth of cancer (Non Patent Litetature 3).

Nevertheless, no reports have been made on association of the presence or absence of a mutation of B-Raf and the presence or absence of a mutation or loss of expression of PTEN with the anti-tumor effect of an angogenesis inhibitor.

### Citation List

### Patent Literature

Patent Literature 1: WO02/032872
Patent Literature 2: WO2007/136103


### Non Patent Literature

Non Patent Literature 1: Matsui et al., Clinical Cancer Research, 2008,14 (17), p. 5459-5465.
Non Patent Literature 2: Davies et al., Nature, 2002,417, p. 949-954.
Non Patent Litetature 3: Besson et al., European Journal of Biochemistry, 1999, 263, p. 605-611.

## Summary of Invention

### Technical Problem

The present invention was made in the aforementioned circumstances. A problem to be solved by the invention is finding a method of predicting the responsiveness of a subject suffering from a cancer to an angiogenesis inhibitor, particularly to a VEGF receptor inhibitory, an FGF receptor inhibitor, a RET kinase inhibitor or a KIT kinase inhibitor.

Another problem to be solved by the invention is selecting a subject suffering from a cancer by the above prediction method and treating the subject by administering an angiogenesis inhibitor.

### Solution to Problem

The present inventors made a great effort to solve the aforementioned problems and surprisingly found that simultaneous occurrence of a mutation of B-Raf and a mutation or loss of expression of PTEN correlates with the responsiveness of cancer cells to an angiogenesis inhibitor.

More specifically, the present inventors investigated the responsiveness of melanoma cells to an angiogenesis inhibitor. As a result, the present inventors elucidated that the case where (a1) B-Raf and PTEN are wild type or (a2) B-Raf and PTEN have a mutation or loss of expression exhibits high responsiveness to an angiogenesis inhibitor.

Additionally, the present inventors found that the presence or absence of a mutation or loss of expression in B-Raf and PTEN in melanoma cells correlates with the expression levels of angiopoietin-1 (ANG1) and angiopoietin-2 (ANG2). To describe it more specifically, it was elucidated that, in a case where (b1) the expression levels of ANG1 and ANG2 in a sample are low compared to a control value, (b2) the expression level of ANG2 in a sample is high compared to a control value or (b3) the ratio of expression levels of ANG1 and ANG2 is low compared to a control value, the responsiveness of a subject suffering from a tumor to an angiogenesis inhibitor is high.

Accordingly, by use of the presence or absence of a mutation or loss of expression in B-Raf and PTEN, expression levels of ANG1 and ANG2 or the ratio of expression levels of ANG1 and ANG2 in a sample derived from a subject, as an indicator, the responsiveness of the subject to an angiogenesis inhibitor can be predicted without administration of an angiogenesis inhibitor to the subject.

In addition, the present inventors found that the anti-tumor effect pattern of an angiogenesis inhibitor, which is fluctuated with the presence or absence of a mutation or loss of expression in B-Raf and PTEN in melanoma cells, also correlates with the expression levels of SHC1, IL6, CXCR4, COL4A3, NRP2, MEIS1, ARHGAP22, SCG2, FGF9, PML, FGFR23, FGPR2, FGFR1, FGFR4 and VEGFR1. To describe it more specifically, they elucidated that the responsiveness of a subject suffering from a tumor to an angiogenesis inhibitor is high in a case where (c1) the expression level of SHC1 is low compared to a control value, (c2) the expression level of IL6 is high compared to a control value, (c3) the expression level of CXCR4 is high compared to a control value, (c4) the expression level of COL4A3 is high compared to a control value, (c5) the expression level of NRP2 is low compared to a control value, (c6) the expression level of MEIS1 is high compared to a control value, (c7) the expression level of ARHGAP22 is low compared to the a control value, (c8) the expression level of SCG2 is low compared to a control value, (c9) the expression level of FGF9 is high compared to a control value, (c10) the expression level of PML is low compared to a control value, (c11) the expression level or FGFR2 is high compared to a control value, (c12) the expression level of FGFR2 is high compared to a control value, (c13) the expression level of FGFR1 is high compared to a control value, (c14) the expression level of FGFR4 is high compared to a control value, or (c15) the expression level of VEGFR1 is high compared to a control value.

Specifically. the present invention relates to the following.

(1) A method for predicting the responsiveness of a subject suffering from a tumor to an angiogenesis inhibitor, comprising

(a) detecting the presence or absence of a mutation or loss of expression of B-Raf and the presence or absence of a mutation or loss of expression of PTEN in a sample derived from a tumor tissue of the subject, wherein in the detection step, a case where

(a1) B-Raf is wild type and PTEN is wild type, or

(a2) B-Raf has at least one mutation selected from Table 1 or loss of expression and PTEN has at least one mutation selected from Table 2 or loss of expression is indicative of the high responsiveness of the subject to the angiogenesis inhibitor, wherein the angiogenesis inhibitor is 4-(3-chloro-4-(cyclopropylaminocarbonyl)aminophenoxy)-7-methoxy-6-quinolinecarboxamide or a pharmalogically acceptable salt thereof, and wherein the tumor is melanoma.

(2) The method according to (1), wherein, in the detection step (a), a case where B-Raf is wild type end PTEN is wild type is indicative of the high responsiveness of the subject to the angiogenesis inhibitor.
(3) The method according to (1), wherein, in the detection step (a), a case where B-Raf has at least one mutation selected from Table 1 or loss of expression and PTEN has at least one mutation selected from Table 2 or loss of expression is indicative of the high responsiveness of the subject to the angiogenesis inhibitor
(4) The method according to (1) or (3), wherein the mutation of B-Raf is a V600E mutation in an amino acid sequence or a mutation in a nucleotide sequence corresponding to the mutation.
(5) The method according to (1) or (3), wherein the mutation of PTEN is at least one mutation in a nucleotide sequence selected from the group consisting of A499G, T202C and T335A or at least one mutation in an amino acid sequence selected from the group consisting of T167A, Y68H and L112Q.
(6) The method according to any one of (1) to (5), wherein the angiogenesis inhibitor is a mesylate salt of 4-(3-chloro-4-(cyclopropylaminocarbonyl)aminopbenoxy)-7-methoxy-6-quinolinecarboxamide.
(7) The method according to any one of (1) to (6), wherein the tumor is a tumor having a V600E mutation in B-Raf.
(8) The method according to anyone of (1) to (7). wherein, in the step (a), the high responsiveness of the subject to the angiogenesis inhibitor is predicted; and the method further comprises a step (b) of quantifying expression levels of at least one selected from the group consisting of ANG1 and ANG2 in the sample derived from the tumor tissue of the subject, wherein, in the quantification step, a casr where

(b1) the expression level of ANG1 is low compared to a control value
(b2) the expression level of ANG2 is high compared to a control value, or
(b3) the ratio of the expression levels of ANG1 and ANG2 is low compared to a control value

is indicative of the high responsiveness of the subject to the angiogenesis inhibitor.
(9) The method according to any one of (1) to (7), wherein, in the step (a), the high responsiveness of the subject to the angiogenesis inhibitor is predicted; and the method further comprises a step (c) of quantifying an expression level of at least one selected from the group consisting of SHC1, YL6, CXCR4, COL4A3, NRP2, MEIS1, ARHGAP22, SCG2, FGF99, PML FGFR23, FGFR2, FGFR1, FGFR24 and VEGFR1 in the sample derived from the tumor tissue of the subject wherein, in the quantification step, a case where

(c1) the expression level of SHC1 is low compared to a control value,
(c2) the expression level of NRP2 is low compared to a control value,
(c3) the expression level of ARHGAP22 is low compared to a control value,
(c4) the expression level of SCG2 is low compared to a control value,
(c5) the expression level of PML is low compared to a control value,
(c6) the expression level of IL6 is high compared to a control value,
(c7) the expression level of CXCR4 is high compared to a control value,
(c8) the expression level of COL4A3 is high compared to a control value,
(c9) the expression level of MEIS1 is high compared to a control value,
(c10) the expression level of FGF9 is high compared to a control value,
(c11) the expression level of FGFR3 is high compared to a control value,
(c12) the expression level of FGHR2 is high compared to a control value,
(c13) the expression level of FGFR1 is high compared to a control value,
(c14) the expression level of FGFR4 is high compared to a control value, or
(c15) the expression level of VEGFR1 is high compared to a control value is indicative of the high responsiveness of the subject to the angiogenesis inhibitor.

(10) A method for predicting the responsiveness of a subject suffering from a tumor to an angiogenesis inhibitor, comprising

(b) quantifying expression levels of at least one selected from the group consisting of ANG1 and ANG2 in a sample derived from a tumor tissue of the subject, wherein, in the quantification step, a case where
(b1) the expression level of ANG1 is low compared to a control value
(b2) the expression level of ANG2 is high compared to a control value, or
(b3) the ratio of expressions level of ANG1 and ANG2 is low compared to a control value

is indicative of the high responsiveness of the subject to the angiogenesis inhibitory, wherein the angiogenesis inhibitor is 4-(3-chloro-4-(cyclopropylaminocarbonyl)aminophenoxy)-7-methoxy-6-quinolinecarboxamide or a pharmalogically acceptable salt thereof, and wherein the tumor is melanoma.

(11) The method according to (10), wherein, in the step (b), the high responsiveness of the subject to the angiogenesis inhibitor is predicted, and the method further comprise a step (c) of quantifying an expression level of at least one selected from the group consisting of SHC1, IL6, CXCR4, COL4A3, NRP2, MBIS1, ARHGAP22, SCG2, FGF9, PML, FGFR3, FGFR2, FGFR1, FGFR4 and VEGFR1 in the sample derived from the tumor tissue of the subject, wherein, in the quantification step, a case where

(c1) the expression level of SHC1 is low compared to a control value,
(c2) the expression level of NRP2 is low compared to a control value,
(c3) the expression level of ARHGAP22 is low compared to a control value,
(c4) the expression level of SCG2 is low compared to a control value,
(c5) the expression level of PML is low compared to a control value,
(c6) the expression level of IL6 is high compared to a control value,
(c7) the expression level of CXCR4 is high compared to a control value,
(c8) the expression level of COL4A3 is high compared to a control value,
(c9) the expression level of MEIS1 is high compared to a control value,
(c10) the expression level of FGF9 is high compared to a control value,
(c11) the expression level of FGFR3 is high compared to a control value,
(c12) the expression level of FGFR2 is high compared to a control value,
(c13) the expression level of FGFR1 is high compared to a control value,
(c14) the expression level of FGFR4 is high compared to a control value, or
(c15) the expression level of VBGFR1 is high compared to a control value is indicative of the high responsiveness of the subject to the angiogenesis inhibitor

(12) The method according to any one of (1) to (11), wherein the step (a) to (c) comprise a step of bringing the sample derived from the tumor tissue of the subject into contact with probes of B-Raf and PTEN, Particularly, the probes are preferably a nucleic acid probe, a specific antibody or a combination thereof.
(13) A pharmaceutical composition comprising an angiogenesis inhibitor for treating a subject suffering from melanoma, wherein the subject has been predicted to be highly responsive to the angiogenesis inhibitor by the method according to any one of (1) to (12), wherein the angiogenesis inhibitor is 4-(3-chloro-4-(cyclopropylaminocarbonyl)aminoplmoxy)-7-methoxy-6-quinolinecarboxamide or a pharmacologically acceptable salt thereof, wherein the subject has been predicted to be highly responsive to the angiogenesis inhibitor by the method according to any one of (1) to (14) by a doctor or another medical practitioner who administer the therapy,

As the angiogenesis inhibitor, a mesylate salt of 4-(3-chloro-4-(cyclopropylaminocarbonyl)aminophenoxy)-7-methoxy-6-quinolinecarboxamide is particularly preferable.
(22) Use of a kit comprising probes of B-Raf and PTEN or probes of ANG1 and ANG2 for predicting the responsiveness of a subject suffering from melanoma to an genesis inhibitor, , wherein the responsiveness of the subject suffering from melanoma to the angiogenesis inhibitor is predicted by the method according to any one of (1) to (11) and wherein the angiogenesis inhibitor is 4-(3-chloro-4-(cyclopropylaminocarbonyl)aminophenoxy)-7-methoxy-6-quinolinecarboxamide or a pharmacologically acceptable salt thereof and a mesylate salt of 4-(3-chloro-4-(cyclopropylaminocarbonyl)aminophenoxy)-7-methoxy-6-quinolinearboxamide is particularly preferable,

## Advantageous Effects of Invention

The present invention enables to predict the responsiveness of a subject suffering from a cancer to an angiogenesis inhibitor, and in particular, predict the responsiveness to a VEGF receptor inhibitor, an FGF receptor inhibitor, a RET kinase inhibitor or a KIT kinase inhibitor.
As a result, whether administration of an angiogenesis inhibitor to a subject suffering from a cancer is effective or not is determined, and thereafter the angiogenesis inhibitor can be administered bo the subject Therefore, cancer patients, for which administration of the angiogenesis inhibitor is effective, are selected, and then, the angiogenesis inhibitor can be administered. In this manner, cancer can be treated white reducing the risk of aside effect

## Brief Description of Drawings

Figure 1 is a graph showing the anti-tumor effect of E7090 on each of melanoma cells, which are classified into groups based on the presence or absence of a mutation or loss of expression in BRAF and PTEN. In BRAF, "+"

indicates the presence of a mutation or loss of expression; whereas "-" indicates the absence of a mutation or loss of expression. In PTEN, "+" indicates the presence of a mutation or a loss of expression; wherea "-" indcates the absence of a mutation or loss of expression.

Figure 2 is a graph showing the ratio of pericyte-covered blood vessels in each of the tumors of melanoma cells, which are classified into groups based on the presence or absence of a mutation or loss of expression in BRAF and PTEN. In BRAF, "+" indicates the presence of a mutation or loss of expression; whereas "-" indicates the absence of a mutation or loss of expression. In PTEN, "+" indicates the presence of a mutation or loss of expression; whereas "-" indicates the absence of a mutation or loss of expression.

Figure 3 is a graph showing the correlation between the anti-tumor effect of E7080 on melanoma cells and the ratio of pericyte-covered blood vessels, in the tumor.

Figure 4 is a graph showing (a) the expression level of ANG1 protein and (b) the expression level of ANG1 mRNA in melanoma cells, which are classified into groups based on the presence or absence of a V600E mutation in BRAF The symbol "+" indicates the presence of a mutation or loss of expression; whereas "-" indicates the absence of a mutation or loss of expression.

Figure 5 is a graph showing (a) the expression level of ANG2 protein and (b) the expression level of ANG2 mRNA, in melanoma. cells, which are classified into groups based on the presence or absence of a mutation or loss of expression in PTEN. The symbol "+" indicates the presence of a mutation or loss of expression; whereas "-" indicates the absence of a mutation or loss of expression.

Figure 6 is a graph showing the ratio of expression levels of ANG1 and ANG2 in melanoma cells, which are classified into groups based on the presence or absence of a mutation or loss of expression in BRAF and PTEN, In BRAF, "+" indicates the presence of a mutation or loss of expression; whereas "-" indicates the absence of a mutation or loss of expressions, In PTEN. "+" indicates the presence of a mutation or loss of expression; whereas "-" indicates the absence of a mutation or loss of expression.

Figure 7 is a graph showing the ratio of expression levels of FGFR2 and FGFR3 in melanoma cells, which are classified into groups based on the presence or absence of a mutation or loss of expression in BRAF and PTEN. The term "BRAF wt/PTEn wt" indicates a case where BRAF and PTEN are wild type; "BRAF mu/PTEN mu" indicates a case where BRAF and PTEN have a mutation or loss of expression; and "BRAF mu/PTEN wt" indicates a case where BRAF has a mutation or loss of expression and PTEN is wild type.

**Descriplion of Embodiments**

Embodiments of the present invention will be described below.

Note that literatures and publications of patent application laid-open, patent gazettes and other patent literatures are incorporated in the specification as references.

The present invention relates to a method for predicating the responsiveness of a subject to an angiogenesis inhibitor.

The method of the present invention comprises a step of detecting the presence or absence of a mutation or loss of expression of B-Raf and the presence or absence or a mutation or loss of expression of PTEN in a sample derived from a tumor tissue of a subject, In the detection step, the following case of (a1) or (a2) serves as an indicator that the responsiveness of the subject to an angiogenesis inhibitor is high.

(a1) B-Raf is wild type and PTEN is wild type.

(a2) B-Raf has at least one mutation selected from Table 1 or loss of expression mutation and PTEN has at least one mutation selected from Table 2 or loss of expression mutation.

Furthermore, the method of the present invention comprises a step of quantifying expression levels of ANG1 and ANG2 in a sample derived from a tumor tissue of a subject In the quantification step, these quantification results in the following (b1), (b2) or (b3) serve as an indicator that the responsiveness of the subject to an angiogenesis inhibitor is high.

(b1) the expression level of ANG1 is low compared to a control value.

(b2) the expression level of ANG2 is high compared to a control value.

(b3) the ratio of the expression levels of ANG1 and ANG2 is low compared to a control value,

Furthermore, the present invention comprises a step of quantifying the expression level of at least one selected from the group consisting of SHC1, IL6, CXCR4, COL4A3, NRP2. MEIS1, ARHGAP22, SCG2, FGF9, PML, FGFR3, FGPR2, FGPR1, FGFR4 and VEGFR1 in a sample derived from a tumor tissue of the subject In the quantification, step, if a case corresponds to the following (c1) to (c15), these quantification results serve as an indicator that the responsiveness of the subject to an angioganesis inhibitor is high.

(c1) the expression level of SHC1 is low compared to a control value,
(c2) the expression level of NRP2 is low compared to a control value,
(c3) the expression level of ARHGAP22 is low compared to a control value.
(c4) the expression level of SCG2 is low compared to a control value.
(c5) the expression level of PML is low compared to a control value.
(c6) the expression level of IL6 is high compared to a control value.
(c7) the expression level of CXCR4 is high compared to a control value.
(c8) the expression level of COL4A3 is high compared to a control value.
(c9) the expression level of MEIS1 is high compared to a control value.
(c10) the expression level of FGF9 is high compared to a control value.
(c11) the expression level of FGFR3 is high compared to a control value.
(c12) the expression level of FGFR2 is high compared to a control value.
(c13) the expression level of FGFR1 is high compared to a control value.
(c14) the expression level of FGFR4 is high compared to a control value.
(c15) the expression level of VEGFR1 is high compared to a control value.

Furthermore, the present invention comprises a step of detecting the presence or absence of a mutation or loss of expression in B-Raf and PTEN and the expression levels of FGFR3 or FGFR2 in a sample derived from a tumor tissue of the subject In the detection step, the following case of (d1) or (d2) serves as an indicator that the responsiveness of the subject to an angiogenesis inhibitor is high.

(d1) B-Raf and PTEN each are wild type and FGFR3 or FGFR2 is expressed
(d2) B-Raf has at least one mutation selected from Table 1 or loss of expression, and PTEN has at least one mutation selected from Table 2 or loss of expression, and FGFR3 or FGFR2 is expressed.

Furthermore, the method of the present invention relates to a method for predicting the responsiveness to an angiogenesis inhibitor by use of the above indicators.
More specifically, the method of the present invention comprises a step of detecting the presence or absence of a mutation or loss of expression mutation of B-Raf and PTEN; expression levels of ANG1 and ANG2; or the ratio of expression levels of ANG1 and ANG2, and associating these detection results used as an indicator with the responsiveness to an angiogenesis inhibitor. The method of the present invention also comprises a step of quantifying the expression level of SHC1, IL6, CXCR4, COL4A3, NRP2, MEIS1, ARHGAP22, SCG2, FGF9, PML, FGFR3, FGFR2, FGFR1, FGFR4 or VEGFR1 and associating these detection results used as an indicator with the responsiveness to an angiogenesis inhibitor.
In the present invention, the detection step may comprise a step of determining expression level or the ratio of expression levels or a step of analyzing the determination results obtained; and the quantification step may comprise a step of determining expression level or the ratio of expression levels, or a step of analyzing the determination results obtained.
The above detection results and quantification results obtained by the method of the present invention are provided as information for determining whether or not the subject is highly responsible to an angiogenesis inhibitor. These pieces of information are mainly used by medical practitioner;
When it is determined that the responsiveness to an angiogenesis inhibitor is high by the method of the present invention, it can be expected that the angiogenesis inhibitor effectively works (has an anti-tumor effect). Thus, the method of the present invention can be used as an indicator for a cancer therapy,
**[0036]** The angiogenesis inhibitor, which is a target of the method of the present invention, 4-(3-chloro-4-(cyclopropylaminocarbonyl)aminophenoxy)-7-methoxy-6-quinolinecarboxamide or a pharmalogically acceptable salt thereof.
In the present invention, the "responsiveness" to an angiogenesis inhibitor refers to a nature of cancer cells, the growth of which is suppressed by administration of an angiogenesis inhibitor, used as an indicator for sensitivity to an angiogenesis inhibitor.
The "tumor" herein is melanoma.
The "high responsiveness" of a subject to an angiogenesis inhibitor can refer to a nature of tumor cells, the growth of which is strongly suppressed by administration of the angiogenesis inhibitor, and, for example, means that growth of tumor cells, for example, in terms of growth rate or growth yield of tumor cells, relative to a control value, is 1/2 or less, preferably 1/5 or less and further preferably 1/10 or less; or that the colony forming activity of tumor cells relative to a control value is 1/2 or less, preferably 1/5 or less and further preferably 1/10 or less.
Alternatively. in a clinical scene, the "high responsiveness" can mean that an increase of lesions is suppressed,

for example, within 20% compared to a control value by administration of an angiogenesis inhibitory substance; and preferably means that the sum or the longest diameter of target lesions decreases by 30% or more compared to that before administration, and further preferably means that all target lesions disappear, however, the "high responsiveness" is not limited to these examples.

In the present invention, "a sample derived from a tumor tissue of a subject" refers to a tumor tissue taken from a subject, tumor cells dissociated from a tumor tissue such as circulating tumor cells, or DNA, RNA (for example, mRNA, miRNA, tRNA, rRNA, ncRNA, dsRNA, snRNA, snoRNA), other nucleic acids or proteins derived from tumor cells, or preparations made from these into the forms suitable for carrying out the present invention, The tumor tissue or tumor cells taken from a subject may be a body fluid or blood. Note that a person who takes samples and makes preparations may be same or different from a medical practitioner performing the steps of the present invention.

In the present invention, the "medical practitioner" refers to doctors, dentists, laboratory technicians (including experts for performing testing in testing service provider), nurses and workers of other medical institutions.

In the present invention, the type of tumor, the responsiveness of which to an angiogenesis inhihitor is a target to be predicted or the type of tumor that a subject has, is melanoma, and preferably includes melanoma having a V600E mutation in B-Raf. Whether a tumor is one having a V600E mutation in B-Raf can be checked by a detection method (described later) for a mutation or a loss of expression in B-Raf.

The subject in the present invention includes a subject suffering from melanoma. As long as a subject is suffering from at least melanoma, the subject may be suffering from other diseases.

In the present invention, "B-Raf" (v-raf murine sarcoma viral oncogene homolog B1) (also referred to as "BRAF"), which is a serine/threonine protein kinase belonging to a rat/mil family, refers to the gene (SEQ ID NO: 1) represented by the polynucleotide sequence under GenBank Accession No. NM_004333.4 which is determined from its mRNA, and refers to the protein (SEQ ID NO: 2) under GenBank Accession No. NP_0043242, which is translated from the gene. The protein has a function of controlling the MAP kinase/ERKs signaling pathway.

In the present invention, "PTEN" (phosphatase and tensin homolog deleted on chromosome 10) refers to the gene (SEQ ID NO: 3) represented by the polynucleotide sequence under GenBank Accession No. NM_000314.4 which is determined from its mRNA, and refers to the protein (SEQ ID NO: 4) under GenBank Accession No. NP_0003053, which is translated from the gene,

In the present invention, a "mutation," of B-Raf or PTEN refers to a variation of a single or a plurality of nucleotides in the polynucleotide sequence and/or a single or a plurality of amino acids in the amino acid sequence of B-Raf or PTEN, caused by substitution, deletion, insertion and/or addition. Therefore, if the state in which a substitution, deletion, insertion and/or addition of one or a plurality of nucleotides in the Polynucleotide sequence and/or one or a plurality of amino acids in the amino acid sequence, of B-Raf or PTEN is detected, it is determined that B-Raf or PTEN has a mutation.

In the present invention, a mutation of B-Raf is, e.g., a mutation of the amino acid sequence selected from the mutations shown in the following Table 1 or a mutation of the nucleotide sequence corresponding to the mutation of the amino acid sequence.

[Table 1]

Table 1 Mutation of B-Raf

| Amino acid | | |
|---|---|---|
| D587A<br>D587E | G596R | R444Q<br>R444W |
| D594E<br>D594V<br>D594G | G615E | R462I |
| E586K | I463S | S60SF<br>S605N |
| F468C | I592M<br>I592V | T599_V600insTT<br>T599I |
| F595L<br>F595S | K601del<br>K601E<br>K601N | V471F |
| G464R<br>G464V<br>G464E | L597Q<br>L597V<br>L597S | V600A<br>V600D<br>V600E |

(continued)

| Amino acid | | |
|---|---|---|
| | L597R | V600K<br>V600M<br>V600R<br>V600L |
| G466R<br>G466V | N581S | A145V |
| C469S<br>G469E<br>G469A | R443T | |

In Table 1, the numeric character sandwiched between alphabets indicates the position in the amino acid sequence (SEQ ID NO: 2) of B-Raf; and the alphabet before the numeric character is an amino acid of wild type and the alphabet after the numeric character is an amino acid or mutant

To explain more specifically, a mutation D587A in the amino acid sequence means that, in the amino acid sequence (SEQ ID NO: 2) encoded by the B-Raf gene (SEQ ID NO: 1), aspartic acid at position 587 is mutated to alanine or refers to a mutation of the polynucleotide sequence corresponding to the mutation of the amino acid sequence.

A mutation V600E in the amino acid sequence means that, in the amino acid sequence (SEQ ID NO: 2) encoded by the B-Raf gene (SEQ ID NO: 1), valine at position 600 is mutated to glutamic acid, or refers to a mutation of the corresponding polynucleotide sequence; for example, in "gtg" corresponding to the positions from 1798 to 1800 of the nucleotide sequence, the nucleotide at position 1799 is mutated from thymine to adenine.

A mutation K601del in the amino acid sequence means that, in the amino acid sequence (SEQ ID NO: 2) encoded by the B-Raf gene (SEQ ID NO: 1), lysine at position 601 is deleted or refers to a mutation of the corresponding polynucleotide sequence.

A mutation TS99_V600insTT in the amino acid sequence means that, in the amino acid sequence (SEQ ID NO: 2) encoded by the B-Raf gene (SEQ ID NO: 1), two threonine residues are inserted between 599th threonine and 600th valine or refers to a mutation of the corresponding nucleotide sequence.

In the present invention, the "loss of expression" or "loss of expression mutation," of B-Raf means that B-Raf protein is not expressed by deletion of the B-Raf gene or a mutation of a polynucleotide sequence of the B-Raf gene (including an intron). Therefore, if the detection level of polynucleotide sequence and/or amino acid sequence of B-Raf in a sample derived from a tumor tissue of a subject is statistically significantly low compared to a control value or less than a previously determined cutoff value, or if B-Raf is a detection limit or less, it is determined that the expression of B-Raf is lost.

In the present invention, the "wild type" of B-Raf refers to the state where if the presence or absence of at least one of the mutation sites shown in Table 1 is checked, neither mutation nor loss of expression is detected. Furthermore, B-Raf being "wild type" is referred also to B-Raf "under normal".

In the present invention, the mutation of PTEN is one selected from those shown in the following Table 2.
[Table 2]

Table 2 Mutation of PTEN

| Nucleotide | Amino acid |
|---|---|
| T170G | L57W |
| T202C | Y68H |
| T228G | Y76stop |
| T335A | L112Q |
| C367T | H123Y |
| T370A<br>G371C | C124S |
| G385C<br>G385A | G129R |

(continued)

| Nucleotide | Amino acid |
|---|---|
| G493A | G165R |
| A499G | T167A |
| A499C | T167P |

In Table 2, the numeric character sandwiched between alphabets indicates the position of the polynucleotide sequence (SEQ ID NO: 3) or the amino acid sequence (SEQ ID NO: 4) of PTEN; the alphabet before the numeric character is the nucleotide sequence or amino acid sequence of wild type; and the alphabet after the numeric character is the nucleotide sequence or amino acid sequence of mutant. To explain more specifically, the nucleotide mutation T170G means that, the nucleotide of position 170 in the protein coding region (SEQ ID NO: 3) of PTEN gene is mutated from thymine to guanine. The amino acid mutation L57W means that the 57th leucine in the corresponding amino acid sequence (SEQ ID NO: 4) of the protein is mutated to tryptophan. Y76stop means that the 76th tyrosine codon of the amino acid sequence of PTEN varies to a stop cordon, by which translation is terminated.

In the present invention, the "loss of expression" or "loss of expression mutation" of PTEN means the state where PTEN protein is not expressed by deletion of the PTEN gene or a mutation of a polynucleotide sequence of the PTEN gene (including an intron). Therefore, if the detection level of the Polynucleotide sequence and/or amino acid sequence of PTEN in a sample derived from a tumor tissue of a subject is statistically significantly low compared to a control value or less than a previously determined cutoff value, or if PTEN is a detection limit or less, it is determined that the expression of PTEN is lost.

In the present invention, the "wild type" of PATEN refers to the state where if the presence or absence of at least one of the mutation sites shown in Table 2 is checked, neither mutation nor loss of expression is detected. Furthermore, PTEN being "wild type" is referred also to PTEN "under normal".

In the present invention, "ANG1" and "ANG2", which are angiopoietin-1 and angiopoietin-2, respectively, refer to the genes (ANG1; SEQ ID NO: 45, ANG2: SEQ ID NO: 47) represented by the polynucleotide sequences under GenBank Accession No. NM_001146.3, and NM_00111888.1, which are determined from their mRNA, respectively, and refer to the proteins (ANG1: SEQ ID NO: 46, GenBank Accession No. NP_001137.2 and ANG2: SEQ ID NO: 48, GenBank Accession No. NP-001112360.1), which are translated from the genes, respectively

In the present invention, "SHC1" (src homology2 domain containing transforming protein 1) refers to the gene ((SEQ ID NO: 5) represented by the polynucleotide sequence under GenBank Accession No. NM_003029.4, which is determined from its mRNA, and refers to the protein (SEQ ID NO: 6) under GenBank Accession No. NP_003020.2, which is translated from the gene. The protein has an apoptosis-associated function.

In the present invention, "IL6" (interleukin 6), which is a cytokine playing an important role in hemogenesis and inflammation reactions, refers to the gene (SEQ ID NO: 7) represented by the polynucleotide sequence under GenBank Accession No. NM_005600.3, which is determined from its mRNA, and refers to the protein (SEQ ID NO: 8) under GenBank Accession No. NP_000591.1, which is translated from the gene. The protein has a function of controlling the JAK/STAT signaling pathway and the MAP kinase/ERKs signaling pathway.

In the present invention, "CXCR4" (CXC chemokine receptor 4, (also referred to as fusin)), which is an $\alpha$-chemokine receptor specific to stroma-derived factor 1, refers to the gene (SEQ ID NO: 9) represented by the polynucleotide sequence under GenBank Accession No. NM_001008540.1, which is determined from its mRNA, and refers to the protein (SEQ ID NO: 10) under GenBank Accession No. NP_001008540.1, which is translated from the gene. The protein has a function of enhancing cell migration.

In the present invention, "COL4A3" (collagen, type IV, alpha 3), which is a component constituting extracellular matrix, refers to the gene (SEQ ID NO: 11) represented by the polynucleotide sequence under GenBank Accession No. NM_000091.4, which is determined from its mRNA, and refers to the protein (SEQ 11a NO: 12) under GenBank Accession No. NP_0000822, which is translated from the gene. The protein has a function of forming cytoskelton.

In the present invention, "NRP2" (neuropilin-2), which is a transmembrane receptor protein, refers to the gene (SEQ ID NO: 13) represented by the polynucleotide sequence under GenBank Accession No. NM_003872.2, which is determined from its mRNA, and refers to the protein (SEQ ID NO: 14) under GenBank Accession No. NP_003863.2, which is translated from the gene. The protein has a function of enhancing angiogenesis in a development stage and a tumorigenesis stage.

In the present invention, "MEISI" (Meis homeobox 1), which is one of HOX genes, refers to the gene (SEQ ID NO: 15) represented by the polynucleotide sequence under GenBank Accession No. NM_002398.2, which is determined from its mRNA, and refers to the protein (SEQ ID NO: 16) under GenBank Accession No. NP_002389.1, which is translated from the gene. The protein has a function of controlling induced differentiation.

In the present invention, "ARHGAP22" (Rho GTPase activating protein 22), which is a molecule involved in intracellular signal transmission, refers to the gene (SEQ ID NO: 17) represented by the polynucleotide sequence under GenBank Accession No. NM_021226.2, which is determined from its mRNA, and refers to the protein (SEQ ID NO: 18) under GenBank Accession No. NP_067049.2, which is translated from the gene. The protein has a function of controlling remodeling of cytoskelton,

In the present invention, "SCG2" (secretogranin 2) refers to the gene (SEQ ID NO: 49) represented by the polynucleotide sequence under GenBank Accession No. NM_003469.4, which is determined from its mRNA, and refers to the protein (SEQ ID NO: 50) under GenBank Accession No. NP_003460.2, which is translated from the gene. The protein is a secretory protein having a function of enhancing cell migration.

In the present invention, "FGF9" (fibroblast growth factor 9), which is a secretory protein playing an important role in cell differentiation and functional maintenance, refers to the gene (SEQ ID NO: 51) represented by the polynucleotide sequence under GenBank Accession No. NM_002010.2, which is determined from its mRNA, and refers to the protein (SEQ ID NO: 52) under GenBank Accession No. NP_002001.1, which is translated from the gene. The protein has a function of interacting with FGFR3 (described later).

In the present invention, "PML" (promyelocytic leukemia), which is a type of transcription factor, refers to the gene (SEQ ID NO: 53) represented by the polynucleotide sequence under GenBank Accession No. NM_002675.3, which is determined from its mRNA, and refers to the protein (SEQ ID NO: 54) under GenBank Accession No. NP_002666.1, which is translated from the gene. The protein has a function of controlling cell-proliferation as a tumor suppressor.

In the present invention, "FGFR3" (fibroblast growth factor receptor 3), which is a protein having a function of enhancing cell-proliferation and differentiation, refers to the gene (SEQ ID NO: 55) represented by the polynucleotide sequence under GenBank Accession No. NM_000142.3, which is determined from its mRNA, and refers to the protein (SEQ ID NO: 56) under GenBank Accession No, NP_000133.1, which is translated from the gene. FGFR3 is known to have two isoforms, i.e., FGFR3b and FGFR3c.

In the present invention, "FGFR2" (fibroblast growth factor receptor 2), which is a protein having a function of enhancing cell-proliferation and differentiation, refers to the gene (SEQ ID NO: 57) represented by the polynucleotide sequence under GenBank Accession No. NM_001144918.1, which is determined from its mRNA, and refers to the protein (SEQ ID NO: 58) under GenBank Accession No. NP_001138390.1, which is translated from the gene.

In the present invention, "FGFR1" (fibroblast growth factor receptor 1), which is a protein having a function of enhancing cell-proliferation and differentiation, refers to the gene (SEQ ID NO: 59) represented by the polynucleotide sequence under GenBank Accession No. NM_001174063.1, which is determined from its mRNA, and refers to the protein (SEQ ID NO: 60) under GenBank Accession No. NP_001167534.1, which is translated from the gene.

In the present invention, "FGFR4" (fibroblast growth factor receptor 4) is a protein having a function of enhancing cell-proliferation and differentiation, refers to the gene (SEQ ID NO: 61) represented by the polynucleotide sequence under GenBank Accession No. NM_002011.3, which is determined from its mRNA, and refers to the protein (SEQ ID NO: 62) under GenBank Accession No. NP_002002.3, which is translated from the gene.

In the present invention, "VEGFR1" (vascular endothelial growth factor receptor 1), which is a protein having a function of enhancing cell-proliferation and differentiation and angiogenesis, refers to the gene (SEQ ID NO: 63) represented by the polynucleotide sequence under GenBank Accession No. NM_001159920.1, which is determined from its mRNA, and refers to the protein (SEQ ID NO: 64) under GenBank Accession No. NP_001153392.1, which is translated from the gene.

In the present imvention, the "inhibitor" refers to a substance having an inhibitory activity against the function of a target molecule such as a compound, an antibody, an antisense oligonucleotide ("Antisense Drug Technology: Principles, Strategies, and Applications (Second Edition)", CRC Press, 2007), an RNAi oligonucleotide ("RNA Methodologies (Third Edition)", Elsevier, 2005, Chapter 24), a peptide nucleic acid (Kaihatsu et al., Chemistry & Biology, 2004,11 (6), p.749-758) and a peptidic antagonist (Ladner et al., Drug Discovery Today, 2004,9, p.525-529).

In the present invention, the "angiogenesis inhibitor" refers to 4-(3-chloro-4-(cyclopropylaminocarbonyl)aminophenoxy)-7-methoxy-6-quinolinecarboxamide or a pharmalogically acceptable salt thereof.

If the angiogenesis inhibitor to be used in the present invention is a compound, it may form pharmacologically acceptable salts with acids or bases. The angiogenesis inhibitor of the present invention includes these pharmacologically acceptable salts. Examples of the salts with acids include, but not limited to, inorganic acid salts such as a hydrochloride, a hydrobromide, a sulfide and a phosphate; and organic acid salts such as formic acid, acetic acid, lactic acid, succinic acid, fumaric acid, maleic acid, malic acid, citric acid, tartaric acid, tosic acid, stearic acid, benzoic acid, mesyl acid, benzene sulfonic acid, p-toluene sulfonic acid and trifluoroacetic acid. Furthermore, examples of the salts with bases include, but not limited to, alkali metal salt such as a sodium salt and a potassium salt; alkaline earth metal salts such as a calcium salt and a magnesium salt, organic base salts such as trimethylamine, triethylamine, pyridine, picoline, dicyclohexyl amine, N,N'-dibenzylethylenediamine, arginine, and lysine; and ammonium salts.

Furthermore, if the angiogenesis inhibitor to be used in the present invention is a compound, which has solvates

and optical isomers, these solvates and optical isomers are included As the solvates, e.g., hydrates and nonhydrates and preferably hydrates can be mentioned, but are not limited to these. Examples of solvents include, but not limited to, water, alcohol (for example, methanol, ethanol, n-propanol) and dimethylformamide.

Furthermore, in the present invention, if the angiogenesis inhibitor is a compound, the compound may be a crystal or amorphous. Furthermore, if there are crystal polymorphisms, a crystal form of any one of them and a mixture thereof may be used.

Furthermore, the angiogenesis inhibitor of the present invention includes an angiogenesis inhibitor, which is metabolized in a living body by oxidation, reduction, hydrolysis and/or conjugation, Furthermore, the angiogenesis inhibitor of the present invention also includes a compound, which is metabolized in a living body by oxidation, reduction, or hydrolysis to produce an angiogenesis inhibitor.

4-(3 -Chloro-4-(cyclopropylaminocarbonyl)aminophenoxy)-7-methoxy-6-quinolinecarboxamide (Formula (IV))

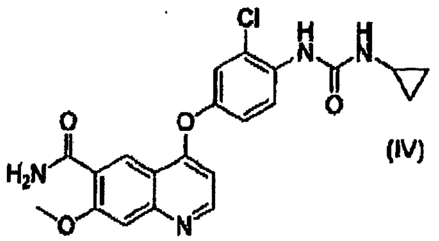

(IV)

The compound can be produced by the method described in WO02/032872. The form of the compound is preferably a but not limited to this. The compound in the form of a mesylate is referred also to as "E7080". The compound is known to have an inhibitory activity against a receptor tyrosine kinase such as VEGF receptor, FGF receptor, RET kinase and KIT kinase (WO2007/136103, Matsui et al., Clinical Cancer Research, 2008,14 (17), p.5459-5465).

Examples of the angiogenesis inhibitor of the present invention include 4-(3-chloro-4-(cyclopropylaminocarbonyl)aminophenoxy)7-methoxy-6-quinolinecarboxamide, or a pharmacologically acceptable salt thereof. As a pharmacologically acceptable salt of 4-(3-chloro-4-(cyclopropylaminocarbonyl)aminophenoxy)-7-methoxy-6-quinolinecarboxamide, preferably mesylate or 4-(3-chloxo-4-(cyclopropylaminocarbonyl)aminophenoxy)-7-methoxy-6-quinolinecarboxamide is mentioned.

A method for determining the presence or absence of a mutation or loss of expression in B-Raf and PTEN in a sample derived from a tumor tissue of a subject will be described below.

A tumor tissue can be obtained by excising out from a subject, for example, by a surgical procedure (for example, biopsy). The size of the tumor tissue taken from a subject is not limited as long as a mutation or loss of expression of B-Raf and PTEN in the tumor tissue can be determined For example, in the case of solid cancer, the size of the tumor tissue taken by biopsy (for example, 2 to 3 mm) is acceptable, and the size of a tissue piece cut by scalpel is acceptable. The size is not limited.

Furthermore, the tumor tissue may be specific cells further excised from the tissue piece taken out, by e.g., a method such as a laser capture micro-dissection method (Murray et al. (Ed.), "Laser Capture Microdissection: Methods and Protocols", Humana Press, 2004).

Furthermore, blood is taken from a subject. Cancer cells circulating through the peripheral blood are isolated by the method of e.g., Kitago et al. From the cancer cell, a mutation or loss of expression of B-Raf and PTEN can be detected (Kitago et al., Clinical Chemistry, 2009, 55 (4), p.757-764).

Furthermore, by use of a highly sensitivity nucleic acid detection method such as a gap-ligase chain reaction (GLCR) method, a mutation or loss of expression of B-Raf and PTEN can be directly detected from DNA circulating through the blood (Chuang et al., Head and Neck, 2010,32, p.229-234).

A mutation or loss of expression of B-Raf and PTEN can be detected by a conventional method such as a method of determining a nucleic acid sequence, a method using a nucleic acid or a specific antibody as a probe and a method using mass spectrometry; however a method of bringing a sample derived from a tumor tissue of a subject into contact with a probe is preferable. As a probe for detecting a mutation or loss of expression of B-Raf and PTEN, a nucleic acid probe or specific antibody to B-Raf or PTEN is mentioned. The "bringing into contact writh" means that a sample derived from a tumor tissue of a subject and a probe are allowed to be present under conditions at which the sample derived from a tumor tissue of a subject and the probe can react with each other, for example, by mixing a sample and a probe and hybridizing a sample with a probe, although the method is not united to these.

In detecting a mutation and loss of expression by determining a nucleotide sequence, the mutation or loss of expression of B-Raf and PTEN can be detected by subjecting a high-molecular DNA product (referred also to as an extracted high-molecular DNA), which is extracted from a sample or a product obtained by amplifying it by a polymerase chain reaction (PCR), to a direct nucleotide sequence determination method, Southern blot method, Northern blot method,

a PCR-strand conformation polymorphism (PCR-SSCP) method, an allelic gene specific oligonucleotide probe (ASO) method, a direct gel assay method, Amplification Refractory Mutation System (ARMS) method, a dot blot analysis method using a mutation specific oligomer or an analogous method thereof. Furthermore, the mutation or loss of expression of B-Raf and PTEN can be detected also by a next generation sequencer such as Applied Biosystems 3730 DNA Analyzer (Applied Biosystems), Roche 454 Genome Sequencer FLX System (Roche), Genome Analyzer II (Illumina), Applied Biosystems SOLiD System (Applied Biosystems) and HeliScope Single Molecule Sequencer (Helicos) ("Current Protocols in Molecular Biology", John Wiley & Sons, 2010, Chapter 7). As the nucleic acid probe to be used for detecting the mutation or loss of expression of B-Raf, for example, primers (SEQ IDNOs: 39 to 44) used in Example 1 are mentioned but not limited to these. As the nucleic acid probe to be used for detecting a mutation or loss of expression of PTEN, for example, primers (SEQ ID NOs: 19 to 38) used in Example 1 are mentioned but not limited to these.

In detecting a mutation and loss of expression by use of the Sanger method, genomic DNA is extracted from a sample derived from a subject in accordance with a conventional method and the obtained genomic DNA is subjected to PCR to amplify exon regions of B-Raf and PTEN. The amplified DNA is subjected to 1% agarose gel electrophoresis. After it is confirmed that the amplified DNA has a desired length, the PCR product is recovered from the gel and purified. The purified product is sequenced by a sequencer. In this manner, information such as gene mutation can be obtained.

The case where detection of a mutation and loss of expression is performed by a next generation sequencer will be described below.

For example, when Genome Analyzer II manufactured by Illumina is used as a next generation sequencer, RNA is extracted from a sample derived from a subject in accordance with a conventional method and cDNA is prepared based on the extracted RNA. The extracted RNA can be quantified by a technique such as Northern blot analysis, DNA microarray, RT-PCR and quantitative PCR. As a preferable quantitative method for mRNA, DNA microarray and quantitative PCR are mentioned; however, the quantitative method is not limited to the above methods.

The cDNA prepared is cut into fragments of about 200 bp suitable for analysis by a next generation sequencer and an adaptor sequence is added to prepare a cDNA library. The library prepared is allowed to bind onto a flow cell via the adaptor sequence to form a cluster. To the cluster a sequence primer is added and a step of detecting fluorescence is repeatedly performed. In this manner, acquisition of data and analysis for a single base extension are performed to obtain information such as gene mutation.

When a mutation and loss of expression are detected by using a specific antibody as a probe, if a partial peptide having the amino acid sequence of a wild type and a partial peptide having the amino acid sequence of a mutant with respect to each of the mutation sites of B-Raf and PETN, are respectively used as antigens, antibodies specific to the mutation sites can be prepared by a conventional method. When a loss of expression is detected, if a partial peptide having a wild-type amino acid sequence and a partial peptide having the amino acid sequence leading to loss of expression with respect to each of loss of expression sites of B-Raf and PETN, are respectively used as antigens, antibodies specific to the loss of expression sites can be prepared by a conventional method. In preparing an antibody specific to a loss of expression site of PTEN, the loss of expression site can be detected if no detection is made by the antibody recognizing a wild type. When a mutation or loss of expression is detected, mutation-site specific antibodies may be used alone or in combination of two or more types.

When a mutation and loss of expression are detected by mass spectrometry, detection can be made, for example, by MassARRAY system manufactured by Sequenom in accordance with the method of Gabriel et al., (Gabriel et al., "Current Protocols in Human Genomics", John Wiley & Sons, 2009, Unit 2.12).

A mutation or loss of expression of B-Raf and PTEN can be detected by any one of the aforementioned methods or in combination of them.

In the above detection, if the result that (a1) B-Raf is wild type and PTEN is wild type, or (a2) B-Raf has at least one mutation selected from Table 1 or loss of expression and PTEN has at least one mutation selected from Table 2 or loss of expression, is obtained, the result serves as an indicator that the responsiveness to the angiogenesis inhibitor in the subject is high. The forms of B-Raf and PTEN, which serve as an indicator that the responsiveness of the subject to the angiogenesis inhibitor is high, are shown in Table 3.

[Table 3]

| | B-Raf | PTEN | Responsiveness |
|---|---|---|---|
| a1 | Wild type | Wild type | High |
| a2 | At least one mutation selected from Table 1 or loss of expression is present | At least one mutation selected from Table 2 or loss of expression is present | High |

In (a2) of Table 2, the forms of B-Raf and PTEN, which serve as an indicator that the responsiveness of the subject to the angiogenesis inhibitor is particularly high, are obtained when the result that B-Raf has a V600E mutation

and PTEN has a T167A, Y68H or L112Q mutations, is obtained.

A method for quantifying the expression levels of ANG1 or ANG2 in a sample derived from a tumor tissue of a a subject will be described below.

A sample derived from a tumor tissue of a subject is taken by the aforementioned method.

The expression levels of ANG1 or ANG2 in the sample can be obtained by quantifying the amount of mRNA or protein by a conventional method.

In quantifying the amount of mRNA, a conventional technique such as Northern blot analysis, DNA microarray, RT-PCR, and/or quantitative PCR can be used; however, DNA microarray or quantitative PCR are preferably used.

As the probe for use in quantifying the expression levels of ANG1 or ANG2, a nucleic acid probe or antibody against ANG1 or ANG2 is mentioned. The nucleic acid probe can be purchased, for example, through ASSAYS-ON-DEMAND of Applied Biosystems (assay IDs of ANG1 and ANG2 are Hs 00181613 and Hs 00169867, respectively). The expression levels may be quantified in accordance with the manual attached to the probes. Alternatively, the nucleic acid probe can be appropriately set and prepared based on the nucleotide sequence of ANG1 or ANG2 by use of Primer Express of Perkin-Elmer Applied Biosystems or a software equivalent to it

A plurality of test substances are compared by correcting quantitative value based on mRNA level of a house keeping gene (transcription amount is not so much fluctuated), preferably β-actin of each test subject,. Note that when mRNA is used for RT-PCR, a primer is used in detecting with a fluorescent dye, SYBR Green (intercalator); whereas, in a detection method using a Master mix, not only a primer but also a probe is required. Either one of them can be designed by use of a software. When a probe is used, a commercially available probe designed by Applied Biosystems may be used.

Furthermore, the expression level of a protein can be determined by a commercially available ELISA kit or by Western blotting, an antibody array, mass spectrometry or immunohistostaining. A specific antibody to ANG1 or ANG2 can be prepared also by the method described in the above paragraph of the angiogenesis inhibitor. When the serum or the plasma is used as a sample, quantification using ELISA and a multiplex beads technique can be used.

The expression level of ANG1 or ANG2 can be quantified by any one of the aforementioned methods or in combination of them.

In the present invention, to quantify the expression level of ANG1 or ANG2, a sample derived from a tumor tissue of a subject is preferably brought into contact with a probe. The meaning of "brought into contact with" is the same as defined above.

In the aforementioned detection, if the result that (b1) the expression level of ANG1 is low compared to control value; (b2) the expression level of ANG2 is high compared to a control value; or (b3) the ratio of the expression levels of ANG1 and ANG2 is low compared to a control value is obtained, the result serves as an indicator that the responsiveness of the subject to an angiogenesis inhibitor is high. The forms of the expression levels of ANG1 and ANG2, which serve as an indicator that the responsiveness of a subject to an angiogenesis inhibitor is high, are shown in Table 4.

[Table 4]

| | Expression level of ANG1 | Expression level of ANG2 | Responsiveness |
|---|---|---|---|
| b1 | Lower than control value | - | High |
| b2 | - | Higher than control value | High |
| b3 | Ratio of ex pression levels of ANG1 and ANG2 is lower than control value | | High |

In the above b1, herein, the "control" includes a sample obtained in the past. The sample is to be used as a reference for future comparison with a test sample derived from a subject who was predicted to have therapeutic respoossiveness, More specifically, the control value means a cutoff value, which is obtained by administering a specific angiogenesis inhibitor to patients who are suffering from the same type of tumor, and analyzing expression levels of ANG1 in patients who are evaluated as being resistant and patients who are evaluated as being sensitive. The cutoff value can be easily determined. For example, a control value may be determined as a cutoff value, which is the expression level of ANG1 in a sample derived from a tumor tissue of patients who have been predicted to have therapeutic responsiveness by administration of an angiogenesis inhibitor.

Alternatively, the control value may mean a cutoff value determined based on the presence or absence of a mutation or loss of expression in B-Raf and PTEN. In this case, the control value refers to the expression level of ANG1 in a patient suffering from a tumor having wild-type B-Raf. In this case, a preferable control value is the expression level of ANG1 in a patient suffering from a tumor having wild-type B-Raf and wild-type PTEN.

In this case, the form of expression level of ANG1, which serves as an indicator that the responsiveness of a

subject to the angiogenesis inhibitor is high, is obtained when the result of being equal to or less than the expression level of ANG1 observed in a patient suffering from a tumor having wild-type B-Raf, and particularly preferably, being equal to or less than the expression level of ANG1 observed in a patient suffering from a tumor having wild-type B-Raf and wild type PTEN, is obtained.

In the above b2, the "control" includes a sample obtained in the past. The sample is to be used as a reference for future comparison with a test sample derived from a subject who was predicted to have therapeutic responsiveness. More specifically, the control value means a cutoff value, which is obtained by administering a specific angiogenesis inhibitor to patients who are suffering from the same type of tumor and analyzing expression levels of ANG2 in patients who are evaluated as being resistant and patients who are evaluated as being sensitive. The cutoff value can be easily determined. For example, a control value may be determined as a cutoff value, which is the expression level of ANG2 in a sample derived from a tumor tissue of patients who have been predicted to have therapeutic responsiveness by administration of an angiogenesis inhibitor.

Alternatively, the control value may mean a cutoff value determined based on expression levels of ANG1 and ANG2. In this case, the phrase that the expression level of ANG2 is higher than a control value means that the expression level of ANG2 in terms of absolute value is higher than the expression level of ANG1. In this case, a preferable control value refers to the expression levels of ANG1 and ANG2 in a patient suffering from a tumor in which B-Raf has at least one mutation from Table 1 or loss of expression selected and PTEN has at least one mutation selected from Table 2 or loss of expression.

In this case, particularly, the form of expression level of ANG2, which serves as an indicator that the responsiveness of a subject to the angiogenesis inhibitor is high, is obtained when the result of being higher than the expression level of ANG1 observed in a patient suffering from a tumor in which B-Raf has at least one mutation selected from Table 1 or loss of expression and PTEN has at least one mutation selected from Table 2 or loss of expression, is obtained.

The expression levels of ANG1 and ANG2 in a patient suffering from a tumor in which B-Raf has at least one mutation selected from Table 1 or loss of expression and PTEN has at least one mutation selected from Table 2 or loss of expression are preferably, the expression levels of ANG1 and ANG2 in a patient suffering from a tumor in which B-Raf has V600E mutation and PTEN has T167A, Y68H or L112Q mutation.

In the above b3, the "control" includes a sample obtained in the past. The sample is to be used as a reference for future comparison with a test sample derived from a subject who was predicted to have therapeutic responsiveness. More specifically, the control value means a cutoff value, which is obtained by administering a specific angiogenesis inhibitor to patients who are suffering from the same type of tumor and analyzing the ratio of expression levels of ANG1 and ANG2 in patients who are evaluated as being resistant and patients who are evaluated as being sensitive. The cutoff value can be easily determined. For example, a control value may be determined as a cutoff value, which is the ratio of expression levels of ANG1 and ANG2 (ANG1 expression level/ANG2 expression level) in a sample derived from a tumor tissue of patients who have been predicted to have therapeutic responsiveness by administration of an angiogenesis inhibitor.

Alternatively, the control value may be a cutoff value, which is determined based on the presence or absence of a mutation or loss of expression in B-Raf and PTEN in place of the analysis of the ratio of expression levels of ANG1 and ANG2.

In this case, the preferable control value is a cutoff value, which is the ratio of expression levels of ANG1 and ANG2 in patients in which B-Raf has a mutation or loss of expression and PTEN is a wild type, preferably, the ratio of expression levels of ANG1 and ANG2 in a patient suffering from a tumor in which B-Raf has at least one mutation selected from Table 1 or loss of expression and PTEN is wild type, and more preferably, the ratio of expression levels of ANG1 and ANG2 in a patient suffering from a tumor in which B-Raf has, for example, V600E mutation or A145V mutation and PTEN is wild type.

In this case, particularly the form of the ratio of expression levels of ANG1 and ANG2, which serves as an indicator that the responsiveness of a subject to the angiogenesis inhibitor is high, is obtained when the result of 1) being equal to or less than the ratio of expression levels of ANG1 and ANG2 in a patient suffering from a tumor in which B-Raf is wild type and PTEN is wild type; 2) bring equal to or less than the ratio of expression levels of ANG1 and ANG2 in a patient suffering from a tumor in which B-Raf has at least one mutation selected from Table 1 or loss of expression and PTEN has at least one mutation selected from Table 2 or loss of expression; or 3) being lower than the ratio of expression levels of ANG1 and ANG2 in a patient suffering from a tumor in which B-Raf has at least one mutation selected from Table 1 or loss of expression and PTEN is wild type, is obtained.

A preferable aspect of the ratio of expression levels of ANG1 and ANG2 in a patient suffering from a tumor in which B-Raf has at least one mutation selected from Table 1 or loss of expression and PTEN has at least one mutation selected from Table 2 or loss of expression is the ratio of expression levels of ANG1 and ANG2 in a patient suffering from a tumor in which B-Raf has V600E mutation and PTEN has T167A, Y68H or L112Q mutation. A preferable aspect of the ratio of expression levels of ANG1 and ANG2 in a patient suffering from a tumor in which B-Raf bas at least one mutation selected from Table 1 or loss of expression and PTEN is wild type is the ratio of expression levels of ANG1

and ANG2 in a patient suffering from a tumor in which B-Raf has V600E or A145V mutation and PTEN is wild type.

A method for quantifying the expression levels of SHC1, IL6, CXCR4, COL4A3, NRP2, MEIS1, ARHGAP22, SCG2, FGF9, PML, FGFR3, FGPR2, FGFR1, FGFR4 and VEGFR1 in a sample derived from a tumor tissue of a subject will be described below.

A method of quantifying the expression levels of SHC1, IL6, CXCR4, COL4A3, NRP2, MEIS1, ARHGAP22, SCG2, FGF9, PML, FGFR3, FGPR2, FGFR1, FGFR4 and VEGFR1 in a sample derived from a tumor tissue of a subject is the same as in the aforementioned method for quantifying the expression levels of ANG1 and ANG2 in a sample derived from a tumor tissue of a subject except that an object to be quantified is changed to mRNA or protein of SHC1, IL6, CXCR4, COL4A3, NRP2, MEIS1, ARHGAP22, SCG2, FGF9, PML, FGFR3, FGFR2, FGFR1, FGFR4 or VEGFR1.

Alternatively, the expression level of SHCl, IL6, CXCR4, COL4A3, NRP2, MEIS1, ARHGAP22, SCG2, FGF9, PML, FGFR3, FGPR2, FGFR1, FGFR4 or VEGFR1 in a sample derived from a tumor tissue of a subject can be quantified by analyzing gene expression by a DNA microarray.

In the aforementioned detection, as the probe for quantifying the expression level of SHC1, NRP2, ARHGAP22, SCG2, PML, IL6, CXCR4, COL4A3, MEIS1, FGF9, FGFR3, FGFR2, FGFR1, FGFR4 or VEGFR1, a commercially available product (for example, if a nucleic acid probe is used, it can be purchased through ASSAYS-ON-DEMAND of Applied Biosystems) can be appropriately used.

In the aforementioned detection, since the expression level of SHC1, NRP2, ARHGAP22, SCG2, PML, IL6, CXCR4, COL4A3, MEIS1, FGF9, FGFR3, FGFR2, FGFR1, FGFR4 or VEGFR1 is correlated with the presence or absence of a mutation or loss of expression in B-Raf and PTEN, the expression levels of these genes are correlated with anti-tumor effect determined by the presence or absence of a mutation or loss of expression in B-Raf and PTEN. The expression levels of SHC1, NRP2, ARHGAP22, SCG2 and PML each exhibit the same behavior as the fluctuation pattern of the anti-tumor effect determined by the presence or absence of a mutation or loss of expression in B-Raf and PTEN; whereas the expression levels of IL6, CXCR4, COL4A3, MEIS1, FGF9, FGFR3, FGPR2, FGFR1, FGFR4 and VEGFR1 each exhibit opposite behavior to the fluctuation pattern of the anti-tumor effect determined by the presence or absence of a mutation or loss of expression in B-Raf and PTEN.

Therefore, in the case where the result: (c1) the expression level of SHC1 is low compared to a control value, (c2) the expression level of NRP2 is low compared to a control value, (c3) the expression level of ARHGAP22 is low compared to a control value, (c4) the expression level of SCG2 is low compared to a control value, (c5) the expression level of PML is low compared to a control value, (c6) the expression level of IL6 is high compared to a control value, (c7) the expression level of CXCR4 is high compared to a control value, (c8) the expression level of COL4A3 is high compared to a control value, (c9) the expression level of MEIS1 is high compared to a control value, (c10) the expression level of FGF9 is high compared to a control value, (c11) the expression level of FGFR3 is high compared to a control value, (c12) the expression level of FGFR2 is high compared to a control value, (c13) the expression level of FGFR1 is high compared to a control value, (c14) the expression level of FGFR4 is high compared to a control value, or (c15) the expression level of VEGFR1 is high compared to a control value is obtained, the result serves as an indicator that the responsiveness of a subject to the angiogenesis inhibitor is high. The responsiveness to an angiogenesis inhibitor can be predicted by analyzing one or a plurality of the cases selected from (c1) to (c15) in combination.

In the above (c1) to (c15), the control value refers to the expression level of SHC1, NRP2, ARHGAP22, SCG2, PML, IL6, CXCR4, COL4A3, MEIS1, FGF9, FGFR3, FGFR2, FGFR1, FGFR4 or VEGFR1 in a patient suffering from a tumor in which B-Raf has at least one mutation selected from Table 1 or loss of expression and PTEN is wild type.

In the above (c1) to (c5), particularly, the forms of the expression levels of SHC1, NRP2, ARHGAP22, SCG2 and PML, which serve as an indicator that the responsiveness of a subject to the angiogenesis inhibitor is high, are obtained when the result of being low compared to the expression levels of SHC1, NRP2, ARHGAP22, SCG2 and PML in a patient suffering from a tumor in which B-Raf has at least one mutation selected from Table 1 or loss of expression and PTEN is wild type, is obtained.

In this case, a preferable aspect of each of the expression levels of SHCl, NRP2, ARHGAP22, SCG2 and PML in a patient suffering from a tumor in which B-Raf has at least one mutation selected from Table 1 or loss of expression and PTEN is wild type is the expression level of each of SHCl, NRP2, ARHGAP22, SCG2 and PML in a patient suffering from a tumor in which B-Raf has V600E or A145V mutation and PTEN is wild type.

In the above (c6) to (c15), particularly, the forms of the expression levels of IL6, CXCR4, COL4A3, MEIS1, FGF9, FGFR3, FGFR2, FGFR1, FGFR4 and VEGFR1, which serve as an indicator that the responsiveness of a subject to the angiogenesis inhibitor is high, are obtained when the result of being high compared to the expression levels of IL6, CXCR4, COL4A3, MEIS1, FGF9, FGFR3, FGFR2, FGFR1, FGFR4 and VEGFR1 in a patient suffering from a tumor in which B-Raf has at least one mutation selected from Table 1 or loss of expression and PTEN is wild type, is obtained.

In this case, a preferable aspect of each of the expression levels of IL6, CXCR4, COL4A3, MEIS1, FGF9, FGFR3, FGFR2, FGFR1, FGFR4 and VEGFR1 in a patient suffering from a tumor in which B-Raf has at least one mutation selected from Table 1 or loss of expression and PTEN is wild is the expression level of each of IL6, CXCR4, COL4A3, MEIS1, FGF9, FGFR3, FGFR2, FGFR1, FGFR4 and VEGFR1 in a patient suffering from a tumor in which B-Raf has V600E or A145V mutation and PTEN is wild type.

The forms of the expression levels of SHC1, NRP2, ARHGAP22, SCG2, PML, IL6, CXCR4, COL4A3, MEIS1, FGF9, FGFR3, FGFR2, FGFR1, FGFR4 and VEGFR1, which serve as an indicator that the responsiveness of a subject to the angiogenesis inhibitor is high, are shown in Table 5.

[Table 5]

| | Target to be quantified | Expression level | Responsiveness |
|---|---|---|---|
| c1 | SHC1 | Lower than control value | High |
| c2 | NRP2 | Lower than control value | High |
| c3 | ARHGAP22 | Lower than control value | High |
| c4 | SCG2 | Lower than control value | High |
| c5 | PML | Lower than control value | High |
| c6 | IL6 | Higher than control value | High |
| c7 | CXCR4 | Higher than control value | High |
| c8 | COL4A3 | Higher than control value | High |
| c9 | MEIS1 | Higher than control value | High |
| c10 | FGF9 | Higher than control value | High |
| c11 | FGFR3 | Higher than control value | High |
| c12 | FGFR2 | Higher than control value | High |
| c13 | FGFR1 | Higher than control value | High |
| c14 | FGFR4 | Higher than control value | High |
| c15 | VEGFR1 | Higher than control value | High |

Another aspect of the present invention is the case where the determination result of the following determination target:

(a) B-Raf and PTEN,
(b) ANG1 and ANG2, or
(c) at least one selected from the group consisting of SHC1, NRP2, ARHGAP22, SCG2, PML, IL6, CXCR4, COL4A3, MEIS1, FGF9, FGFR3, FGFR2, FGPR1, FGFR4 and VEGFR1

determined in a sample taken form a single patient is compared to the control value of each of the targets to thereby associate the responsiveness to an angiogenesis inhibitor, and, in addition, the aforementioned determination targets are quantified or detected in samples derived from a plurality of patients. Accordingly, the presence or absence of mutation or the expression level of each of the aforementioned determination targets is detected or determined in the predetermined number of patients (primary population) and the obtained detection value or measurement value, which is used as basic data, can be compared to measurement data in the sample derived from a single subject or samples derived from a plurality of populations (secondary population).

Alternatively, the measurement data of individual patients are added to values of the primary population and the entire data are processed again. In this manner, the number of target patients or cases of the secondary population can be increased. The prediction accuracy for responsiveness to an angiogenesis inhibitor can be enhanced by increasing the number of cases.

The method according to the present invention can be used for predicting the level of efficacy of an angiogenesis inhibitor in a subject before the angiogenesis inhibitor is administered to the subject In this way, a subject in which a higher effect of an angiogenesis inhibitor can be expected is selected to treat a disease. As the case where a higher anti-tumor effect can be expected, a case where a higher anti-tumor effect can be expected than an average anti-tumor effect in subjects presenting similar symptoms; a case where a higher anti-tumor effect can be expected than those in other subjects suffering from the same type of cancer; or a case where a higher anti-tumor effect can be expected than that of a subject suffering from another type of cancer, can be mentioned. Therefore, the present invention is clinically very useful.

As another aspect of the present invention, there is provided a method of using data, in administering an angiogenesis inhibitor to a subject suffering from a tumor or in treating the tumor; based on

(a) the presence or absence of a mutation or loss of expression in B-Raf and PTEN, (b) the expression levels of ANG1 and ANG2, or

(c) the expression level of at least one selected from the group consisting of SHC1, NRP2, ARHGAP22, SCG2, PML, IL6, CXCR4, COL4A3, MEIS1, FGF9, FGFR3 and FGFR2. As described above, a case where (a1) B-Raf is wild type and PTEN is wild type, (a2) B-Raf has at least one mutation selected from Table 1 or loss of expression and PTEN has at least one mutation selected from Table 2 or loss of expression, (b1) the expression level of ANG1 is low compared to a control value (b2) the expression level of ANG1 is equal to and higher compared to a control value and the expression level of ANG2 is sufficient to cancel out the expression of ANG1 (b3) the ratio of the expression levels of ANG1 and ANG2 is low compared to a control value (c1) the expression level of SHC1 is low compared to a control value (c2) the expression level of NRP2 is low compared to a control value (c3) the expression level of ARHGAP22 is low compared to a control value (c4) the expression level of SCG2 is low compared to a control value (c5) the expression level of PML is low compared to a control value (c6) the expression level of IL6 is high compared to a control value (c7) the expression level of CXCR4 is high compared to a control value (c8) the expression level of COL4A3 is high compared to a control value (c9) the expression level of MEIS1 is high compared to a control value (c10) the expression level of FGF9 is high compared to a control value (c11) the expression level of FGFR3 is high compared to a control value (c12) the expression level of FGFR2 is high compared to a control value (c13) the expression level of FGFR1 is high compared to a control value (c14) the expression level of FGFR4 is high compared to a control value or (c15) the expression level of VEGFR1 is high compared to a control value serves as an indicator that it is effective to administer an angiogenesis inhibitor to the subject or treat a tumor. Use of the indicator enables to evaluate how highly a subject responds to an angiogenesis inhibitor and evaluate the possibility of a subject to respond to an angiogenesis inhibitor. The evaluation results are useful data as a reference in determining the right or wrong of administration of an angiogenesis inhibitor to a subject or in selecting e.g., a tumor therapeutic regimen using an angiogenesis inhibitor. However, in the present invention, since an angiogenesis inhibitor basically has an inhibitory action on angiogenesis, even if a subject is determined not to be highly sensitive to an angiogenesis inhibitor, it is not predicted that the angiogenesis inhibitor has no anti-tumor effect.

Note that a person who administers an angiogenesis inhibitor to a subject suffering from a tumor or a person who treats a tumor and a person who performs the measurement of the above (a1) to (c15) may be the same or different.

Another aspect of the present invention, there is provided a method for administering an angiogenesis inhibitor to a subject suffering from a tumor or treating the tumor by using

(a) the presence or absence of a mutation or loss of expression in B-Raf and PTEN,

(b) the expression levels of ANG1 andANG2, or

(c) the expression level of at least one selected from the group consisting of SHC1, IL6, CXCR4, COL4A3, NRP2, MEIS1, ARHGAP22, SCG2, FGF9, PML, FGFR3, FGFR2, FGFR1, FGFR4 and VEGFR1, as an indicator. As described above, the case where (a1) B-Raf is wild type and PTEN is wild type; (a2) B-Raf has at least one mutation selected from Table 1 or loss of expression and PTEN has at least one mutation selected from Table 2 or loss of expression; (b1) the expression level of ANG1 is low compared to a control value; (b2) the expression level of ANG1 is equal to and higher compared to a control value and the expression level of ANG2 is sufficient to cancel out the expression of ANG1; (b3) the ratio of the expression levels of ANG1 and ANG2 is low compared to a control value; (c1) the expression level of SHC1 is low compared to a control value; (c2) the expression level of NRP2 is low compared to a control value; (c3) the expression level of ARHGAP22 is low compared to a control value; (c4) the expression level of SCG2 is low compared to a control value; (c5) the expression level of PML is low compared to a control value; (c6) the expression level of IL6 is high compared to a control value; (c7) the expression level of CXCR4 is high compared to a control value; (c8) the expression level of COL4A3 is high compared to a control value; (c9) the expression level of MEIS1 is high compared to a control value; (c10) the expression level of FGF9 is high compared to a control value; (c11) the expression level of FGFR3 is high compared to a control value; (c12) the expression level of FGFR2 is high compared to a control value; (c13) the expression level of FGFR1 is high compared to a control value; (c14) the expression level of FGFR4 is high compared to a control value; or (c15) the expression level of VEGFR1 is high compared to a control value, is an indicator that it is effective to administer the angiogenesis inhibitor to the subject and treat a tumor. In determining right or wrong of administration of an angiogenesis inhibitor to a subject or selecting e.g., a tumor therapeutic regimen using an angiogenesis inhibitor, the subject, to whom administration of the angiogenesis inhibitor or treatment of the tumor is predicted to be effective, can be selected as an administration target for the angiogenesis inhibitor. Therefore, the present invention encompasses a method for treating a subject, who is suffering from a tumor and predicted to be highly responsive to an

angiogenesis inhibitor by the prediction method of the present invention, by administering the angiogenesis inhibitor. However, in the present invention, since an angiogenesis inhibitor basically has an inhibitory action on angiogenesis, even if a subject is determined not to be highly sensitive to an angiogenesis inhibitor it is not predicted that the angiogenesis inhibitor has no anti-tumor effect.

Note that a person who administers an angiogenesis inhibitor to a subject suffering from a tumor or a person who treats a tumor and a person who performs the measurement of the above (a1) to (c15) may be the same or different.

As another aspect of the present invention, there is provided a method for selecting a subject who is highly sensitive to an angiogenesis inhibitor by using

(a) the presence or absence of a mutation or loss of expression in B-Raf and PTEN,

(b) the expression levels of ANG1 and ANG2, or

(c) the expression level of at least one selected from the group consisting of SHC1, IL6, CXCR4, COL4A3, NRP2, MEIS1, ARHGAP22, SCG2, FGF9, PML, FGFR3, FGFR2, FGFR1, FGFR4 and VEGFR1, as an indicator. As described above, the case where (a1) B-Raf is wild type and PTEN is wild type; (a2) B-Raf has at least one mutation selected from Table 1 or loss of expression and PTEN has at least one mutation selected from Table 2 or loss of expression, (b1) the expression level of ANG1 is low compared to a control value; (b2) the expression level of ANG1 is equal to and higher compared to a control value, and the expression level of ANG2 is sufficient to cancel out the expression of ANG1; (b3) the ratio of the expression levels of ANG1 and ANG2 is low compared to a control value; (c1) the expression level of SHC1 is low compared to a control value, (c2) the expression level of NRP2 is low compared to a control value; (c3) the expression level of ARHGAP22 is low compared to a control value; (c4) the expression level of SCG2 is low compared to a control value; (c5) the expression level of PML is low compared to a control value; (c6) the expression level of IL6 is high compared to a control value; (c7) the expression level of CXCR4 is high compared to a control value; (c8) the expression level of COL4A3 is high compared to a control value; (c9) the expression level of MEIS1 is high compared to a control value; (c10) the expression level of FGF9 is high compared to a control value; (c11) the expression level of FGFR3 is high compared to a control value; (c12) the expression level of FGFR2 is high compared to a control value; (c13) the expression level of FGFR1 is high compared to a control value (c14) the expression level of FGFR4 is high compared to a control value; or (c15) the expression level of VEGFR1 is high compared to a control value, is an indicator that the subject is highly sensitive to an angiogenesis inhibitor. Accordingly, such a subject can be selected as the subject who is highly sensitive to the angiogenesis inhibitor.

Note that a person who selects a subject who is highly sensitive to the angiogenesis inhibitor and a person who performs the measurement of the above (a1) to (c15) may be the same or different.

As another aspect of the present invention, there is provided a pharmaceutical composition comprising an angiogenesis inhibitor. A subject to which the pharmaceutical composition of the present invention is to be administered is a subject, who is suffering from a tumor and has been predicted to be highly responsive to the angiogenesis inhibitor by the method of the present invention. Furthermore, the present invention provides use of an angiogenesis inhibitor for producing a medicinal drug to be administered to a subject suffering from a tumor. The subject is a subject who has been predicted to be highly responsive to the angiogenesis inhibitor by the method of the present invention. Furthermore, the present invention provides an angiogenesis inhibitor for treating a subject suffering from a tumor and the subject is a subject who has been predicted to be highly responsive to the angiogenesis inhibitor by the method of the present invention.

In the pharmaceutical composition and tumor therapy method of the present invention targeting tumor cells having a mutation or loss of expression of B-Raf and PTEN, one or a plurality of other anti-tumor agents may be used in combination. The other anti-tumor agent is not particularly limited as long as it is a preparation having an anti-cancer activity. Examples of the other anti-tumor agent include irinotecan hydrochloride (CPT-11), carboplatin, oxaliplatin, 5-fluorouracil (5-FU), docetaxel (Taxotere (registered trade mark)), paclitaxel, gemcitabine hydrochloride (Gemzar (registered trade mark)), calcium folinate (Leucovorin), bevacizumab (Avastin (registered trade mark)) and everolimus (Certican (registered trade mark) or Afinitor (registered trade mark)). Furthermore, examples of the other anti-tumor agent particularly preferably include dacarbazine or temozolomide when the kind of tumor to be treated by a tumour therapeutic agent is melanoma; irinotecan hydrochloride, oxaliplatin, 5-fluorouracyl, calcium folinate or bevacizumab when it is large bowel cancer; gemcitabine hydrochloride or bevacizumab when it is pancreatic cancer, carboplatin or gemcitabine hydrochloride when it is ovarian cancer, bevacizumab or everolimus when it is kidney cancer; and carboplatin, docetaxel or paclitaxel when it is lung cancer.

The pharmaceutical composition of the present invention can be used as a tumor therapeutic agent. In the present invention, the tumor therapeutic agent includes an anti-tumor agent, a cancer prognosis improving agent, a cancer recurrence preventive and a cancer, metastasis inhibitor.

The effects of cancer therapy can be confirmed by observation such as radiograph, CT and histopathological diagnosis such as biopsy or a value of a tumor marker.

When the pharmaceutical composition of the present invention is used, it can be orally or parenterally administered, In using the pharmaceutical composition of the present invention, the dose of an angiogenesis inhibitor varies depending upon e.g., severity of symptom, the age, sex, weight and degree of sensitiveness of a subject, an administration route, an dosing timing, an dosing interval, properties, formulation and type of a pharmaceutical formulation and type of active ingredient. Although it is not particularly limited; the dose is usually 0.1 mg to 10 g per adult (body weight: 60 kg) per day, which is divided into portions and administered at a frequency of usually from one per week to three times per day.

The pharmaceutical composition of the present invention can be formulated into e.g., an oral solid formulation and an injection. Examples of the oral solid formulation include a tablet, a coated table, a granule, a fine grain formulation, a powder and an encapsulated formulation, Examples of the injection include an intravenous injection, a subcutaneous injection and an intramuscular injection. If necessary, they can be lyophilized by a conventional method,

In formulating into a formulation, additives conventionally used such as an excipient, a binding agent, a lubricant, a colorant and a flavoring agent can be used and, if necessary, a stabilizer, an emulsifier, an absorption accelerator, a surfactant and others can be used. Generally, components used as raw materials for a pharmaceutical formulation are blended and formulated into a formulation in accordance with a conventional method.

Examples of these components include animal and vegetable oils (e.g., soybean oil, beef tallow, synthetic glyceride), hydrocarbon (e.g., fluid paraffin, squalane, solid paraffin), ester oils (e.g., octyldodecyl myristate, isopropyl myristate), higher alcohols (e.g., cetostearyl alcohol, behenyl alcohol), silicon resin, silicon oil, surfactants (e.g., polyoxyethylene fatty acid ester, sorbitan fatty acid ester, glycerin fatty acid ester, polyoxyethylene sorbitan fatty acid ester, polyoxyethylene hydrogenated castor oil, polyoxyethylene-polyoxypropylene block copolymer), water soluble polymer (e.g., hydroxyethylcellulose, polyacrylic acid, carboxyvinyl polymer polyethylene glycol, polyvinyl pyrrolidone, methylcellulose), alcohols (e.g., ethanol, isopropanol), polyhydric alcohols (e.g., glycerin, propylene glycol, dipropylene glycol, sorbitol), sugars (e.g., glucose, sucrose), inorganic powders (silicic anhydride, magnesium aluminum silicate, aluminum silicate) and purified water, For controlling pH, e.g., an inorganic acid (e.g., hydrochloric acid, phosphoric acid), an alkaline metal salt of an inorganic acid (e.g., sodium phosphate), an inorganic base (e.g., sodium hydroxide), an organic acid (e.g., lower fatty acid, citric acid, lactic acid), an alkali metal salt of an organic acid (e.g., sodium citrate, sodium lactate) and/or an organic base (e.g., arginine, ethanolamine) can be used. If necessary, an antiseptic agent and/or an antioxidant can be added.

As another aspect of the present invention, the present invention provides a kit for predicting responsiveness of a subject suffering from a tumor to an angiogenesis inhibitor, characterized by comprising probes of B-Raf and PTEN or ANG1 and ANG2. Prediction of the responsiveness to an angiogenesis inhibitor can be performed by the method of the present invention. As the angiogenesis inhibitory, preferably, 4-(3-chloro-4-(cyclopropylaminocarbonyl)aminophenoxy)-7-methoxy-6-quinolinecarboxamide or a pharmacologically acceptable salt thereof is mentioned.

The pharmaceutical composition of the present invention and/or kit can be applied to mammals (for example, a human, a rat, a rabbit, a sheep, a pig, a cow, a cat, a dog, a monkey).

The pharmaceutical composition of the present invention and/or kit may comprise, other than the angiogenesis inhibitor or probes, e.g., a package container, an instruction booklet and a package insert. In the package container, instruction booklet and package insert, for example, combination of one or a plurality of other anticancer agents concomitantly used can be described. Furthermore, dosage and administration with respect to the form for administering independent substances in combination or a form as a mixture. The dosage and administration can be described, with reference to the above.

## Examples

The present invention will be more specifically described by way of Examples; however, the present invention is not limited to these examples.

[Example 1] Detection of mutation or loss of expression of BRAF and PTEN

Human melanoma cell lines, SK-MEL-2, MeWo, CHL-1, HMV-1, HMCB, MDA-MB-435, LOX, G361, FEM, SEKI, SK-MEL-28, A375 and A2058 were each obtained from the manufacturers shown in the column of "distributor" of Table 6 and analyzed by the Sanger method or a next generation sequence method (Bridge PCR method: Solexa/Illumina) to detect a mutation or loss of expression of BRAF and PTEN.

(1) Detection by the Sanger method

(i) Preparation af genomic DNAfrom melanoma cell line

Genomic DNA was purified from cells (about 1 × $10^6$) by use of DNeasy Blood & Tissue Kit (purchased from QIAGEN).

(ii) Amplification of PTEN exon region

The obtained genomic DNA was subjected to PCR to amplify the exon region of PTEN. PCR was performed by PrimeSTAR GXL DNA Polymerase (purchased from Takara Bio In.). Genomic DNA (100 ng), 5 × PrimeSTAR GXL Buffer (4 μL), a dNTP mixture (2.5 mM) (1.6 μL), a sense primer and an anti-sense primer (5 pmol for each) and PrimeSTAR GXL DNA Polymerase (0.4 μl) were mixed to prepare a solution having a total volume of 20 μL. The solution was subjected to a reaction which was performed by repeating, 40 times, a cycle consisting of a reaction at 95°C for 10 seconds, a reaction at 55°C for 15 seconds and a a reaction at 68°C for 30 seconds.

The sequences of primers used in the PCR are shown below.

Exon1 Sense Primer: AGTCGCCTGTCACCAITTC(SEQ ID NO: 19)
Exon1 Antisense Primer: ACTACGGACATTTTCGCATC(SEQ ID NO: 20)
Exon2 Sense Primer: GTTTGATTGCTGCATATTTCAG(SEQ ID NO: 21)
Exon2 Antisense Primer: GGCFTAGAAATCTTTTCTAAATG(SEQ ID NO: 22)
Exon3 Sense Primer; AATGACATGATTACTACTCTA(SEQ ID NO: 23)
Exon3 Antisense Primer: TTAATCGGTTTAGGAATACAA(SEQ ID NO: 24)
Exon4 Sense Primer: CATTATAAAGATTCAGGCAATG(SEQ ID NO: 25)
Exon4 Antisense Primer: GACAGTAAGATACAGTCTATC(SEQ ID NO: 26)
Exon5 Sense Primer: ACCTGTTAAGTTTGTATGCAAC(SEQ ID NO: 27)
Exon5 Antisense Primer: TCCAGGAAGAGGAAAGGAAA(SEQ ID NO: 28)
Exon6 Sense Primer: CATAGCAATTTAGTGAAATAACT(SEQ ID NO: 29)
Exon6 Antisense Primer: GATATGGTTAAGAAAACTGTTC(SEQ ID) NO: 30)
Exon7 Sense Primer: TGACAGTTTGACAGTTAAAGG(SEQ ID NO: 31)
Exon7 Antisense Primer: GGATATTTCTCCCAATGAAAG(SEQ ID NO: 32)
Exon8 Sense Primer: CTCAGATTGCCTTATAATAGT(SEQ ID NO: 33)
Exon8 Antisense Primer: TCATGTTACTGCTACGTAAAC(SEQ ID NO: 34)
Exon9 Sense Primer: AAGGCCCTCTTAAAGATCATG(SEQ ID NO: 35)
Exon9 Antisense Primer: TTTTCATGGTGTTTTATCCCT(SEQ ID NO: 36)

(iii) Recovery of PCR product

The PCR product which was confirmed to have a desired length by 1% agarose gel electrophoresis, was recovered from the gel and purified by use of Wizard SV Gel and PCR Clean-Up System (purchased from Promega). The purified genomic DNA was subjected to PCR by a commercially available kit to determine the sequence.

(2) Detection by the next generation sequence method

(i) Preparation of total RNA from melanoma 13 cell line

Cells were cultured in a 5% $CO_2$ condition at 37°C. After a predetermined period of time, the cells were lysed with TRIZOL reagent (purchased from GIBCO BRL) in accordance with the operation manual described in the attachment of the reagent.

The method was more specifically performed as follows. TRIZOL reagent was added at a ratio of 1 ml per culture area (10 $cm^2$), pipetted several times and then the liquid containing cell lysis was recovered, The sample thus recovered was centrifuged and the resultant supernatant was allowed to stand at room temperature for 5 minutes. To the sample, chloroform (purchased from Junsei Chemical Co., Ltd.) was added at a ratio of 0.2 ml to the volume of TRIZO reagent (1 ml). This solution was vigorously shaken and stirred for 15 seconds and stirred, allowed to stand at room temperature for 2 to 3 minutes and centrifuged (12000 × g, 10 minutes, 4°C). After centrifugation, an aqueous layer was transferred to a new tube. To this, isopropyl alcohol (purchased from Wako Pure Chemical Industries Ltd.) was added in a ratio of 0.5 ml to TRIZO reagent (1 ml). The mixture was allowed to stand at room temperature for 10 minutes and then centrifuged (12000 × g, 10 minutes, 4°C). Precipitation was obtained, washed with 75% ethanol

(purchased from Wako Pure Chemical Industries Ltd.) and dried in air to obtain total RNA, which was subjected to the following operations.

(ii) Amplification of sequences encoding BRAF and PTEN protein

Using the RNA obtained above as a template, cDNA was synthesized in accordance with the method described in the package insert of High capacity cDNA Reverse Transcription kit.

The resultant cDNAs of melanoma 13 cell lines were subjected to PCR to amplify sequences encoding B-Raf and PTEN proteins. PCR was performed with PrimeSTAR GXL DNA Polymerase (purchased from Takara Bio Inc.) or Phusion High-Fidelity DNA Polymerase (purchased from Finnzymes). In the case of using PrimeSTAR GXL DNA Polymer cDNA (100 ng), 5 × PrimeSTAR GXL Buffer (4 μL), a dNTP mixture (2,5 mM)(1.6 μL), a sense primer and an anti-sense primer (5 pmol for each) (with respect to B-Raf, a reaction was performed by using three types of primers for each) and PrimeSTAR GXL DNA Polymerase (0.4 μl) were mixed to prepare a solution of a total volume of 20 μL and the solution was subjected to a reaction which was performed by repeating, 40 times, a cycle consisting of a reaction at 95°C for 10 seconds, a a reaction at 55°C for 15 seconds and a reaction at 68°C for 2 minutes. In the case of using Phusion High-Fidelity DNA Polymerase, cDNA (100 ng), 5 × Phusion GC Buffer (4 μL), a dNTP mixture (2.5 mM) (1.6 μL), a sense primer and an anti-sense primer (10 pmol for each) and Phusion High-Fidelity DNA Polymerase (0.2 μL) were mixed to prepare a solution having a total volume of 20 μL and the solution was subjected to a reaction which was performed by repeating, 40 times, a cycle consisting of a, a reaction at 8°C for 10 seconds, a a reaction at 55°C for 30 seconds and a a reaction at 72°C for 2 minutes.

The sequences of the primers used in PCR are shown below.

PTEN Sense Primer: TCTGCCATCTCTCTCCTCCTTTT(SEQ ID NO: 37)
PTEN Antisense Primer: TCTGACACAATGTCCTATTGCCAT(SEQ ID NO: 38)
BRAF Sense Primer 1: GCCCCGGCTCTCGGQTIATAAGATG(SEQ ID NO: 39)
BRAF Antisense Primer 1; CCGTTCCCCAGAGATTCCAA(SEQ ID NO: 40)
BRAF Sense Primer 2: TGCCATTCCGGAGGAGGTGT(SEQ ID NO: 41)
BRAF Antisense Primer 2: GCCCATCAGGAATCTCCCAA(SEQ ID NO: 42)
BRAF Sense Primer 3: ATCTGGATCATCCCCTTCCGC(SEQ ID NO: 43)
BRAF Antisense Primer 3: CCCGGAACAGAAAGTAAAGCCTCTAG(SEQ ID NO: 44)

(iii) Recovery, purification, blunting and ligation of PCR product

The PCR product, which was confirmed to have a desired length by 1% agarose gel electrophoresis, was recovered from the gel and purified by use of Wizard SV Gel and PCR Clean-Up System (purchased from Promega). The purified PCR products were collected to a single tube per cell line and a total volume (10 μL) was subjected to a blunting treatment by a DNA Blunting Kit (purchased from Takara Bio Inc.). Thereafter, phenol/chloroform extraction and ethanol precipitation were performed to obtain DNA pellets and a ligation treatment was performed by use of DNA Ligation Kit (purchased from Takara Bio Inc.) in a total volume of 10 μL at 16°C for 6 hours. The DNA ligated was subjected to phenol/chloroform extraction and ethanol precipitation to obtain DNA pellets.

(iv) Preparation of library for analysis by a next generation sequencer

A library was prepared by use of Genomic DNA Sample Prep Kit (purchased from Illumina) in accordance with the operation manual attached. The outline of the method is as follows.

DNA was subjected to nebulization to obtain fragments. The DNA fragments are blunted and 5' terminals thereof were phosphorylated. After an adaptor was added, a 2% agarose gel electrophoresis was performed. A product of 150 bp to 200 bp in length was recovered from the gel and purified. DNA was subjected to PCR using the purified DNA as a template and purified. Absorbance of the resultant DNA was measured to check the concentration and purity thereof.

(v)Acquisition of data by next generation sequencer

Using GAII DNA Sample Cluster Generation Kit (purchased from Illumina) and 36-Cycle SBS Sequencing Kit (purchased from Illumina), a cluster was formed in accordance with the operation manual attached and data were obtained by Genome Analyzer II (Illumina). A sample (3 pmol) derived from a single cell line was used per lane. The number of cycles performed was 36 or 76.

(vi) Data analysis

Using IPAR/GAPipeline manufactured by Illumina, TXT-form sequence data were prepared from an image of TIFF-form and converted into FASTQ-form. Thereafter, alignment was performed by use of MAQ with reference to Refseq sequence data of a related gene. To the SNPs data extracted, e.g., amino acid substitution data were added and converted into GFF-form by use of built-in software. Mutation information and depth information (mutation or loss of expression information) were checked by use of Gbrowse. The results are shown in Table 6.
[Table]

Table 6 Presence or absence of mutation or loss of expression of B-Raf and PTEN in human melanoma cell line

| Cell line | Manufacturer | Culture medicum | Mutation or Loss of expression | |
|---|---|---|---|---|
| | | | B-Raf | PTEN |
| SK-MEL-2 | ATCC | MEM | None | None |
| MeWo | ATCC | MEM | None | None |
| CHL-1 | ATCC | DMEM | None | None |
| HMV-1 | Dainippon Pharma Co., Ltd. | RPMI1640 | None | None |
| HMCB | ATCC | MEM | None | None |
| MDA-MB-435 | Dr. Mary J; C. Hendrix at the University of Arizona | RPMI1640 | V600E | None |
| LOX | Dainippon Pharma Co., Ltd. | RPMI1640 | V600E | None |
| G361 | Dainippon Pharma Co., Ltd. | MacCoy’s | V600E | None |
| FEM | Dr, Fodstad at the Norw. Rad. Hosp. | RPMI1640 | A145V | None |
| SEKI | Dainippon Pharma Co., Ltd. | RPMI1640 | V600E | None |
| SK-MEL-28 | ATCC | MEM | V600E | T167A |
| A375 | Dainippon Pharma Co., Ltd. | RPMI1640 | V600E | Y68H |
| A2058 | ATCC | DMEM | V600E | L112Q |

[Example 2] Calculation of anti-tumor effect of angiogenesis inhibitor on mouse model grafted with melanoma cell line.

The human melanoma cell lines used in Example 1 were respectively cultured in the mediums shown in the column of "Culture medium" (containing 10% FBS) of Table 6 until about 80% confluency was obtained (in an incubator under 5% carbon dioxide gas), After culturing, cells were collected by typsin-EDTA treatment in accordance with a conventional method. The cells were suspended with a phosphate buffer or a matrigel solution (mixture of phosphate buffer and matrigel in a common ratio of 1:1) to prepare suspension solution of $1 \times 10^8$ cells/mL or $5 \times 10^7$ cells/mL. The cell suspension (0.1 mL) was subcutaneously grafted to the side of the body of each nude mouse. In this manner, human melanoma cell line grafted mouse models were prepared.

After grafting, from the time point when a tumor volume reached about 200 $mm^3$, a mesylate of 4-(3-chloro-4-(cyclopropylaminocarbonyl)aminophenoxy)-7-methoxy-6-quinolinecarboxamide (hereinafter referred to as E7080) (100 mg/kg/day) was orally administered. The major axis and minor axis of the tumor were measured everyday by Digimatic Caliper (Mitsutoyo). The tumor volume and specific tumor volume were calculated in accordance with the following formula and thereby the anti-tumor effect ($\Delta$T/C) of an angiogenesis inhibitor on the mouse model was measured.

$$\text{Tumor volume (TV)} = \text{tumor longest diameter (mm)} \times \text{tumor short diameter}^2 (\text{mm}^2)/2$$

$$\text{Anti-tumor effect } (\Delta T/C) = (\text{TV of medicinal drug administered group - TV before initiation of administration})/(\text{TV of control group - TV before initiation of administration}) \times 100$$

The anti-tumor effect ΔT/C on the 7th day after initiation of administration is shown in Figure 1, In Figure 1, the same cell lines used in Example 1 were used

The anti-tumor effects (ΔT/C) of E7080 on melanoma cells of the case where both of BRAF and PTEN are wild type, the case where both of BRAF and PTEN have a mutation or loss of expression, and the case where BRAF has a mutation and PTEN has no mutation, were 16, 23 and 45%, respectively. The anti-tumor effect of E7080 was observed particularly in the case where both of BRAF and PTEN have a mutation or loss of expression and in the case where BRAF and PTEN do not have a mutation or loss of expression. It is clear that the anti-tumor effect of E7080 varies between melanoma cells which are classified based on the presence or absence of a mutation in BRAF and PTEN (Figure 1). It was demonstrated that the presence or absence of a mutation in BRAF and PTEN is used as an indicator for predicting the effect of E7080.

[Example 3] Correlation with the ratio of blood vessels covered with periderm cells (pericytes) depending upon the presence or absence of a mutation or loss of expression in BRAF and PTEN

As a result of imperfect angiogenesis in a tumor tissue, a phenomenon where blood vessels covered with periderm cells is not formed is observed. In the case where the tumor cells were classified based on the presence or absence of a mutation or loss of expression in BRAF and PTEN, whether the ratio of blood vessels covered with periderm cells changes or not was investigated.

Human melanoma cell line grafted mouse models were prepared using the human melanoma cell lines used in Example 1 in accordance with the method of Example 2. After grafting, at the time point when a tumor volume reached about 100-300 $mm^3$, the mouse was sacrificed with $CO_2$ and the grafted tumor tissue was excised out by a surgical operation.

Thereafter, from the tumor tissue excised out, a tumor tissue sections were prepared. The sections were stained.

To describe this procedure more specifically, the portion at a distance about 5 mm inside from the periphery of the tumor tissue was cut by knife, the tissue was embedded in OCT Compound. Thereafter, the tissue was frozen with dry ice and a frozen tissue was prepared at -80°C. From the frozen tissue, sections of 8 μm in thickness were prepared, attached to a slide glass, washed with running water and allowed to stand in cold acetone at 4°C for 10 minutes to prepare samples. Thereafter, the samples were washed three times with a 0.01 M phosphate buffer containing 0.1% Tween20 (hereinafter referred to as washing PBS) and allowed to react with an avidin blocking solution in DAKO biotin blocking kit for 10 minutes at room temperature. After completion of the reaction, the sample was washed three times with washing PBS, and allowed to react with a biotin blocking solution in the kit at room temperature for 10 minutes. Thereafter, the sample was washed three times with washing PBS and allowed to react with normal serum in VECTOR STAIN ABC peroxidase rat IgG kit at room temperature for 20 minutes. After the reaction solution was removed from the samples, primary antibody, i.e., an anti-CD31 antibody (name of clone: MEC13.3, rat IgG, PharMingen, BD Biosciences), which was diluted 600 times with a 0.1 M phosphate buffer containing a 1% fetal bovine serum, was added and allowed to react overnight at 4°C. Thereafter, the samples were washed, and a secondary antibody labeled with biotin in the kit was added and allowed to react at room temperature for 30 minutes. The samples were washed and then further reacted with an avidin reagent in the kit at room temperature for 30 minutes. Thereafter, the samples were washed three times with a 0.01 M phosphate buffer and color was developed with DAB to stain CD31.

Subsequently, the samples were washed with running water and washed three times with Tris buffer. Thereafter, with the samples, alkali phosphatase-labeled anti-α-SMA antibody (name of clone: 1A4, mouse IgG, SIGMA-ALDRICH) diluted 100 times with Tris buffer was allowed to react at room temperature for one hour. Thereafter, the samples were washed three times with Tris buffer and color was developed with a fuchsine solution in DAKO LSAB kit to stain α-SMA.

Each of the samples stained was placed under a microscope and the number of blood vessels and the number of blood vessels covered with pericytes were counted by a CCD camera HYPER SCOPE (KEYENCE) in e.g., 5 sites per sample, and averaged. The number of blood vessels or pericytes per unit area was obtained. Furthermore, the ratio of the blood vessels with pericytes in the number of the blood vessels was calculated in each melanoma cell line. The results are shown in Figure 2. In Figure 2, the same cell lines used in Example 1 were used.

Between the classes of the melanoma cells, which were classified based on the presence or absence of a mutation or loss of expression in BRAF and PTEN, the ratio of the blood vessels covered with pericytes in the tumor tissue tended to differ (Figure 2). Furthermore, the anti-tumor effect of E7080 and the ratio of the blood vessels covered with pericytes in a tumor tissue were analyzed. As a result, the ratio of the blood vessels covered with pericytes μm in

a tumor tissue exhibited high correlation with the anti-tumor effect of E7080 (Figure 3). From this, it was suggested that the presence or absence of a mutation or loss of expression in BRAF and PTEN influences the properties of blood vessels in a tumor and varies the ratio of blood vessels covered with pericytes in the tumor. The results suggest that, in melanoma where the ratio of blood vessels covered with pericytes is low, E7080 may possibly tend to have an effect on the blood vessels, and that particularly in cases where both of BRAF and PTEN are wild type and where both of BRAF and PTEN have a mutation or loss of expression, E7080 easily produces an effect.

[Example 4] Expression regulation of ANG1 and ANG2 by the presence or absence of a mutation or loss of expression in BRAF and PTEN

(1) Correlation between the presence or absence of a mutation or loss of expression in BRAF and PTEN and the expressions of ANG1 and ANG2

In human melanoma cell lines, the correlation between the presence or absence of a mutation or loss of expression in BRAF and PTEN and the expressions of ANG1 and ANG2 was investigated by using the quantitative RT-PCR and ELISA.

1, Investigation by quantitative RT-PCR method

From each of the human melanoma cell lines used in Example 1, total RNA was prepared in the same method as in Example 1 (2) (i) and subjected to the following measurements.

The quantitative RT-PCR method for various types of angiogenesis factors and various types of angiogenesis factor receptors was performed by use of a gene specific probe (TaqMan Gene Expression Assays mixture, purchased through ASSAYS-ON-DEMAND of Applied Biosystems) and gene analysis BioMark™ system (purchased from Fluidigm) based on the operation manual, as follows.

The names of genes of the angiogenesis factors and angiogenesis factor receptors used herein and the assay ID of the probes purchased are shown in Table 7.

The operation consisting of three stages, a reverse transcription reaction, a pre-amplification and PCR, was performed.

In the first stage, i.e., reverse transcription reaction, RNase Free dH2O (6.5 μL) was added to the RNA prepared. To this, 5 x PrimeScript buffer (2 μL), PrimeScript RT Enzyme Mix I (0.5 μL), Oligo dT Primer (50 μM) (0.5 μl) and Random 6 mer (100 μM) (0.5 μL) were further added. After allowed to react at 37°C for 15 minutes, the mixture was heated at 85°C for 5 seconds to terminate the reaction to obtain a cDNA solution. The obtained cDNA solution was subjected to the second stage, pre-amplification reaction.

In the pre-amplification reaction, a low-TE buffer (58 μL) was added to gene specific Primer/Probe (42 μL). From the solution, an aliquot (56.25 μL) was taken. To this, PreAmp Master Mix (112.5 μL) and the cDNA solution (56.25 μL) were added. The mixture was allowed to react at 95°C for 10 minutes, and then, a reaction cycle consisting of a reaction at 95°C for 15 seconds and a reaction at 60°C for 4 minutes was repeated 14 times. After completion of the reaction, the reaction solution was diluted (1:5) with TE buffer, and used as a pre-amplification solution. The obtained pre-amplification solution was subjected to the third stage, PCR.

A sample solution was prepared by adding Loading Buffer (27.5 μL) to 2 x ABI Master Mix (275 μL) and further adding, to 5.5 μL of the solution, 4.5 μL of the pre-amplification solution. Furthermore, an assay solution was prepared by adding 10% Tween to water (4750 μL), taking an aliquot (5 μL) from the solution, and adding 20 x assays (5 μL) to the aliquot. The sample solution and the assay solution were separately added to 48.48 Dynamic tray and a sample was loaded by NanoFlex ICF controlled and thereafter, measurement was performed by Biomark (Fluidigm Corporation).
[Table 7]

Table 7 Angiogenesis factor and angiogenesis factor receptor quantified

| No. | Gene Name | Assay ID |
|---|---|---|
| 1 | beta-actin | Hs99999903_m1 |
| 2 | Ang-1 | Hs_00181613 |
| 3 | Ang-2 | Hs_00169867 |

For performing quantitative analysis of each gene from the obtained PCR product, a calibration curve was established by use of a mRNA sample prepared by adding equivalent amounts of all samples. The expression level of

a gene in each of the melanoma cell lines was obtained by calculating Ct (stands for a threshold cycle value which is the member of cycles of PCR required for a PCR product to reach a predetermined concentration) from the calibration curve. The expression level of a gene in each melanoma cell line was connected by β-actin expression level to obtain an expression level ratio of the gene in the melanoma cell line and used for comparison analysis,

2. Validation by ELISA

Human melanoma cell line grafted mouse models were prepared in accordance with the method of Example 2 by using the human melanoma cells used in Example 1. After grafting, in the stage where a tumor volume reached 100 $mm^3$ or more, the mouse was sacrificed and the tumor tissue grafted was recovered. To the tumor tissue, a cell lysis buffer (purchased from Cell Signaling) was added to prepare a cell sap and stored at -80°C. The expression level of ANG1 protein and the expression level of ANG2 protein in the preparation solution and the ratio of them (ANG1/ANG2) were determined by an ELISA Kit (purchased from R&D systems) and quantified based on a calibration curve.

The results are shown in Figures 4 and 5. In Figure 4, the bar indicated by BRAF "-" includes the results of anti-tumor effects of SK-MEL-2, MeWo, CHL-1, HMV-1 and HMCB cell lines shown in Table 6; whereas, the bar of BRAF "+" includes the results of anti-tumor effects of MDA-MB-435, LOX, G361, FEM, SEKI, SK-MEL-28, A375 and A2058 cell lines shown in Table 6. In Figure 5, the bar indicated by PTEN "-" includes the results of anti-tumor effects of SK-MEL-2, MeWo, CHL-1, HMV-1, HMCB, MDA-MB-435, LOX, G361, FEM and SEKI cell lines shown in Table 6; whereas a bar indicated by PTEN "+" includes the results of anti-tumor effects of SK-MEL-28, A375 and A2058 cell lines shown in Table 6.

It was demonstrated that the mRNA expressions level of ANG1 (No.11 in Table 7) significantly increases in a human melanoma cell line in which BRAF has a mutation (Figure 4 (b)) and the expression level of ANG1 protein increases in a human melanoma cell line in which BRAF has a mutation (Figure 4 (a)).

Furthermore, the mRNA expression level of ANG2 (No.12 in Table 7) increases in a human melanoma cell line in which PTEN has a mutation (Figure 5 (b)), and the expression level of ANG2 protein (No. 12 in Table 7) significantly increases in a human melanoma cell line in which PTEN has a mutation (Figure 5 (a)).

(2) Correlation between the presence or absence of a mutation or loss of expression in BRAF and PTEN and the ratio (ANG1/ANG2) of expression levels of ANG1 and ANG2

It is known that ANG1 binds to TIE-2 receptor and the ANG1-TIE-2 signal induces maturation of blood vessels. To TIE-2 receptor, ANG2 and ANG1 competitively bind. If ANG2 binds to the receptor, no signal flows downstream. This state becomes equivalent to the state where ANG1 is not expressed (or expression is low). In other words, if BRAF is normal, it is known that a signal of VEGF contributes to angiogenesis and survival, and maturation of blood vessels does not occur.

When melanoma cells are classified based on the presence or absence of a mutation of BRAF and PTEN, whether the ANG1-TIE-2 signaling is influenced was investigated based on the ratio (ANG1/ANG2) of the expression level of ANG1 protein and the expression level of ANG2 protein. The results are shown in Figure 6. In Figure 6, the cell lines used in Example 1 were used.

When melanoma cells are classified based on the presence or absence of a mutation of BRAF and PTEN, the ratio (ANG1/ANG2) of the expression level of ANG1 protein and the expression level of ANG2 protein tended to be low in the case where both of BRAF and PTEN are wild type and in the case where BRAF and PTEN have a mutation or a loss of expression; whereas, the ratio (ANG1/ANG2) tended to be high in the case where BRAF has a mutation or loss of expression and PTEN is wild type (Figure 6). Furthermore, compared to the case where BRAF has a mutation or a loss of expression and PTEN is wild type, the ratio of expression levels of ANG1 and ANG2 was shown to be significantly low in the case where both of BRAF and PTEN are wild type and μm in the case where BRAF and PTEN have a mutation or loss of expression. This means that in a mutation or loss of expression of BRAF and PTEN, a combination of mutations likely responsive to E7080 and ANG1/ANG2 exhibit similar tendencies.

From the above results, it was elucidated that the anti-tumor effect of an angiogenesis inhibitor is defined by the presence or absence of a mutation or loss of expression in BRAF and PTEN. In a subject having no BRAF mutation, the ratio of periderm cells around a blood vessel in a tumor tissue decreases. It was elucidated that expression control of ANG1 and ANG2, which are involved in formation of pericyte-covered blood vessels, is involved in the cause thereof. In the case where ANG1 is not expressed or the expression level of ANG1 is low, in other words, in the state where BRAF is normal, particularly if FGFR3 is expressed, an FGFR kinase inhibitor is expected to directly kill cancer cells. This suggested that E7080 has a potential to not only inhibit angiogenesis but also enhance an anti-tumor effect in a melanoma patient in which BRAF is normal and FGFR3 is expressed.

In contrast, it was suggested that if ANG1 is highly expressed, in other words, in the case where BRAF has a mutation or loss of expression, maturation of blood vessels varies depending upon the ANG2 expression; whereas if

ANG2 is highly expressed, in other words, in the case where PTEN has a mutation or loss of expression, no maturation occurs because of competitive inhibition by ANG1 and ANG2; conversely, if the expression of ANG2 is low, in other words, in the case where PTEN is normal, maturation of blood vessels occurs. Therefore, it was suggested that, in the case where ANG1 and ANG2 expression are high, the anti-tumor effect of E7080 due to angiogenesis inhibition can be expected; whereas, in the case where ANG1 expression is high and ANG2 expression is low and if ANG2 expression is higher than ANG1 expression, the anti-tumor effect of E7080 can be expected.

Accordingly, it became possible that the effect of E7080 can be predicted based on ANG1 andANG2 expression levels or the ratio of the expression levels.

[Example 5] Correlation between the presence or absence of a mutation or loss of expression in BRAF and PTEN and the expressions of SHC1, IL6, CXCR4, COL4A3, NRP2, MEIS1, ARHGAP22, SCG2, FGF9, PML, PGFR1, FGFR4 and VEGFR1

In human melanoma cell lines, the correlation between the presence or absence of a mutation or loss of expression in BRAF and PTEN and the expressions of SHC1, IL6, CXCR4, COL4A3, NRP2, MEIS1, ARHGAP22, SCG2, FGF9, PML, FGFR1, FGFR4 and VEGFR1 was investigated by use of a DNAmicroarray method.

1. Extraction of total RNA from sample

Human melanoma cells used in Example 1 were used to prepare human melanoma cell grafted mouse models in the same manner as in Example 2. After grafting, in the stage where a tumor volume reached 200 $mm^3$ or more, the mouse was sacrificed and the tumor tissue grafted was excised out. Total RNA was prepared in the same method as in Example 1 (2) (i) and subjected to the following operations.

2. RNAquantification

1) Quantification by DNA microarray

cDNA synthesis and biotin labeling were performed based on the method of Schena et al. (Schena et al., Science, 1995, 270, p.467-470), the method of Lockhart et al. (Lockhart et al., Nature Biotechnology, 1996, 14, p.1675-1680) or the latest operation manual of GeneChip (registered trade mark) Array Station manufactured by Affimetrix. Thereafter, hybridization with a DNA microarray (human Genome U133 Plus 2.0 Array) manufactured by Affimetrix and measurement were performed based on the operation manual to obtain data.

3. Data analysis

Data were statistically analyzed by a trend test using the cumulative chi-square method and genes which showed a significant change in expression level by the presence or absence of a mutation or loss of expression in BRAF and PTEN were extracted.

As a result, it was elucidated that the expression levels of IL6, CXCR4, COL4A3, MEIS1, FGF9, FGFR1, FGFR4 and VEGPR1 significantly decrease from the expression level of a case where BRAF has a mutation and PTEN is wild type; whereas, the expression levels of SHC1, NRP2, ARHGAP22, SCG2 and PML significantly increase from the expression level of a case where BRAF has a mutation and PTEN is wild type. Thus, it was elucidated that the expression levels of SHC1, IL6, CXCR4, COL4A3, NRP2, MEIS1, ARHGAP22, SCG2, FGF9, PML, FGFR1, FGFR4 and VEGFR1 significantly change when melanoma cells were classified based on the presence or absence of a mutation or loss of expression in BRAF and PTEN.

More specifically, if the responsiveness of a subject to an angiogenesis inhibitor is high, it was suggested that

the expression level of SHC1 significantly decreases compared to a control value,
the expression level of IL6 significantly increases compared to a control value,
the expression level of CXCR4 significantly increases compared to a control value,
the expression level of COL4A3 significantly increases compared to a control value,
the expression level of NRP2 significantly decreases compared to a control value,
the expression level of MEIS1 significantly increases compared to a control value,
the expression level of ARHGAP22 significantly decreases compared to a control value,
the expression level of SCG2 significantly decreases compared to a control value,
the expression level of FGF9 significantly increases compared to a control value,
the expression level of PML significantly decreases compared to a control value,
the expression level of FGFR1 significantly increases compared to a control value,
the expression level of FGFR4 significantly increases compared to a control value, and/or

the expression level of VEGFR1 significantly increases compared to a control value.

[Example 6] Correlation between the presence or absence of a mutation or loss of expression in BRAF and PTEN and expressions of FGFR3 and FGFR2

In human melanoma cell lines, the correlation between the presence or absence of a mutation or loss of expression μm in BRAF and PTEN and the expressions of FGFR3 and FGFR2 was investigated by a quantitative RT-PCR method.

Human melanoma cell lines used in Example 1 were used to prepare human melanoma cell grafted mouse models in the same manner as in Example 2. Total RNA was prepared in the same method as in Example 1 (2) (i) and a quantitative RT-PCR was performed in the same method as in Example 4, Section 1. The name of genes of the angiogenesis factors and angiogenesis factor receptors used herein and the assay ID of the probe purchased are shown in Table 8 and the results are shown in Figure 7.

[Table 8]

Table 8 Angiogenesis factor and angiogenesis factor receptor quantified

| No. | Gene Name | Assay ID |
|---|---|---|
| 1 | beta-actin | Hs99999903_m1 |
| 2 | FGFR3 | Hs_00179829 |
| 3 | FGFR2 | Hs_00256527 |

As a result, the expression levels of FGFR3 and FGFR2 significantly decrease from the expression level of a case where BRAF has a mutation and PTEN is wild type. It was thus elucidated that the expression levels of FGFR3 and FGFR2 significantly change when melanoma cells were classified based on the presence or absence of a mutation or loss of expression in BRAF and PTEN (Figure 7).

More specifically, it was suggested that if the responsiveness of a subject to an angiogenesis inhibitor is high,

the expression level of FGFR2 significantly increases compared to a control value, and
the expression level of FGFR3 significantly increases compared to a control value. **Industrial Applicability**

The present invention provides a method for predicting the responsiveness of a subject to an angiogenesis inhibitor. The prediction results obtained by the method of the present invention can be used as information for selecting an angiogenesis inhibitor for treating a tumor.

**Sequence Listing Free Text**

SEQ ID NO: 1; B-Raf Polynucleotide Sequence, GenBank Accession No. NM_004333.4
SEQ ID NO: 2: B-Raf Amino Acid Sequence, GenBank Accession No. NP_004324.2
SEQ ID NO: 3: PTEN Polynucleotide Sequence, GenBank Accession No. NM_000314.4
SEQ ID NO: 4: PTEN Amino Acid Sequence, GenBank Accession No. NP_000305.3
SEQ ID NO: 5: SHC1 Polynucleotide Sequence, GenBank Accession No.NM_003029.4
SEQ ID NO: 6: SHC1 Amino Acid Sequence, GenBank Accession No. NP_003020.2
SEQ ID NO: 7: IL6 Polynucleotide Sequence, GenBank Accession No. NM_000600.3
SEQ ID NO: 8: IL6 Amino Acid Sequence, GenBank Accession No. NP_000591.1
SEQ ID NO: 9: CXCR4 Polynucleotide Sequence, GenBank Accession No.NM_001008540.1
SEQ ID NO: 10: CXCR4 Amino Acid Sequence, GenBank Accession No.NP_001008540.1
SEQ ID NO: 11: COL4A3 Polynucleotide Sequence, GenBank Accession No.NM_000091.4
SEQ ID NO: 12: COL4A3 Amino Acid Sequence, GenBank Accession No.NP_000082.2
SEQ ID NO:13: NRP2 Polynucleotide Sequence, GenBank Accession No.NM_003872.2
SEQ ID NO: 14: NRP2 Amino Sequence, GenBank Accession No. NP_003863.2
SEQ ID NO: 15: MEIS1 Polynucleotide Sequence, GenBank Acoession No.NM _002398.2
SEQ ID NO: 16: MEIS1 Amino Acid Sequence, GenBank Accession No.NP_002389.1
SEQ ID NO: 17: ARHGAP22 Polynucleotide Sequence, GenBank Accession No.NM_021226.2

SEQ ID NO: 18: ARHGAP22 Amino Acid Sequence, GenBank Accession No.NP_067049.2
SEQ ID NO: 19-44: Synthetic DNA
SEQ ID NO: 45: ANG1 Polynucleotide Sequence, GenBank Accession No. NM_001146.3
SEQ ID NO: 46: ANG1 Amino Acid Sequence, GemBank Accession No. NP_001137.2
SEQ ID NO: 47: ANG2 Polynucleotide Sequence, GenBank Accession No. NM_001118888.1
SEQ ID NO: 48: ANG2 Amino Acid Sequence, GenBank Accession No. NP_001112360.1
SEQ ID NO: 49: SCG2 Polynucleotide Sequence, GenBankAccssion No.NM_003469.4
SEQ ID NO: 50: SCG2 Amino Acid Sequence, GenBank Accession No.NP_003460.2
SEQ ID NO: 51: FGF9 Polynucleotide Sequence, GenBank Accession No.NM_002010.2
SEQ ID NO: 52: FGF9 Amino Acid Sequence, GenBank Accession No.NP_002001.1
SEQ ID NO: 53: PML Polynucleotide Sequence, GenBank Accession No.NM_002675.3
SEQ ID NO: 54: RML Amino Acid Sequence, GenBank Accession No.NP_002666.1
SEQ ID NO: 55: FGFR3 Polynucleotide Sequence, GenBank Accession No. NM_000142.3
SEQ ID NO: 56: FGFR3 Amino Acid Sequence, GenBank Accession No. NP_000133.1
SEQ ID NO: 57: FGFR2 polynucleotide Sequence, GenBank Accession No. 7.NM_001144918.1
SEQ ID NO:58: FGFR2 Amino Acid Sequence, GenBank Accession No. NP_001138390.1
SEQ ID NO: 59: FGPR1 Polynucleotide Sequence, GenBank Accession No. NM_001174063.1
SEQ ID NO: 60: FGFR1 Amino Acid Sequence, GenBank Accession No. NP_001167534.1
SEQ ID NO: 61: FGFR4 Polynucleotide Sequence, GenBank Accession No. NM_002011.3
SEQ ID NO: 62: FGFR4 Amino Acid Sequence, GenBank Accession No. NP_002002.3
SEQ ID NO: 63: VEGFR1 Polynucleotide Sequence, GenBank Accession No. NM_001159920.1
SEQ ID NO: 64: VEGFR1 Amino Acid Sequence, GenBank Accession No. NP_001153392.1

**Claims**

**1.** A method for predicting the responsiveness of a subject suffering from a tumor to an angiogenesis inhibitor, comprising

(a) detecting the presence or absence of a mutation or loss of expression of B-Raf and the presence or absence of a mutation or loss of expression of PTEN in a sample derived from a tumor tissue of the subject, wherein in the detection step, a case where

(a1) B-Raf is wild type and PTEN is wild type, or
(a2) B-Raf has at least one mutation selected from Table 1 or loss of expression and PTEN has at least one mutation selected from Table 2 or loss of expression

is indicative of the high responsiveness of the subject to the angiogenesis inhibitor, wherein the angiogenesis inhibitor is 4-(3-chloro-4-(cyclopropylaminocarbonyl)aminophenoxy)-7-methoxy-6-quinolinecarboxamide or a pharmacologically acceptable salt thereof, and wherein the tumor is melanoma.

**2.** The method according to Claim 1, wherein, in the detection step (a), a case where B-Raf is wild type and PTEN is wild type is indicative of the high responsiveness of the subject to the angiogenesis inhibitor.

**3.** The method according to Claim 1, wherein, in the detection step (a), a case where B-Raf has at least one mutation selected from Table 1 or loss of expression and PTEN has at least one mutation selected from Table 2 or loss of expression is indicative of the high responsiveness of the subject to the angiogenesis inhibitor.

**4.** The method according to Claim 1 or Claim 3, wherein the mutation of B-Raf is a V600E mutation in an amino acid sequence or a mutation in a nucleotide sequence corresponding to the mutation.

**5.** The method according to Claim 1 or Claim 3, wherein the mutation of PTEN is at least one mutation in a nucleotide sequence selected from the group consisting of A499G, T202C and T335A or at least one mutation in an amino acid sequence selected from the group consisting of T167A, Y68H andL112Q.

**6.** The method according to any one of Claims 1 to 5, wherein the angiogenesis inhibitor is a mesylate salt of 4-(3-chloro-4-(cyclopropylaminocarbonyl)aminophenoxy)-7-methoxy-6-quinolinecarboxamide.

**7.** The method according to any one of Claims 1 to 6, wherein the tumor is a tumor having a V600E mutation in B-Raf.

**8.** The method according to any one of Claims 1 to 7, wherein, in the step (a), the high responsiveness of the subject to the angiogenesis inhibitor is predicted; and the method further comprises a step (b) of quantifying expression levels of at least one selected from the group consisting of ANG1 and ANG2 in the sample derived from the tumor tissue of the subject, wherein, in the quantification step, a case where

(b1) the expression level of ANG 1 is low compared to a control value
(b2) the expression level of ANG2 is high compared to a control value, or
(b3) the ratio of the expression levels of ANG1 and ANG2 is low compared to a control value

is indicative of the high responsiveness of the subject to the angiogenesis inhibitor.

**9.** The method according to any one of Claims 1 to 7, wherein, in the step (a), the high responsiveness of the subject to the angiogenesis inhibitor is predicted; and the method further comprises a step (c) of quantifying an expression level of at least one selected from the group consisting of SHC1, IL6, CXCR4, COL4A3, NRP2, MEIS1, ARHGAP22, SCG2, FGF9, PML, FGFR3, FGFR2, FGFR1, FGFR4 and VEGFR1 in the sample derived from the tumor tissue of the subject, wherein, in the quantification step, a case where

(c1) the expression level of SHC1 is low compared to a control value,
(c2) the expression level of NRP2 is low compared to a control value,
(c3) the expression level of ARHGAP22 is low compared to a control value,
(c4) the expression level of SCG2 is low compared to a control value,
(c5) the expression level of PML is low compared to a control value,
(c6) the expression level of IL6 is high compared to a control value,
(c7) the expression level of CXCR4 is high compared to a control value,
(c8) the expression level of COL4A3 is high compared to a control value,
(c9) the expression level of MEIS1 is high compared to a control value,
(c 10) the expression level of FGF9 is high compared to a control value,
(c11) the expression level of FGFR3 is high compared to a control value,
(c12) the expression level of FGFR2 is high compared to a control value,
(c 13) the expression level of FGFR1 is high compared to a control value,
(c14) the expression level of FGFR4 is high compared to a control value, or
(c 15) the expression level of VEGFR1 is high compared to a control value

is indicative of the high responsiveness of the subject to the angiogenesis inhibitor.

10. A method for predicting the responsiveness of a subject suffering from a tumor to an angiogenesis inhibitor, comprising

(b) quantifying expression levels of at least one selected from the group consisting of ANG1 and ANG2 in a sample derived from a tumor tissue of the subject, wherein, in the quantification step, a case where

(b 1) the expression level of ANG1 is low compared to a control value
(b2) the expression level of ANG2 is high compared to a control value, or
(b3) the ratio of expression level of ANG1 and ANG2 is low compared to a control value

is indicative of the high responsiveness of the subject to the angiogenesis inhibitor, wherein the angiogenesis inhibitor is 4-(3-chloro-4-(cyclopropylaminocarbonyl)aminophenoxy)-7-methoxy-6-quinolinecarboxamide or a pharmacologically acceptable salt thereof and wherein the tumor is melanoma.

11. The method according to Claim 10, wherein, in the step (b), high responsiveness of the subject to the angiogenesis inhibitor is predicted, and the method further comprises a step (c) of quantifying an expression level of at least one selected from the group consisting of SHC1, IL6, CXCR4, COL4A3, NRP2, MEIS1, ARHGAP22, SCG2, FGF9, PML, FGFR3, FGFR2, FGFR1, FGFR4 and VEGFR1 in the sample derived from the tumor tissue of the subject, wherein, in the quantification step, a case where

(c1) the expression level of SHC1 is low compared to a control value,
(c2) the expression level of NRP2 is low compared to a control value,
(c3) the expression level of ARHGAP22 is low compared to a control value,
(c4) the expression level of SCG2 is low compared to a control value,
(c5) the expression level of PML is low compared to a control value,
(c6) the expression level of IL6 is high compared to a control value,
(c7) the expression level of CXCR4 is high compared to a control value,
(c8) the expression level of COL4A3 is high compared to a control value,
(c9) the expression level of MEIS1 is high compared to a control value,
(c10) the expression level of FGF9 is high compared to a control value,
(c11) the expression level of FGFR3 is high compared to a control value,
(c12) the expression level of FGFR2 is high compared to a control value,
(c13) the expression level of FGFR1 is high compared to a control value,
(c14) the expression level of FGFR4 is high compared to a control value, or
(c15) the expression level of VEGFR1 is high compared to a control value

is indicative of the high responsiveness of the subject to the angiogenesis inhibitor.

12. A pharmaceutical composition comprising an angiogenesis inhibitor for use in treating a subject suffering from melanoma, wherein the subject has been predicted to be highly responsive to the angiogenesis inhibitor by the method according to any one of Claims 1 to 11, and wherein the angiogenesis inhibitor is 4-(3-chloro-4-(cyclopropylaminocarbonyl)aminophenoxy)-7-methoxy-6-quinolinecarboxamide or a pharmacologically acceptable salt thereof.

13. The pharmaceutical composition for use according to Claim 12, wherein the angiogenesis inhibitor is a mesylate salt of 4-(3-chloro-4-(cyclopropylaminocarbonyl)aminophenoxy)-7-methoxy-6-quinolinecarboxamide.

14. A use of a kit comprising probes of B-Raf and PTEN or probes of ANG1 and ANG2 for predicting the responsiveness of a subject suffering from melanoma to an angiogenesis inhibitor, wherein the responsiveness of the subject suffering from melanoma to the angiogenesis inhibitor is predicted by the method according to any one of Claims 1 to 11, and wherein the angiogenesis inhibitor is 4-(3-chloro-4-(cyclopropylaminocarbonyl)aminophenoxy)-7-methoxy-6-quinolinecarboxamide or a pharmacologically acceptable salt thereof.

## Patentansprüche

1. Verfahren zum Vorhersagen der Empfindlichkeit eines an einem Tumor leidenden Subjekts gegenüber einem Angiogeneseinhibitor, umfassend

(a) Detektieren der Anwesenheit oder Abwesenheit einer Mutation oder Expressionsverlust von B-Raf und der Anwesenheit oder Abwesenheit einer Mutation oder Expressionsverlust von PTEN in einer Probe, abgeleitet von einem Tumorgewebe des Subjekts, wobei im Detektionsschritt ein Fall, wo

(a1) B-Raf Wildtyp und PTEN Wildtyp ist oder
(a2) B-Raf wenigstens eine aus Tabelle 1 ausgewählte Mutation oder Expressionsverlust aufweist und PTEN wenigstens eine aus Tabelle 2 ausgewählte Mutation oder Expressionsverlust aufweist,

indikativ für die hohe Empfindlichkeit des Subjekts gegenüber dem Angiogeneseinhibitor ist, wobei der Angiogeneseinhibitor 4-(3-Chlor-4-(Cyclopropylaminocarbonyl)Aminophenoxy)-7-Methoxy-6-Quinolincarboxamid oder ein pharmakologisch geeignetes Salz davon ist, und wobei der Tumor Melanom ist.

**2.** Verfahren gemäß Anspruch 1, wobei im Detektionsschritt (a) ein Fall, wo B-Raf Wildtyp und PTEN Wildtyp ist, indikativ für die hohe Empfindlichkeit des Subjekts gegenüber dem Angiogeneseinhibitor ist.

**3.** Verfahren gemäß Anspruch 1, wobei im Detektionsschritt (a) ein Fall, wo B-Raf wenigstens eine aus Tabelle 1 ausgewählte Mutation oder Expressionsverlust aufweist und PTEN wenigstens eine aus Tabelle 2 ausgewählte Mutation oder Expressionsverlust aufweist,
indikativ für die hohe Empfindlichkeit des Subjekts gegenüber dem Angiogeneseinhibitor ist.

**4.** Verfahren gemäß Anspruch 1 oder Anspruch 3, wobei die Mutation von B-Raf eine V600E-Mutation in einer Aminosäuresequenz ist oder eine Mutation in einer Nukleotidsequenz, die der Mutation entspricht.

**5.** Verfahren gemäß Anspruch 1 oder Anspruch 3, wobei die Mutation von PTEN wenigstens eine Mutation in einer Nukleotidsequenz, ausgewählt aus der Gruppe, bestehend aus A499G, T202C und T335A, oder wenigstens eine Mutation in einer Aminosäuresequenz, ausgewählt aus der Gruppe, bestehend aus T167A, Y68H und L112Q, ist.

**6.** Verfahren gemäß einem der Ansprüche 1 bis 5, wobei der Angiogeneseinhibitor ein Mesylatsalz von 4-(3-Chlor-4-(Cyclopropylaminocarbonyl)Aminophenoxy)-7-Methoxy-6-Quinolincarboxamid ist.

**7.** Verfahren gemäß einem der Ansprüche 1 bis 6, wobei der Tumor ein Tumor mit einer V600E-Mutation in B-Raf ist.

**8.** Verfahren gemäß einem der Ansprüche 1 bis 7, wobei im Schritt (a) die hohe Empfindlichkeit des Subjekts gegenüber dem Angiogeneseinhibitor vorhergesagt wird; und das Verfahren weiterhin einen Schritt (b) des Quantifizierens von Expressionsniveaus von wenigstens einem, ausgewählt aus der Gruppe, bestehend aus ANG1 und ANG2, in der vom Tumorgewebe des Subjekts abgeleiteten Probe umfasst, wobei im Schritt des Quantifizierens ein Fall, wo

(b1) das Expressionsniveau von ANG1 niedrig ist im Vergleich zu einem Kontrollwert,
(b2) das Expressionsniveau von ANG2 hoch ist im Vergleich zu einem Kontrollwert oder
(b3) das Verhältnis der Expressionsniveaus von ANG1 und ANG2 niedrig ist im Vergleich zu einem Kontrollwert,
indikativ für die hohe Empfindlichkeit des Subjekts gegenüber dem Angiogeneseinhibitor ist.

**9.** Verfahren gemäß einem der Ansprüche 1 bis 7, wobei im Schritt (a) die hohe Empfindlichkeit des Subjekts gegenüber dem Angiogeneseinhibitor vorhergesagt wird; und das Verfahren weiterhin einen Schritt (c) des Quantifizierens eines Expressionsniveaus von wenigstens einem, ausgewählt aus der Gruppe, bestehend aus SHC1, IL6, CXCR4, COL4A3, NRP2, MEIS1, ARGHAP22, SCG2, FGF9, PML, FGFR3, FGFR2, FGFR1, FGFR4 und VEGFR1, in der vom Tumorgewebe des Subjekts abgeleiteten Probe umfasst, wobei im Schritt des Quantifizierens ein Fall, wo (c1) das Expressionsniveau von SHC1 niedrig ist im Vergleich zu einem Kontrollwert,

(c2) das Expressionsniveau von NRP2 niedrig ist im Vergleich zu einem Kontrollwert,
(c3) das Expressionsniveau von ARGHAP22 niedrig ist im Vergleich zu einem Kontrollwert,
(c4) das Expressionsniveau von SCG2 niedrig ist im Vergleich zu einem Kontrollwert,
(c5) das Expressionsniveau von PML niedrig ist im Vergleich zu einem Kontrollwert,
(c6) das Expressionsniveau von IL6 hoch ist im Vergleich zu einem Kontrollwert,
(c7) das Expressionsniveau von CXCR4 hoch ist im Vergleich zu einem Kontrollwert,
(c8) das Expressionsniveau von COL4A3 hoch ist im Vergleich zu einem Kontrollwert,
(c9) das Expressionsniveau von MEIS1 hoch ist im Vergleich zu einem Kontrollwert,
(c10) das Expressionsniveau von FGF9 hoch ist im Vergleich zu einem Kontrollwert,

(c11) das Expressionsniveau von FGFR3 hoch ist im Vergleich zu einem Kontrollwert,
(c12) das Expressionsniveau von FGFR2 hoch ist im Vergleich zu einem Kontrollwert,
(c13) das Expressionsniveau von FGFR1 hoch ist im Vergleich zu einem Kontrollwert,
(c14) das Expressionsniveau von FGFR4 hoch ist im Vergleich zu einem Kontrollwert, oder
(c15) das Expressionsniveau von VEGFR1 hoch ist im Vergleich zu einem Kontrollwert,

indikativ für die hohe Empfindlichkeit des Subjekts gegenüber dem Angiogeneseinhibitor ist.

**10.** Verfahren zum Vorhersagen der Empfindlichkeit eines an einem Tumor leidenden Subjekts gegenüber einem Angiogeneseinhibitor, umfassend

(b) Quantifizieren von Expressionsniveaus von wenigstens einem, ausgewählt aus der Gruppe, bestehend aus ANG1 und ANG2, in einer von einem Tumorgewebe des Subjekts abgeleiteten Probe umfasst, wobei im Schritt des Quantifizierens ein Fall, wo

(b1) das Expressionsniveau von ANG1 niedrig ist im Vergleich zu einem Kontrollwert,
(b2) das Expressionsniveau von ANG2 hoch ist im Vergleich zu einem Kontrollwert oder
(b3) das Verhältnis der Expressionsniveaus von ANG1 und ANG2 niedrig ist im Vergleich zu einem Kontrollwert,

indikativ für die hohe Empfindlichkeit des Subjekts gegenüber dem Angiogeneseinhibitor ist, wobei der Angiogeneseinhibitor 4-(3-Chlor-4-(Cyclopropylaminocarbonyl)Aminophenoxy)-7-Methoxy-6-Quinolincarboxamid oder ein pharmakologisch geeignetes Salz davon ist, und wobei der Tumor Melanom ist.

**11.** Verfahren gemäß Anspruch 10, wobei im Schritt (b) hohe Empfindlichkeit des Subjekts gegenüber dem Angiogeneseinhibitor vorhergesagt wird, und das Verfahren weiterhin einen Schritt (c) des Quantifizierens eines Expressionsniveaus von wenigstens einem, ausgewählt aus der Gruppe, bestehend aus SHC1, IL6, CXCR4, COL4A3, NRP2, MEIS1, ARGHAP22, SCG2, FGF9, PML, FGFR3, FGFR2, FGFR1, FGFR4 und VEGFR1, in der vom Tumorgewebe des Subjekts abgeleiteten Probe umfasst, wobei im Schritt des Quantifizierens ein Fall, wo

(c1) das Expressionsniveau von SHC1 niedrig ist im Vergleich zu einem Kontrollwert,
(c2) das Expressionsniveau von NRP2 niedrig ist im Vergleich zu einem Kontrollwert,
(c3) das Expressionsniveau von ARGHAP22 niedrig ist im Vergleich zu einem Kontrollwert,
(c4) das Expressionsniveau von SCG2 niedrig ist im Vergleich zu einem Kontrollwert,
(c5) das Expressionsniveau von PML niedrig ist im Vergleich zu einem Kontrollwert,
(c6) das Expressionsniveau von IL6 hoch ist im Vergleich zu einem Kontrollwert,
(c7) das Expressionsniveau von CXCR4 hoch ist im Vergleich zu einem Kontrollwert,
(c8) das Expressionsniveau von COL4A3 hoch ist im Vergleich zu einem Kontrollwert,
(c9) das Expressionsniveau von MEIS1 hoch ist im Vergleich zu einem Kontrollwert,
(c10) das Expressionsniveau von FGF9 hoch ist im Vergleich zu einem Kontrollwert,
(c11) das Expressionsniveau von FGFR3 hoch ist im Vergleich zu einem Kontrollwert,
(c12) das Expressionsniveau von FGFR2 hoch ist im Vergleich zu einem Kontrollwert,
(c13) das Expressionsniveau von FGFR1 hoch ist im Vergleich zu einem Kontrollwert,
(c14) das Expressionsniveau von FGFR4 hoch ist im Vergleich zu einem Kontrollwert, oder
(c15) das Expressionsniveau von VEGFR1 hoch ist im Vergleich zu einem Kontrollwert,

indikativ für die hohe Empfindlichkeit des Subjekts gegenüber dem Angiogeneseinhibitor ist.

**12.** Pharmazeutische Zusammensetzung, umfassend einen Angiogeneseinhibitor zur Verwendung bei der Behandlung eines an Melanom leidenden Subjekts, wobei durch das Verfahren gemäß einem der Ansprüche 1 bis 11 vorhergesagt wurde, dass das Subjekt hochgradig empfindlich gegenüber dem Angiogeneseinhibitor ist, und wobei der Angiogeneseinhibitor 4-(3-Chlor-4-(Cyclopropylaminocarbonyl)Aminophenoxy)-7-Methoxy-6-Quinolincarboxamid oder ein pharmakologisch geeignetes Salz davon ist.

**13.** Pharmazeutische Zusammensetzung zur Verwendung gemäß Anspruch 12, wobei der Angiogeneseinhibitor ein Mesylatsalz von 4-(3-Chlor-4-(Cyclopropylaminocarbonyl)Aminophenoxy)-7-Methoxy-6-Quinolincarboxamid ist.

**14.** Verwendung eines Kits, umfassend Sonden von B-Raf und PTEN oder Sonden von ANG1 und ANG2 für das

Vorhersagen der Empfindlichkeit eines an Melanom leidenden Subjekts gegenüber einem Angiogeneseinhibitor, wobei die Empfindlichkeit des an Melanom leidenden Subjekts gegenüber dem Angiogeneseinhibitor durch das Verfahren gemäß einem der Ansprüche 1 bis 11 vorhergesagt wurde, und wobei der Angiogeneseinhibitor 4-(3-Chlor-4-(Cyclopropylaminocarbonyl)Aminophenoxy)-7-Methoxy-6-Quinolincarboxamid oder ein pharmakologisch geeignetes Salz davon ist.

**Revendications**

1. Procédé de prédiction de la réponse d'un sujet souffrant d'une tumeur à un inhibiteur de l'angiogenèse, comprenant

(a) la détection de la présence ou l'absence d'une mutation ou d'une perte d'expression de B-Raf et de la présence ou l'absence d'une mutation ou d'une perte d'expression de PTEN dans un échantillon dérivé d'un tissu tumoral du sujet, dans lequel dans l'étape de détection, un cas où

(a1) B-Raf est de type sauvage et PTEN et de type sauvage, ou
(a2) B-Raf a au moins une mutation sélectionnée dans le Tableau 1 ou une perte d'expression et PTEN a au moins une mutation sélectionnée dans le Tableau 2 ou une perte d'expression

est indicatif de la réponse élevée du sujet à l'inhibiteur de l'angiogenèse, dans lequel l'inhibiteur de l'angiogenèse est du 4-(3-chloro-4-(cyclopropylaminocarbonyl)aminophénoxy)-7-méthoxy-6-quinolinecarboxamide ou un sel de celui-ci acceptable d'un point de vue pharmaceutique, et dans lequel la tumeur est un mélanome.

2. Procédé selon la revendication 1, dans lequel, dans l'étape de détection (a), un cas où B-Raf est de type sauvage et PTEN est de type sauvage est indicatif de la réponse élevée du sujet à l'inhibiteur de l'angiogenèse.

3. Procédé selon la revendication 1, dans lequel, dans l'étape de détection (a), un cas où B-Raf a au moins une mutation sélectionnée dans le Tableau 1 ou une perte d'expression et PTEN a au moins une mutation sélectionnée dans le Tableau 2 ou une perte d'expression est indicatif de la réponse élevée du sujet à l'inhibiteur de l'angiogenèse.

4. Procédé selon la revendication 1 ou la revendication 3, dans lequel la mutation de B-Raf est une mutation V600E dans une séquence d'acides aminés ou une mutation dans une séquence nucléotidique correspondant à la mutation.

5. Procédé selon la revendication 1 ou la revendication 3, dans laquelle la mutation de PTEN est au moins une mutation dans une séquence nucléotidique sélectionnée dans le groupe constitué d'A499G, de T202C et de T335A ou au moins une mutation dans une séquence d'acides aminés sélectionnée dans le groupe constitué de T167A, de Y68H et de L112Q.

6. Procédé selon l'une quelconque des revendications 1 à 5, dans lequel l'inhibiteur de l'angiogenèse est un sel de mésylate de 4-(3-chloro-4-(cyclopropylaminocarbonyl)aminophénoxy)-7-méthoxy-6-quinolinecarboxamide.

7. Procédé selon l'une quelconque des revendications 1 à 6, dans lequel la tumeur est une tumeur ayant une mutation V600E dans B-Raf.

8. Procédé selon l'une quelconque des revendications 1 à 7, dans lequel, dans l'étape (a), la réponse élevée du sujet à l'inhibiteur de l'angiogenèse est prédite; et le procédé comprend en outre une étape (b) de quantification des niveaux d'expression d'au moins un sélectionné dans le groupe constitué d'ANG1 et d'ANG2 dans l'échantillon dérivé du tissu tumoral du sujet, dans lequel, dans l'étape de quantification, un cas où

(b1) le niveau d'expression d'ANG1 est bas par rapport à une valeur témoin
(b2) le niveau d'expression d'ANG2 est élevé par rapport à une valeur témoin, ou
(b3) le rapport des niveaux d'expression d'ANG1 et d'ANG2 est bas par rapport à une valeur témoin

est indicatif de la réponse élevée du sujet à l'inhibiteur de l'angiogenèse.

9. Procédé selon l'une quelconque des revendications 1 à 7, dans lequel, dans l'étape (a), la réponse élevée du sujet à l'inhibiteur de l'angiogenèse est prédite; et le procédé comprend en outre une étape (c) de quantification d'un niveau d'expression d'au moins un sélectionné dans le groupe constitué de SHC1, d'IL6, de CXCR4, de COL4A3,

de NRP2, de MEIS1, d'ARHGAP22, de SCG2, de FGF9, de PML, de FGFR3, de FGFR2, de FGFR1, de FGFR4 et de VEGFR1 dans l'échantillon dérivé du tissu tumoral du sujet, dans lequel, l'étape de quantification, un cas où

(c1) le niveau d'expression de SHC1 est bas par rapport à une valeur témoin
(c2) le niveau d'expression de NRP2 est bas par rapport à une valeur témoin
(c3) le niveau d'expression d'ARHGAP22 est bas par rapport à une valeur témoin
(c4) le niveau d'expression de SCG2 est bas par rapport à une valeur témoin
(c5) le niveau d'expression de PML est bas par rapport à une valeur témoin
(c6) le niveau d'expression d'IL6 est élevé par rapport à une valeur témoin
(c7) le niveau d'expression de CXCR4 est élevé par rapport à une valeur témoin
(c8) le niveau d'expression de COL4A3 est élevé par rapport à une valeur témoin
(c9) le niveau d'expression de MEIS1 est élevé par rapport à une valeur témoin
(c10) le niveau d'expression de FGF9 est élevé par rapport à une valeur témoin
(c11) le niveau d'expression de FGFR3 est élevé par rapport à une valeur témoin
(c12) le niveau d'expression de FGFR2 est élevé par rapport à une valeur témoin
(c13) le niveau d'expression de FGFR1 est élevé par rapport à une valeur témoin
(c14) le niveau d'expression de FGFR4 est élevé par rapport à une valeur témoin, ou
(c15) le niveau d'expression de VEGFR1 est élevé par rapport à une valeur témoin

est indicatif de la réponse élevée du sujet à l'inhibiteur de l'angiogenèse.

**10.** Procédé de prédiction de la réponse d'un sujet souffrant d'une tumeur à un inhibiteur de l'angiogenèse, comprenant

(b) la quantification des niveaux d'expression d'au moins un sélectionné dans le groupe constitué d'ANG1 et d'ANG2 dans un échantillon dérivé d'un tissu tumoral d'un sujet, dans lequel, dans l'étape de quantification, un cas où

(b1) le niveau d'expression d'ANG1 est bas par rapport à une valeur témoin
(b2) le niveau d'expression d'ANG2 est élevé par rapport à une valeur témoin, ou
(b3) le rapport du niveau d'expression d'ANG1 et d'ANG2 est bas par rapport à une valeur témoin

est indicatif de la réponse élevée du sujet à l'inhibiteur de l'angiogenèse, dans lequel l'inhibiteur de l'angiogenèse est du 4-(3-chloro-4-(cyclopropylaminocarbonyl)aminophénoxy)-7-méthoxy-6-quinolinecarboxamide ou un sel de celui-ci acceptable d'un point de vue pharmaceutique et dans lequel la tumeur est un mélanome.

**11.** Procédé selon la revendication 10, dans lequel, dans l'étape (b), la réponse élevée du sujet à l'inhibiteur de l'angiogenèse est prédite, et le procédé comprend en outre une étape (c) de quantification du niveau d'expression d'au moins un sélectionné dans le groupe constitué de SHC1, d'IL6, de CXCR4, de COL4A3, de NRP2, de MEIS1, d'ARHGAP22, de SCG2, de FGF9, de PML, de FGFR3, de FGFR2, de FGFR1, de FGFR4 et de VEGFR1 dans l'échantillon dérivé du tissu tumoral du sujet, dans lequel, dans l'étape de quantification, un cas où

(c1) le niveau d'expression de SHC1 est bas par rapport à une valeur témoin
(c2) le niveau d'expression de NRP2 est bas par rapport à une valeur témoin
(c3) le niveau d'expression d'ARHGAP22 est bas par rapport à une valeur témoin
(c4) le niveau d'expression de SCG2 est bas par rapport à une valeur témoin
(c5) le niveau d'expression de PML est bas par rapport à une valeur témoin
(c6) le niveau d'expression d'IL6 est élevé par rapport à une valeur témoin
(c7) le niveau d'expression de CXCR4 est élevé par rapport à une valeur témoin
(c8) le niveau d'expression de COL4A3 est élevé par rapport à une valeur témoin
(c9) le niveau d'expression de MEIS1 est élevé par rapport à une valeur témoin
(c10) le niveau d'expression de FGF9 est élevé par rapport à une valeur témoin
(c11) le niveau d'expression de FGFR3 est élevé par rapport à une valeur témoin
(c12) le niveau d'expression de FGFR2 est élevé par rapport à une valeur témoin
(c13) le niveau d'expression de FGFR1 est élevé par rapport à une valeur témoin
(c14) le niveau d'expression de FGFR4 est élevé par rapport à une valeur témoin, ou
(c15) le niveau d'expression de VEGFR1 est élevé par rapport à une valeur témoin

est indicatif de la réponse élevée du sujet à l'inhibiteur de l'angiogenèse.

**12.** Composition pharmaceutique comprenant un inhibiteur de l'angiogenèse pour une utilisation dans le traitement d'un sujet souffrant d'un mélanome, dans laquelle le sujet est prédit répondre de manière élevée à l'inhibiteur de l'angiogenèse par le procédé selon l'une quelconque des revendications 1 à 11, et dans laquelle l'inhibiteur de l'angiogenèse est du 4-(3-chloro-4-(cyclopropylaminocarbonyl)aminophénoxy)-7-méthoxy-6-quinolinecarboxamide ou un sel de celui-ci acceptable d'un point de vue pharmaceutique.

**13.** Composition pharmaceutique pour une utilisation selon la revendication 12, dans laquelle l'inhibiteur de l'angiogenèse est un sel de mésylate de 4-(3-chloro-4-(cyclopropylaminocarbonyl)aminophénoxy)-7-méthoxy-6-quinolinecarboxamide.

**14.** Utilisation d'un kit comprenant des sondes de B-Raf et de PTEN ou des sondes d'ANG1 et d'ANG2 pour prédire la réponse d'un sujet souffrant d'un mélanome à un inhibiteur de l'angiogenèse, dans laquelle la réponse du sujet souffrant d'un mélanome à l'inhibiteur de l'angiogenèse est prédite par le procédé selon l'une quelconque des revendications 1 à 11, et dans lequel l'inhibiteur de l'angiogenèse est du 4-(3-chloro-4-(cyclopropylaminocarbonyl)aminophénoxy)-7-méthoxy-6-quinolinecarboxamide ou un sel de celui-ci acceptable d'un point de vue pharmaceutique.

Fig.1

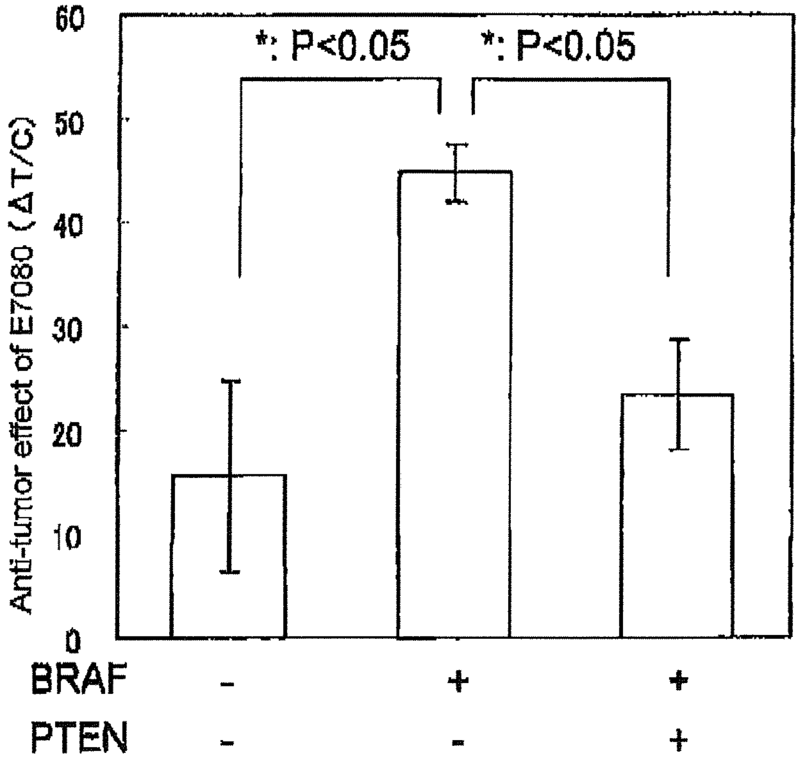
*: P<0.05
*: P<0.05
60
50
40
30
20
10
0
Anti-tumor effect of E7080 (ΔT/C)
BRAF - + +
PTEN - - +

*Fig.2*

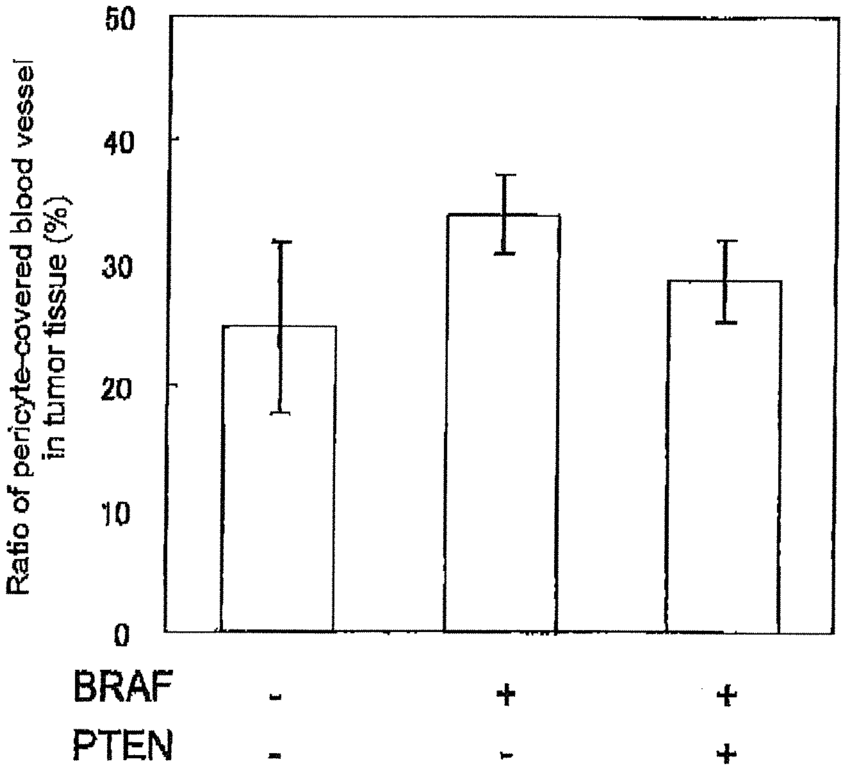
50
40
30
20
10
0
Ratio of pericyte-covered blood vessel in tumor tissue (%)
BRAF - + +
PTEN - - +

*Fig.3*

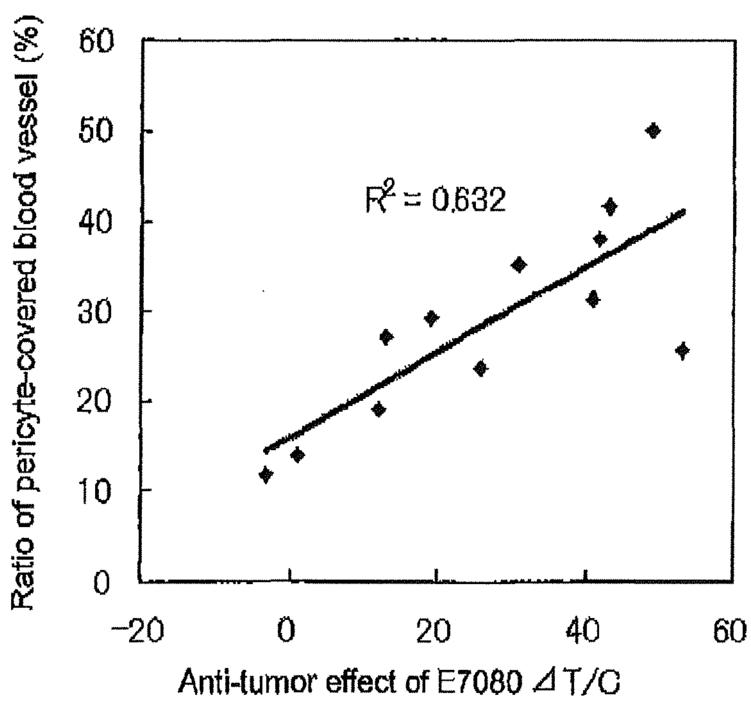
Ratio of pericyte-covered blood vessel (%)
60
50
40
30
20
10
0
$R^2$ = 0.632
-20
0
20
40
60
Anti-tumor effect of E7080 ⊿T/C

*Fig.4*

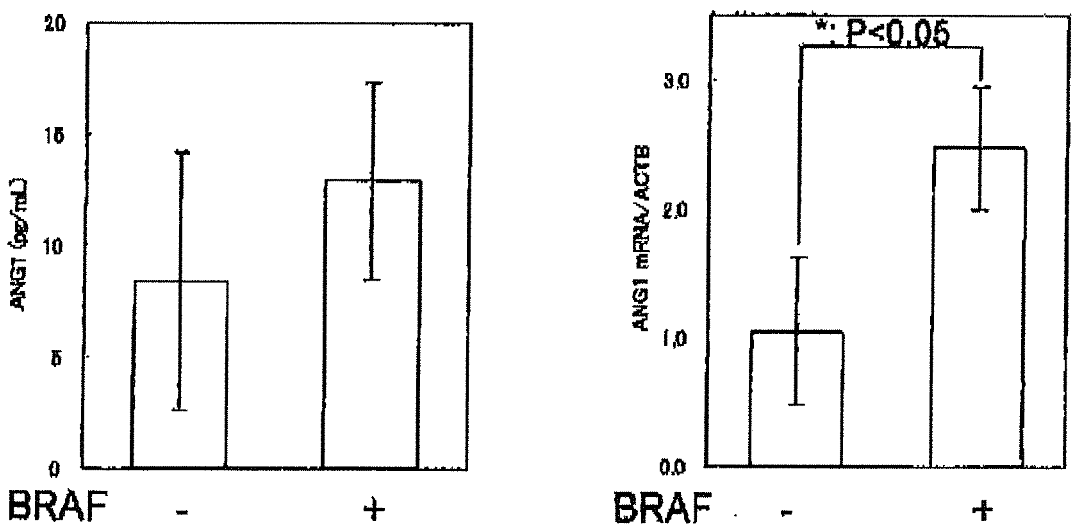


(a) (b)

*Fig.5*

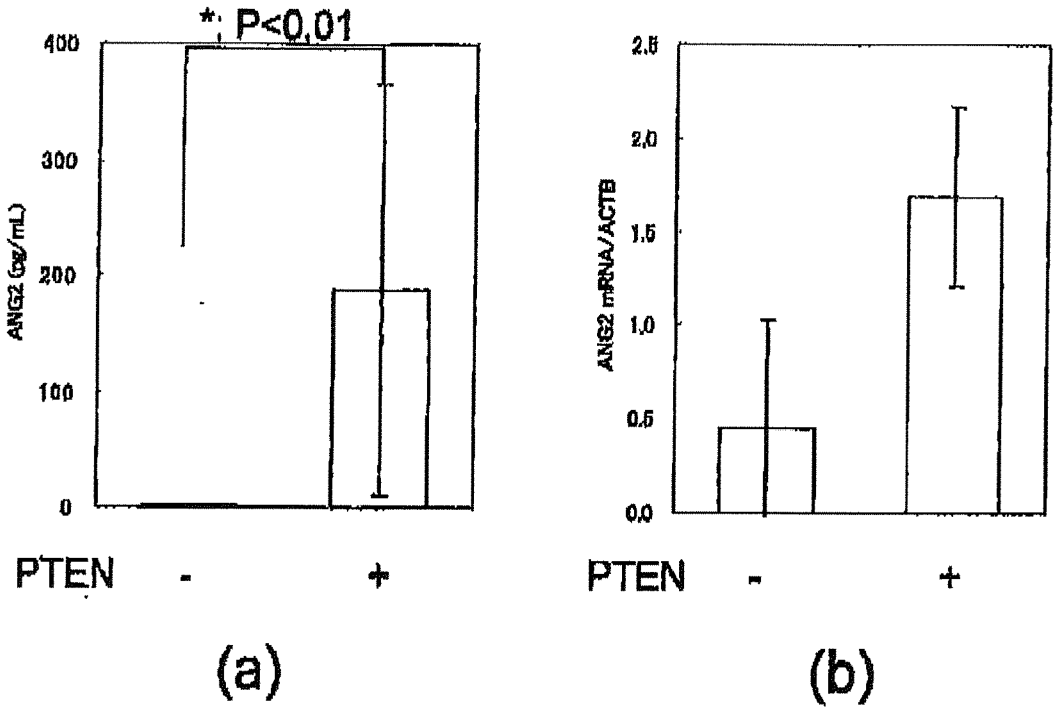
*: P<0.01
400
300
200
100
0
ANG2 (pg/mL)
PTEN
-
+
(a)
2.5
2.0
1.5
1.0
0.5
0.0
ANG2 mRNA/ACTB
PTEN
-
+
(b)

*Fig.6*

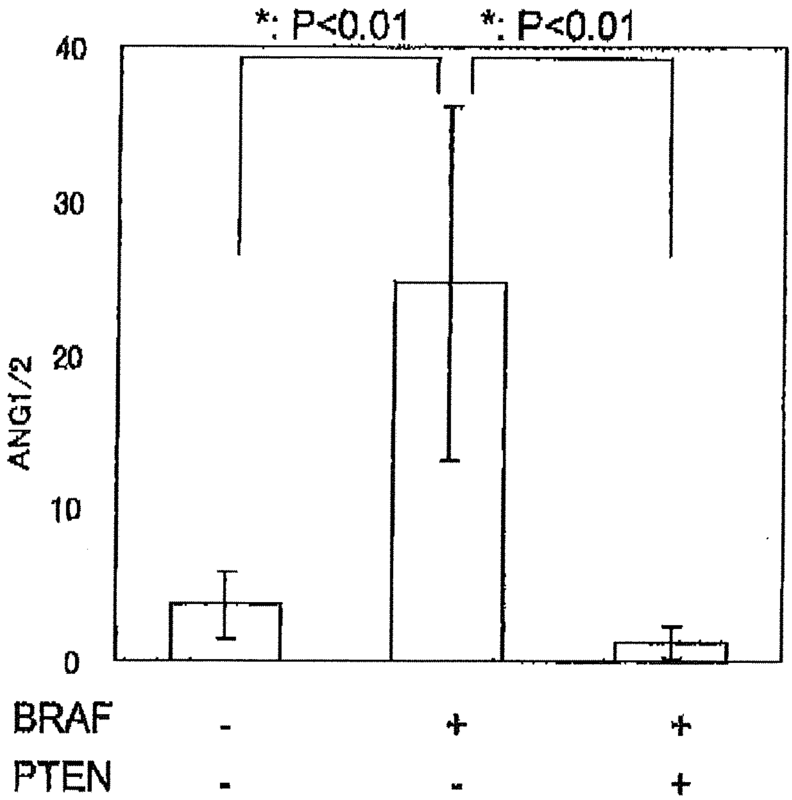

*Fig.7*

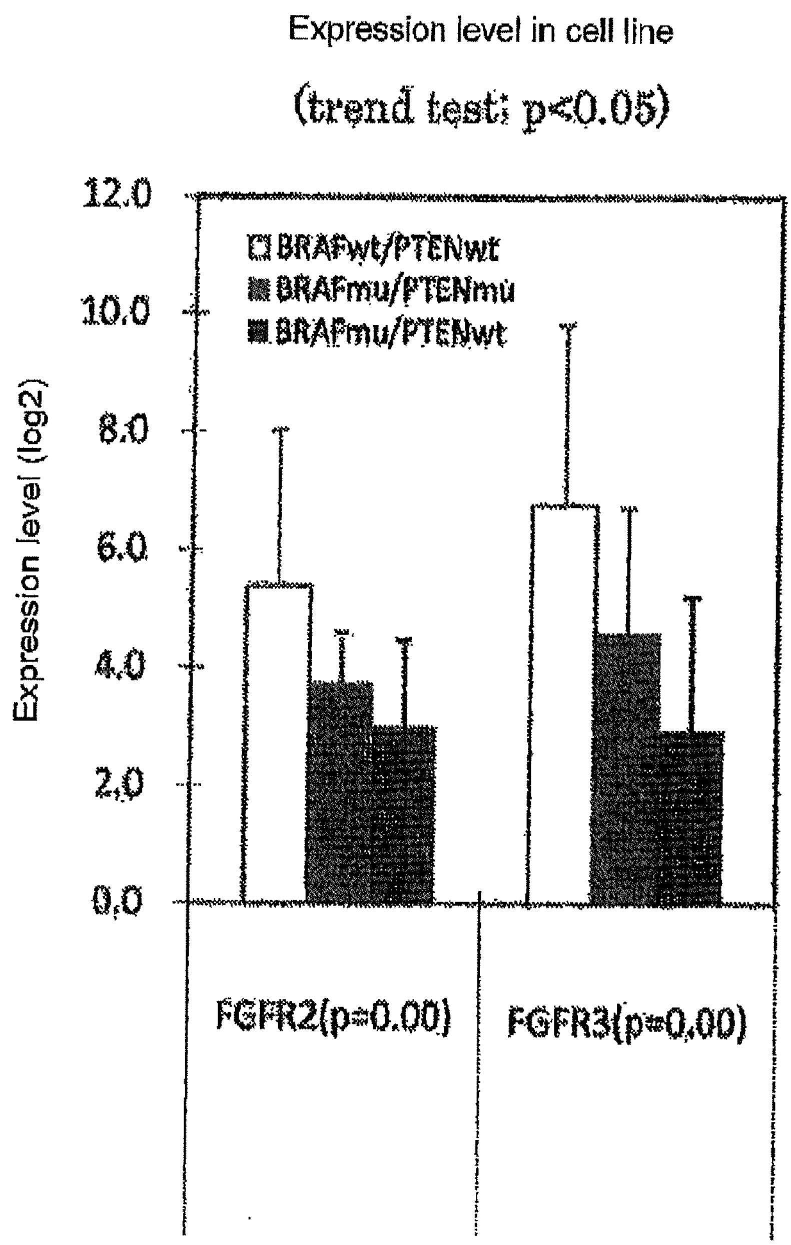
Expression level in cell line
(trend test: p<0.05)
12.0
10.0
8.0
6.0
4.0
2.0
0.0
Expression level (log2)
BRAFwt/PTENwt
BRAFmu/PTENmu
BRAFmu/PTENwt
FGFR2(p=0.00)
FGFR3(p=0.00)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02032872 A
- WO 2007136103 A
- JP 2011110884 A


### Non-patent literature cited in the description


- **MATSUI et al.** *Clinical Cancer Research,* 2008, vol. 14 (17), 5459-5465
- **DAVIES et al.** *Nature,* 2002, vol. 417, 949-954
- **BESSON et al.** *European Journal of Biochemistry,* 1999, vol. 263, 605-611
- Antisense Drug Technology: Principles, Strategies, and Applications. CRC Press, 2007
- RNA Methodologies. Elsevier, 2005
- **KAIHATSU et al.** *Chemistry & Biology,* 2004, vol. 11 (6), 749-758
- **LADNER et al.** *Drug Discovery Today,* 2004, vol. 9, 525-529
- Laser Capture Microdissection: Methods and Protocols. Humana Press, 2004
- **KITAGO et al.** *Clinical Chemistry,* 2009, vol. 55 (4), 757-764
- **CHUANG et al.** *Head and Neck,* 2010, vol. 32, 229-234
- Genome Analyzer II (Illumina), Applied Biosystems SOLiD System (Applied Biosystems) and HeliScope Single Molecule Sequencer (Helicos). Current Protocols in Molecular Biology. John Wiley & Sons, 2010
- **GABRIEL et al.** Current Protocols in Human Genomics. John Wiley & Sons, 2009
- **SCHENA et al.** *Science,* 1995, vol. 270, 467-470
- **LOCKHART et al.** *Nature Biotechnology,* 1996, vol. 14, 1675-1680

SEQUENCE LISTING

<110> Eisai R&D Management Co., Ltd.

<120> A method of predicting an effect of antiangiogenic agent

<130> PCT12-0011

<150> JP 2011-110884
<151> 2011-05-17

<160> 64

<170> PatentIn version 3.4

<210> 1
<211> 2301
<212> DNA
<213> Homo sapiens

<400> 1

```
atggcggcgc tgagcggtgg cggtggtggc ggcgcggagc cgggccaggc tctgttcaac     60
ggggacatgg agcccgaggc cggcgccggc gccggcgccg cggcctcttc ggctgcggac    120
cctgccattc cggaggaggt gtggaatatc aaacaaatga ttaagttgac acaggaacat    180
atagaggccc tattggacaa atttggtggg gagcataatc caccatcaat atatctggag    240
gcctatgaag aatacaccag caagctagat gcactccaac aaagagaaca acagttattg    300
gaatctctgg ggaacggaac tgatttttct gtttctagct ctgcatcaat ggataccgtt    360
acatcttctt cctcttctag cctttcagtg ctaccttcat ctctttcagt ttttcaaaat    420
cccacagatg tggcacggag caaccccaag tcaccacaaa aacctatcgt tagagtcttc    480
ctgcccaaca aacagaggac agtggtacct gcaaggtgtg gagttacagt ccgagacagt    540
ctaaagaaag cactgatgat gagaggtcta atcccagagt gctgtgctgt ttacagaatt    600
caggatggag agaagaaacc aattggttgg gacactgata tttcctggct tactggagaa    660
gaattgcatg tggaagtgtt ggagaatgtt ccacttacaa cacacaactt tgtacgaaaa    720
acgtttttca ccttagcatt ttgtgacttt tgtcgaaagc tgcttttcca gggtttccgc    780
tgtcaaacat gtggttataa atttcaccag cgttgtagta cagaagttcc actgatgtgt    840
gttaattatg accaacttga tttgctgttt gtctccaagt tctttgaaca ccacccaata    900
ccacaggaag aggcgtcctt agcagagact gccctaacat ctggatcatc cccttccgca    960
cccgcctcgg actctattgg gccccaaatt ctcaccagtc cgtctccttc aaaatccatt   1020
ccaattccac agcccttccg accagcagat gaagatcatc gaaatcaatt tgggcaacga   1080
gaccgatcct catcagctcc caatgtgcat ataaacacaa tagaacctgt caatattgat   1140
gacttgatta gagaccaagg atttcgtggt gatggaggat caaccacagg tttgtctgct   1200
acccccccctg cctcattacc tggctcacta actaacgtga aagccttaca gaaatctcca   1260
```

```
ggacctcagc gagaaaggaa gtcatcttca tcctcagaag acaggaatcg aatgaaaaca   1320
cttggtagac gggactcgag tgatgattgg gagattcctg atgggcagat tacagtggga   1380
caaagaattg gatctggatc atttggaaca gtctacaagg gaaagtggca tggtgatgtg   1440
gcagtgaaaa tgttgaatgt gacagcacct acacctcagc agttacaagc cttcaaaaat   1500
gaagtaggag tactcaggaa aacacgacat gtgaatatcc tactcttcat gggctattcc   1560
acaaagccac aactggctat tgttacccag tggtgtgagg gctccagctt gtatcaccat   1620
ctccatatca ttgagaccaa atttgagatg atcaaactta tagatattgc acgacagact   1680
gcacagggca tggattactt acacgccaag tcaatcatcc acagagacct caagagtaat   1740
aatatatttc ttcatgaaga cctcacagta aaaataggtg attttggtct agctacagtg   1800
aaatctcgat ggagtgggtc ccatcagttt gaacagttgt ctggatccat tttgtggatg   1860
gcaccagaag tcatcagaat gcaagataaa aatccataca gctttcagtc agatgtatat   1920
gcatttggaa ttgttctgta tgaattgatg actggacagt taccttattc aaacatcaac   1980
aacagggacc agataatttt tatggtggga cgaggatacc tgtctccaga tctcagtaag   2040
gtacggagta actgtccaaa agccatgaag agattaatgg cagagtgcct caaaaagaaa   2100
agagatgaga gaccactctt tccccaaatt ctcgcctcta ttgagctgct ggcccgctca   2160
ttgccaaaaa ttcaccgcag tgcatcagaa ccctccttga atcgggctgg tttccaaaca   2220
gaggatttta gtctatatgc ttgtgcttct ccaaaaacac ccatccaggc agggggatat   2280
ggtgcgtttc ctgtccactg a                                             2301
```

<210> 2
<211> 766
<212> PRT
<213> Homo sapiens

<400> 2

```
Met Ala Ala Leu Ser Gly Gly Gly Gly Gly Gly Ala Glu Pro Gly Gln
1               5                   10                  15

Ala Leu Phe Asn Gly Asp Met Glu Pro Glu Ala Gly Ala Gly Ala Gly
            20                  25                  30

Ala Ala Ala Ser Ser Ala Ala Asp Pro Ala Ile Pro Glu Glu Val Trp
        35                  40                  45

Asn Ile Lys Gln Met Ile Lys Leu Thr Gln Glu His Ile Glu Ala Leu
    50                  55                  60

Leu Asp Lys Phe Gly Gly Glu His Asn Pro Pro Ser Ile Tyr Leu Glu
65                  70                  75                  80
```

```
Ala Tyr Glu Glu Tyr Thr Ser Lys Leu Asp Ala Leu Gln Gln Arg Glu
                85                  90                  95

Gln Gln Leu Leu Glu Ser Leu Gly Asn Gly Thr Asp Phe Ser Val Ser
            100                 105                 110

Ser Ser Ala Ser Met Asp Thr Val Thr Ser Ser Ser Ser Ser Ser Leu
        115                 120                 125

Ser Val Leu Pro Ser Ser Leu Ser Val Phe Gln Asn Pro Thr Asp Val
    130                 135                 140

Ala Arg Ser Asn Pro Lys Ser Pro Gln Lys Pro Ile Val Arg Val Phe
145                 150                 155                 160

Leu Pro Asn Lys Gln Arg Thr Val Val Pro Ala Arg Cys Gly Val Thr
                165                 170                 175

Val Arg Asp Ser Leu Lys Lys Ala Leu Met Met Arg Gly Leu Ile Pro
            180                 185                 190

Glu Cys Cys Ala Val Tyr Arg Ile Gln Asp Gly Glu Lys Lys Pro Ile
        195                 200                 205

Gly Trp Asp Thr Asp Ile Ser Trp Leu Thr Gly Glu Glu Leu His Val
    210                 215                 220

Glu Val Leu Glu Asn Val Pro Leu Thr Thr His Asn Phe Val Arg Lys
225                 230                 235                 240

Thr Phe Phe Thr Leu Ala Phe Cys Asp Phe Cys Arg Lys Leu Leu Phe
                245                 250                 255

Gln Gly Phe Arg Cys Gln Thr Cys Gly Tyr Lys Phe His Gln Arg Cys
            260                 265                 270

Ser Thr Glu Val Pro Leu Met Cys Val Asn Tyr Asp Gln Leu Asp Leu
        275                 280                 285

Leu Phe Val Ser Lys Phe Phe Glu His His Pro Ile Pro Gln Glu Glu
    290                 295                 300

Ala Ser Leu Ala Glu Thr Ala Leu Thr Ser Gly Ser Ser Pro Ser Ala
305                 310                 315                 320

Pro Ala Ser Asp Ser Ile Gly Pro Gln Ile Leu Thr Ser Pro Ser Pro
                325                 330                 335
```

```
Ser Lys Ser Ile Pro Ile Pro Gln Pro Phe Arg Pro Ala Asp Glu Asp
            340                 345                 350

His Arg Asn Gln Phe Gly Gln Arg Asp Arg Ser Ser Ser Ala Pro Asn
        355                 360                 365

Val His Ile Asn Thr Ile Glu Pro Val Asn Ile Asp Asp Leu Ile Arg
    370                 375                 380

Asp Gln Gly Phe Arg Gly Asp Gly Gly Ser Thr Thr Gly Leu Ser Ala
385                 390                 395                 400

Thr Pro Pro Ala Ser Leu Pro Gly Ser Leu Thr Asn Val Lys Ala Leu
                405                 410                 415

Gln Lys Ser Pro Gly Pro Gln Arg Glu Arg Lys Ser Ser Ser Ser Ser
            420                 425                 430

Glu Asp Arg Asn Arg Met Lys Thr Leu Gly Arg Arg Asp Ser Ser Asp
        435                 440                 445

Asp Trp Glu Ile Pro Asp Gly Gln Ile Thr Val Gly Gln Arg Ile Gly
    450                 455                 460

Ser Gly Ser Phe Gly Thr Val Tyr Lys Gly Lys Trp His Gly Asp Val
465                 470                 475                 480

Ala Val Lys Met Leu Asn Val Thr Ala Pro Thr Pro Gln Gln Leu Gln
                485                 490                 495

Ala Phe Lys Asn Glu Val Gly Val Leu Arg Lys Thr Arg His Val Asn
            500                 505                 510

Ile Leu Leu Phe Met Gly Tyr Ser Thr Lys Pro Gln Leu Ala Ile Val
        515                 520                 525

Thr Gln Trp Cys Glu Gly Ser Ser Leu Tyr His His Leu His Ile Ile
    530                 535                 540

Glu Thr Lys Phe Glu Met Ile Lys Leu Ile Asp Ile Ala Arg Gln Thr
545                 550                 555                 560

Ala Gln Gly Met Asp Tyr Leu His Ala Lys Ser Ile Ile His Arg Asp
                565                 570                 575

Leu Lys Ser Asn Asn Ile Phe Leu His Glu Asp Leu Thr Val Lys Ile
```

```
            580                 585                 590

Gly Asp Phe Gly Leu Ala Thr Val Lys Ser Arg Trp Ser Gly Ser His
        595                 600                 605

Gln Phe Glu Gln Leu Ser Gly Ser Ile Leu Trp Met Ala Pro Glu Val
    610                 615                 620

Ile Arg Met Gln Asp Lys Asn Pro Tyr Ser Phe Gln Ser Asp Val Tyr
625                 630                 635                 640

Ala Phe Gly Ile Val Leu Tyr Glu Leu Met Thr Gly Gln Leu Pro Tyr
                645                 650                 655

Ser Asn Ile Asn Asn Arg Asp Gln Ile Ile Phe Met Val Gly Arg Gly
            660                 665                 670

Tyr Leu Ser Pro Asp Leu Ser Lys Val Arg Ser Asn Cys Pro Lys Ala
        675                 680                 685

Met Lys Arg Leu Met Ala Glu Cys Leu Lys Lys Lys Arg Asp Glu Arg
    690                 695                 700

Pro Leu Phe Pro Gln Ile Leu Ala Ser Ile Glu Leu Leu Ala Arg Ser
705                 710                 715                 720

Leu Pro Lys Ile His Arg Ser Ala Ser Glu Pro Ser Leu Asn Arg Ala
                725                 730                 735

Gly Phe Gln Thr Glu Asp Phe Ser Leu Tyr Ala Cys Ala Ser Pro Lys
            740                 745                 750

Thr Pro Ile Gln Ala Gly Gly Tyr Gly Ala Phe Pro Val His
        755                 760                 765
```

<210> 3
<211> 1212
<212> DNA
<213> Homo sapiens

<400> 3

```
atgacagcca tcatcaaaga gatcgttagc agaaacaaaa ggagatatca agaggatgga      60
ttcgacttag acttgaccta tatttatcca aacattattg ctatgggatt tcctgcagaa     120
agacttgaag gcgtatacag gaacaatatt gatgatgtag taaggttttt ggattcaaag     180
cataaaaacc attacaagat atacaatctt tgtgctgaaa gacattatga caccgccaaa     240
tttaattgca gagttgcaca atatcctttt gaagaccata acccaccaca gctagaactt     300
atcaaaccct tttgtgaaga tcttgaccaa tggctaagtg aagatgacaa tcatgttgca     360
gcaattcact gtaaagctgg aaagggacga actggtgtaa tgatatgtgc atatttatta     420
catcggggca atttttaaa ggcacaagag gccctagatt tctatgggga agtaaggacc     480
agagacaaaa agggagtaac tattcccagt cagaggcgct atgtgtatta ttatagctac     540
ctgttaaaga atcatctgga ttatagacca gtggcactgt tgtttcacaa gatgatgttt     600
gaaactattc caatgttcag tggcggaact tgcaatcctc agtttgtggt ctgccagcta     660
aaggtgaaga tatattcctc caattcagga cccacacgac gggaagacaa gttcatgtac     720
tttgagttcc ctcagccgtt acctgtgtgt ggtgatatca aagtagagtt cttccacaaa     780
cagaacaaga tgctaaaaaa ggacaaaatg tttcactttt gggtaaatac attcttcata     840
ccaggaccag aggaaacctc agaaaaagta gaaaatggaa gtctatgtga tcaagaaatc     900
gatagcattt gcagtataga gcgtgcagat aatgacaagg aatatctagt acttacttta     960
acaaaaaatg atcttgacaa agcaaataaa gacaaagcca accgatactt ttctccaaat    1020
tttaaggtga agctgtactt cacaaaaaca gtagaggagc cgtcaaatcc agaggctagc    1080
agttcaactt ctgtaacacc agatgttagt gacaatgaac ctgatcatta tagatattct    1140
gacaccactg actctgatcc agagaatgaa ccttttgatg aagatcagca tacacaaatt    1200
acaaaagtct ga                                                        1212
```

<210> 4
<211> 403
<212> PRT
<213> Homo sapiens

<400> 4

```
Met Thr Ala Ile Ile Lys Glu Ile Val Ser Arg Asn Lys Arg Arg Tyr
1               5                   10                  15

Gln Glu Asp Gly Phe Asp Leu Asp Leu Thr Tyr Ile Tyr Pro Asn Ile
            20                  25                  30

Ile Ala Met Gly Phe Pro Ala Glu Arg Leu Glu Gly Val Tyr Arg Asn
        35                  40                  45

Asn Ile Asp Asp Val Val Arg Phe Leu Asp Ser Lys His Lys Asn His
    50                  55                  60

Tyr Lys Ile Tyr Asn Leu Cys Ala Glu Arg His Tyr Asp Thr Ala Lys
65                  70                  75                  80

Phe Asn Cys Arg Val Ala Gln Tyr Pro Phe Glu Asp His Asn Pro Pro
                85                  90                  95
```

Gln Leu Glu Leu Ile Lys Pro Phe Cys Glu Asp Leu Asp Gln Trp Leu
100 105 110

Ser Glu Asp Asp Asn His Val Ala Ala Ile His Cys Lys Ala Gly Lys
115 120 125

Gly Arg Thr Gly Val Met Ile Cys Ala Tyr Leu Leu His Arg Gly Lys
130 135 140

Phe Leu Lys Ala Gln Glu Ala Leu Asp Phe Tyr Gly Glu Val Arg Thr
145 150 155 160

Arg Asp Lys Lys Gly Val Thr Ile Pro Ser Gln Arg Arg Tyr Val Tyr
165 170 175

Tyr Tyr Ser Tyr Leu Leu Lys Asn His Leu Asp Tyr Arg Pro Val Ala
180 185 190

Leu Leu Phe His Lys Met Met Phe Glu Thr Ile Pro Met Phe Ser Gly
195 200 205

Gly Thr Cys Asn Pro Gln Phe Val Val Cys Gln Leu Lys Val Lys Ile
210 215 220

Tyr Ser Ser Asn Ser Gly Pro Thr Arg Arg Glu Asp Lys Phe Met Tyr
225 230 235 240

Phe Glu Phe Pro Gln Pro Leu Pro Val Cys Gly Asp Ile Lys Val Glu
245 250 255

Phe Phe His Lys Gln Asn Lys Met Leu Lys Lys Asp Lys Met Phe His
260 265 270

Phe Trp Val Asn Thr Phe Phe Ile Pro Gly Pro Glu Glu Thr Ser Glu
275 280 285

Lys Val Glu Asn Gly Ser Leu Cys Asp Gln Glu Ile Asp Ser Ile Cys
290 295 300

Ser Ile Glu Arg Ala Asp Asn Asp Lys Glu Tyr Leu Val Leu Thr Leu
305 310 315 320

Thr Lys Asn Asp Leu Asp Lys Ala Asn Lys Asp Lys Ala Asn Arg Tyr
325 330 335

Phe Ser Pro Asn Phe Lys Val Lys Leu Tyr Phe Thr Lys Thr Val Glu
340 345 350

```
Glu Pro Ser Asn Pro Glu Ala Ser Ser Ser Thr Ser Val Thr Pro Asp
        355                 360                 365

Val Ser Asp Asn Glu Pro Asp His Tyr Arg Tyr Ser Asp Thr Thr Asp
    370                 375                 380

Ser Asp Pro Glu Asn Glu Pro Phe Asp Glu Asp Gln His Thr Gln Ile
385                 390                 395                 400

Thr Lys Val
```

<210> 5
<211> 1425
<212> DNA
<213> Homo sapiens

<400> 5

```
atgaacaagc tgagtggagg cggcgggcgc aggactcggg tggaaggggg ccagcttggg      60
ggcgaggagt ggacccgcca cgggagcttt gtcaataagc ccacgcgggg ctggctgcat     120
cccaacgaca aagtcatggg acccggggtt tcctacttgg ttcggtacat gggttgtgtg     180
gaggtcctcc agtcaatgcg tgccctggac ttcaacaccc ggactcaggt caccagggag     240
gccatcagtc tggtgtgtga ggctgtgccg ggtgctaagg gggcgacaag gaggagaaag     300
ccctgtagcc gcccgctcag ctctatcctg ggaggagta acctgaaatt tgctggaatg      360
ccaatcactc tcaccgtctc caccagcagc ctcaacctca tggccgcaga ctgcaaacag     420
atcatcgcca accaccacat gcaatctatc tcatttgcat ccggcgggga tccggacaca     480
gccgagtatg tcgcctatgt tgccaaagac cctgtgaatc agagagcctg ccacattctg     540
gagtgtcccg aagggcttgc ccaggatgtc atcagcacca ttggccaggc cttcgagttg     600
cgcttcaaac aatacctcag gaacccaccc aaactggtca cccctcatga caggatggct     660
ggctttgatg gctcagcatg ggatgaggag gaggaagagc cacctgacca tcagtactat     720
aatgacttcc cggggaagga acccccttg gggggggtgg tagacatgag gcttcgggaa      780
ggagccgctc caggggctgc tcgacccact gcacccaatg cccagacccc cagccacttg     840
ggagctacat tgcctgtagg acagcctgtt gggggagatc cagaagtccg caaacagatg     900
ccacctccac caccctgtcc agcaggcaga gagcttttg atgatccctc ctatgtcaac      960
gtccagaacc tagacaaggc ccggcaagca gtgggtggtg ctgggccccc caatcctgct    1020
atcaatggca gtgcaccccg ggacctgttt gacatgaagc ccttcgaaga tgctcttcgc    1080
gtgcctccac ctccccagtc ggtgtccatg gctgagcagc tccgagggga gccctggttc    1140
catgggaagc tgagccggcg ggaggctgag gcactgctgc agctcaatgg ggacttcctg    1200
```

```
gtacgggaga gcacgaccac acctggccag tatgtgctca ctggcttgca gagtgggcag   1260

cctaagcatt tgctactggt ggaccctgag ggtgtggttc ggactaagga tcaccgcttt   1320

gaaagtgtca gtcaccttat cagctaccac atggacaatc acttgcccat catctctgcg   1380

ggcagcgaac tgtgtctaca gcaacctgtg gagcggaaac tgtga                   1425
```

<210> 6
<211> 474
<212> PRT
<213> Homo sapiens

<400> 6

```
Met Asn Lys Leu Ser Gly Gly Gly Gly Arg Arg Thr Arg Val Glu Gly
1               5                   10                  15

Gly Gln Leu Gly Gly Glu Glu Trp Thr Arg His Gly Ser Phe Val Asn
            20                  25                  30

Lys Pro Thr Arg Gly Trp Leu His Pro Asn Asp Lys Val Met Gly Pro
        35                  40                  45

Gly Val Ser Tyr Leu Val Arg Tyr Met Gly Cys Val Glu Val Leu Gln
    50                  55                  60

Ser Met Arg Ala Leu Asp Phe Asn Thr Arg Thr Gln Val Thr Arg Glu
65                  70                  75                  80

Ala Ile Ser Leu Val Cys Glu Ala Val Pro Gly Ala Lys Gly Ala Thr
                85                  90                  95

Arg Arg Arg Lys Pro Cys Ser Arg Pro Leu Ser Ser Ile Leu Gly Arg
            100                 105                 110

Ser Asn Leu Lys Phe Ala Gly Met Pro Ile Thr Leu Thr Val Ser Thr
        115                 120                 125

Ser Ser Leu Asn Leu Met Ala Ala Asp Cys Lys Gln Ile Ile Ala Asn
    130                 135                 140

His His Met Gln Ser Ile Ser Phe Ala Ser Gly Gly Asp Pro Asp Thr
145                 150                 155                 160

Ala Glu Tyr Val Ala Tyr Val Ala Lys Asp Pro Val Asn Gln Arg Ala
                165                 170                 175

Cys His Ile Leu Glu Cys Pro Glu Gly Leu Ala Gln Asp Val Ile Ser
            180                 185                 190
```

```
Thr Ile Gly Gln Ala Phe Glu Leu Arg Phe Lys Gln Tyr Leu Arg Asn
        195                 200                 205

Pro Pro Lys Leu Val Thr Pro His Asp Arg Met Ala Gly Phe Asp Gly
    210                 215                 220

Ser Ala Trp Asp Glu Glu Glu Glu Glu Pro Pro Asp His Gln Tyr Tyr
225                 230                 235                 240

Asn Asp Phe Pro Gly Lys Glu Pro Pro Leu Gly Gly Val Val Asp Met
                245                 250                 255

Arg Leu Arg Glu Gly Ala Ala Pro Gly Ala Ala Arg Pro Thr Ala Pro
            260                 265                 270

Asn Ala Gln Thr Pro Ser His Leu Gly Ala Thr Leu Pro Val Gly Gln
        275                 280                 285

Pro Val Gly Gly Asp Pro Glu Val Arg Lys Gln Met Pro Pro Pro Pro
    290                 295                 300

Pro Cys Pro Ala Gly Arg Glu Leu Phe Asp Asp Pro Ser Tyr Val Asn
305                 310                 315                 320

Val Gln Asn Leu Asp Lys Ala Arg Gln Ala Val Gly Gly Ala Gly Pro
                325                 330                 335

Pro Asn Pro Ala Ile Asn Gly Ser Ala Pro Arg Asp Leu Phe Asp Met
            340                 345                 350

Lys Pro Phe Glu Asp Ala Leu Arg Val Pro Pro Pro Pro Gln Ser Val
        355                 360                 365

Ser Met Ala Glu Gln Leu Arg Gly Glu Pro Trp Phe His Gly Lys Leu
    370                 375                 380

Ser Arg Arg Glu Ala Glu Ala Leu Leu Gln Leu Asn Gly Asp Phe Leu
385                 390                 395                 400

Val Arg Glu Ser Thr Thr Thr Pro Gly Gln Tyr Val Leu Thr Gly Leu
                405                 410                 415

Gln Ser Gly Gln Pro Lys His Leu Leu Leu Val Asp Pro Glu Gly Val
            420                 425                 430

Val Arg Thr Lys Asp His Arg Phe Glu Ser Val Ser His Leu Ile Ser
```

```
                435                 440                 445

        Tyr His Met Asp Asn His Leu Pro Ile Ile Ser Ala Gly Ser Glu Leu
            450                 455                 460

        Cys Leu Gln Gln Pro Val Glu Arg Lys Leu
        465                 470
```

<210> 7
<211> 639
<212> DNA
<213> Homo sapiens

<400> 7

```
atgaactcct tctccacaag cgccttcggt ccagttgcct tctccctggg gctgctcctg      60
gtgttgcctg ctgccttccc tgccccagta cccccaggag aagattccaa agatgtagcc     120
gccccacaca gacagccact cacctcttca gaacgaattg acaaacaaat tcggtacatc     180
ctcgacggca tctcagccct gagaaaggag acatgtaaca agagtaacat gtgtgaaagc     240
agcaaagagg cactggcaga aaacaacctg aaccttccaa agatggctga aaaagatgga     300
tgcttccaat ctggattcaa tgaggagact tgcctggtga aaatcatcac tggtcttttg     360
gagtttgagg tatacctaga gtacctccag aacagatttg agagtagtga ggaacaagcc     420
agagctgtgc agatgagtac aaaagtcctg atccagttcc tgcagaaaaa ggcaaagaat     480
ctagatgcaa taaccacccc tgacccaacc acaaatgcca gcctgctgac gaagctgcag     540
gcacagaacc agtggctgca ggacatgaca actcatctca ttctgcgcag ctttaaggag     600
ttcctgcagt ccagcctgag ggctcttcgg caaatgtag                            639
```

<210> 8
<211> 212
<212> PRT
<213> Homo sapiens

<400> 8

```
Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala Pro Val Pro Pro
            20                  25                  30

Gly Glu Asp Ser Lys Asp Val Ala Ala Pro His Arg Gln Pro Leu Thr
        35                  40                  45

Ser Ser Glu Arg Ile Asp Lys Gln Ile Arg Tyr Ile Leu Asp Gly Ile
    50                  55                  60

Ser Ala Leu Arg Lys Glu Thr Cys Asn Lys Ser Asn Met Cys Glu Ser
65                  70                  75                  80

Ser Lys Glu Ala Leu Ala Glu Asn Asn Leu Asn Leu Pro Lys Met Ala
                85                  90                  95

Glu Lys Asp Gly Cys Phe Gln Ser Gly Phe Asn Glu Glu Thr Cys Leu
            100                 105                 110

Val Lys Ile Ile Thr Gly Leu Leu Glu Phe Glu Val Tyr Leu Glu Tyr
        115                 120                 125

Leu Gln Asn Arg Phe Glu Ser Ser Glu Glu Gln Ala Arg Ala Val Gln
    130                 135                 140

Met Ser Thr Lys Val Leu Ile Gln Phe Leu Gln Lys Lys Ala Lys Asn
145                 150                 155                 160

Leu Asp Ala Ile Thr Thr Pro Asp Pro Thr Thr Asn Ala Ser Leu Leu
                165                 170                 175

Thr Lys Leu Gln Ala Gln Asn Gln Trp Leu Gln Asp Met Thr Thr His
            180                 185                 190

Leu Ile Leu Arg Ser Phe Lys Glu Phe Leu Gln Ser Ser Leu Arg Ala
        195                 200                 205

Leu Arg Gln Met
    210
```

<210> 9
<211> 1071
<212> DNA
<213> Homo sapiens

<400> 9

```
atgtccattc ctttgcctct tttgcagata tacacttcag ataactacac cgaggaaatg     60
ggctcagggg actatgactc catgaaggaa ccctgtttcc gtgaagaaaa tgctaatttc    120
aataaaatct tcctgcccac catctactcc atcatcttct taactggcat tgtgggcaat    180
ggattggtca tcctggtcat gggttaccag aagaaactga gaagcatgac ggacaagtac    240
aggctgcacc tgtcagtggc cgacctcctc tttgtcatca cgcttccctt ctgggcagtt    300
gatgccgtgg caaactggta ctttgggaac ttcctatgca aggcagtcca tgtcatctac    360
acagtcaacc tctacagcag tgtcctcatc ctggccttca tcagtctgga ccgctacctg    420
gccatcgtcc acgccaccaa cagtcagagg ccaaggaagc tgttggctga aaaggtggtc    480
tatgttggcg tctggatccc tgccctcctg ctgactattc ccgacttcat ctttgccaac    540

gtcagtgagg cagatgacag atatatctgt gaccgcttct accccaatga cttgtgggtg    600
gttgtgttcc agtttcagca catcatggtt ggccttatcc tgcctggtat tgtcatcctg    660
tcctgctatt gcattatcat ctccaagctg tcacactcca agggccacca gaagcgcaag    720
gccctcaaga ccacagtcat cctcatcctg gctttcttcg cctgttggct gccttactac    780
attgggatca gcatcgactc cttcatcctc ctggaaatca tcaagcaagg gtgtgagttt    840
gagaacactg tgcacaagtg gatttccatc accgaggccc tagctttctt ccactgttgt    900
ctgaacccca tcctctatgc tttccttgga gccaaattta aaacctctgc ccagcacgca    960
ctcacctctg tgagcagagg gtccagcctc aagatcctct ccaaaggaaa gcgaggtgga   1020
cattcatctg tttccactga gtctgagtct tcaagttttc actccagcta a            1071
```

<210> 10
<211> 356
<212> PRT
<213> Homo sapiens

<400> 10

```
Met Ser Ile Pro Leu Pro Leu Leu Gln Ile Tyr Thr Ser Asp Asn Tyr
1               5                   10                  15

Thr Glu Glu Met Gly Ser Gly Asp Tyr Asp Ser Met Lys Glu Pro Cys
            20                  25                  30

Phe Arg Glu Glu Asn Ala Asn Phe Asn Lys Ile Phe Leu Pro Thr Ile
        35                  40                  45

Tyr Ser Ile Ile Phe Leu Thr Gly Ile Val Gly Asn Gly Leu Val Ile
    50                  55                  60

Leu Val Met Gly Tyr Gln Lys Lys Leu Arg Ser Met Thr Asp Lys Tyr
65                  70                  75                  80

Arg Leu His Leu Ser Val Ala Asp Leu Leu Phe Val Ile Thr Leu Pro
                85                  90                  95

Phe Trp Ala Val Asp Ala Val Ala Asn Trp Tyr Phe Gly Asn Phe Leu
            100                 105                 110

Cys Lys Ala Val His Val Ile Tyr Thr Val Asn Leu Tyr Ser Ser Val
        115                 120                 125

Leu Ile Leu Ala Phe Ile Ser Leu Asp Arg Tyr Leu Ala Ile Val His
    130                 135                 140
```

```
Ala Thr Asn Ser Gln Arg Pro Arg Lys Leu Leu Ala Glu Lys Val Val
145                 150                 155                 160

Tyr Val Gly Val Trp Ile Pro Ala Leu Leu Leu Thr Ile Pro Asp Phe
                165                 170                 175

Ile Phe Ala Asn Val Ser Glu Ala Asp Asp Arg Tyr Ile Cys Asp Arg
            180                 185                 190

Phe Tyr Pro Asn Asp Leu Trp Val Val Val Phe Gln Phe Gln His Ile
        195                 200                 205

Met Val Gly Leu Ile Leu Pro Gly Ile Val Ile Leu Ser Cys Tyr Cys
    210                 215                 220

Ile Ile Ile Ser Lys Leu Ser His Ser Lys Gly His Gln Lys Arg Lys
225                 230                 235                 240

Ala Leu Lys Thr Thr Val Ile Leu Ile Leu Ala Phe Phe Ala Cys Trp
                245                 250                 255

Leu Pro Tyr Tyr Ile Gly Ile Ser Ile Asp Ser Phe Ile Leu Leu Glu
            260                 265                 270

Ile Ile Lys Gln Gly Cys Glu Phe Glu Asn Thr Val His Lys Trp Ile
        275                 280                 285

Ser Ile Thr Glu Ala Leu Ala Phe Phe His Cys Cys Leu Asn Pro Ile
    290                 295                 300

Leu Tyr Ala Phe Leu Gly Ala Lys Phe Lys Thr Ser Ala Gln His Ala
305                 310                 315                 320

Leu Thr Ser Val Ser Arg Gly Ser Ser Leu Lys Ile Leu Ser Lys Gly
                325                 330                 335

Lys Arg Gly Gly His Ser Ser Val Ser Thr Glu Ser Glu Ser Ser Ser
            340                 345                 350

Phe His Ser Ser
        355
```

<210> 11
<211> 5013
<212> DNA
<213> Homo sapiens

<400> 11

atgagcgccc ggaccgcccc caggccgcag gtgctcctgc tgccgctcct gctggtgctc 60

```
ctggcggcgg cgcccgcagc cagcaagggt tgtgtctgta aagacaaagg ccagtgcttc    120
tgtgacgggg ccaaagggga gaagggggag aagggctttc ctggaccccc cggttctcct    180
ggccagaaag gattcacagg tcctgaaggc ttgcctggac cgcagggacc caagggcttt    240
ccaggacttc caggactcac gggttccaaa ggtgtaaggg gaataagtgg attgccagga    300
ttttctggtt ctcctggact tccaggcacc ccaggcaata ccgggcctta cggacttgtc    360
ggtgtaccag gatgcagtgg ttctaagggt gagcaggggt ttccaggact cccagggaca    420
ctgggctacc cagggatccc gggtgctgct ggtttgaaag gacaaaaggg tgctcctgct    480
aaagaagaag atatagaact tgatgcaaaa ggcgaccccg ggttgccagg ggctccagga    540
ccccagggtt tgccaggccc tccaggtttt cctgggcctg ttggcccacc tggtcctccg    600
ggattctttg gctttccagg agccatggga cctagaggac ctaagggtca catgggtgaa    660
agagtgatag gacataaagg agagcggggt gtgaaagggt taacaggacc cccgggacca    720
ccaggaacag ttattgtgac cctaactggc ccagataaca gaacggacct caaggggga    780
aagggagaca agggagcaat gggcgagcct ggacctcctg gaccctcagg actgcctgga    840
gaatcatatg gatctgaaaa gggtgctcct ggagaccctg gcctgcaggg aaaacccgga    900
aaagatggtg ttcctggctt ccctggaagt gagggagtca agggcaacag gggtttccct    960
gggttaatgg gtgaagatgg cattaaggga cagaaagggg acattggccc tccaggattt   1020
cgtggtccaa cagaatatta tgacacatac caggaaaagg gagatgaagg cactccaggc   1080
ccaccagggc ccagaggagc tcgtggccca caaggtccca gtggtccccc cggagttcct   1140
ggaagtcctg gatcatcaag gcctggcctc agaggagccc ctggatggcc aggcctgaaa   1200
ggaagtaaag gggaacgagg ccgcccagga aaggatgcca tggggactcc tgggtcccca   1260
ggttgtgctg gttcaccagg tcttccagga tcaccgggac ctccaggacc gccaggtgac   1320
atcgtttttc gcaagggtcc acctggagat cacggactgc caggctatct agggtctcca   1380
ggaatcccag gagttgatgg gcccaaagga gaaccaggcc tcctgtgtac acagtgccct   1440
tatatcccag ggcctcccgg tctcccagga ttgccagggt tacatggtgt aaaaggaatc   1500
ccaggaagac aaggcgcagc tggcttgaaa ggaagcccag ggtccccagg aaatacaggt   1560
cttccaggat ttccaggttt cccaggtgcc cagggtgacc caggacttaa aggagaaaaa   1620
ggtgaaacac ttcagcctga ggggcaagtg ggtgtcccag gtgacccggg gctcagaggc   1680
caacctggga gaaagggctt ggatggaatt cctggaactc cgggagtgaa aggattacca   1740
ggacctaaag gcgaactggc tctgagtggt gagaaagggg accaaggtcc tccaggggat   1800
cctggctccc ctgggtcccc aggacctgca ggaccagctg gaccacctgg ctacggaccc   1860
caaggagaac ctggtctcca gggcacgcaa ggagttcctg gagcccccgg accacccgga   1920
```

gaagccggcc ctaggggaga gctcagtgtt tcaacaccag ttccaggccc accaggacct 1980
ccagggcccc ctggccatcc tggcccccaa ggtccacctg gtatccctgg atccctgggg 2040
aaatgtggag atcctggtct tccagggcct gatggtgaac caggaattcc aggaattgga 2100
tttcctgggc ctcctggacc taagggagac caaggttttc caggtacaaa aggatcactg 2160
ggttgtcctg gaaaaatggg agagcctggg ttacctggaa agccaggcct cccaggagcc 2220
aagggagaac cagcagtagc catgcctgga ggaccaggaa caccaggttt tccaggagaa 2280
agaggcaatt ctggggaaca tggagaaatt ggactccctg gacttccagg tctccctgga 2340
actccaggaa atgaagggct tgatggacca cgaggagatc cagggcagcc tggaccacct 2400
ggagaacaag gacccccagg aaggtgcata gagggtccca ggggagccca aggacttcca 2460
ggcttaaatg gattgaaagg gcaacaaggc agaagaggta aaacggggcc aaagggagac 2520
ccaggaattc caggcttgga tagatcagga tttcctggag aaactggatc accaggaatt 2580
ccaggtcatc aaggtgaaat gggaccactg ggtcaaagag gatatccagg aaatccggga 2640
attttagggc caccaggtga agatggagtg attgggatga tgggctttcc tggagccatt 2700
ggccctccag ggccccctgg gaacccaggc acaccagggc agagggggag ccctggaatt 2760
ccaggagtaa agggccagag aggaacccca ggagccaagg gggaacaagg agataaagga 2820
aatcccgggc cttcagagat atcccacgta ataggggaca aaggagaacc aggtctcaaa 2880
ggattcgcag gaaatccagg tgagaaagga aacagaggcg ttccagggat gccaggttta 2940
aagggcctca aaggactacc cggaccagca ggaccaccag gccccagagg agatttgggc 3000
agcactggga atcctggaga accaggactg cgtggtatac caggaagcat ggggaacatg 3060
ggcatgccag gttctaaagg aaaaagggga actttgggat tcccaggtcg agcaggaaga 3120
ccaggcctcc caggtattca tggtctccag ggagataagg gagagccagg ttattcagaa 3180
ggtacaaggc caggaccacc gggaccaacg gggggatccag gactgccggg tgatatggga 3240
aagaaaggag aaatggggca acctggccca cctggacatt tggggcctgc tggacctgag 3300
ggagcccctg gaagtcctgg aagtcctggc ctcccaggaa agccaggtcc tcatggtgat 3360
ttgggtttta aaggaatcaa aggcctcctg ggccctccag gaatcagagg ccctccaggt 3420
cttccaggat ttccaggatc tcctggacca atgggtataa gaggtgacca aggacgtgat 3480
ggaattcctg gtccagccgg agaaaaggga gaaacgggtt tattgagggc ccctccaggc 3540
ccaagaggga accctggtgc tcaaggagcc aaaggagaca ggggagcccc aggttttcct 3600
ggcctcccgg gcagaaaagg ggccatggga gatgctggac ctcgaggacc cacaggcata 3660
gaaggattcc cagggccacc aggtctgccc ggtgcaatta tccctggcca gacaggaaat 3720
cgtggtccac caggctcaag aggaagccca ggtgcgcctg gtcccccctgg acctccaggg 3780
agtcatgtaa taggcataaa aggagacaaa gggtctatgg gccaccctgg cccaaaaggt 3840

```
ccacctggaa ctgcaggaga catgggacca ccaggtcgtc tgggagcacc aggtactcca   3900
ggtcttccag gacccagagg tgatcctgga ttccaggggt ttccaggcgt gaaaggagaa   3960
aagggtaatc ctggatttct aggatccatt ggacctccag gaccaattgg gccaaaagga   4020
ccacctggtg tacgtggaga ccctggcaca cttaagatta tctcccttcc aggaagccca   4080
gggccacctg gcacacctgg agaaccaggg atgcagggag aacctgggcc accagggcca   4140
cctggaaacc taggaccctg tgggccaaga ggtaagccag gcaaggatgg aaaaccagga   4200
actcctggac cagctggaga aaaaggcaac aaaggttcta aaggagagcc aggaccagct   4260
ggatcagatg gattgccagg tttgaaagga aaacgtggag acagtggatc acctgcaacc   4320
tggacaacga gaggctttgt cttcacccga cacagtcaaa ccacagcaat tccttcatgt   4380
ccagagggga cagtgccact ctacagtggg ttttcttttc tttttgtaca aggaaatcaa   4440
cgagcccacg gacaagacct tggaactctt ggcagctgcc tgcagcgatt taccacaatg   4500
ccattcttat tctgcaatgt caatgatgta tgtaattttg catctcgaaa tgattattca   4560
tactggctgt caacaccagc tctgatgcca atgaacatgg ctcccattac tggcagagcc   4620
cttgagcctt atataagcag atgcactgtt tgtgaaggtc ctgcgatcgc catagccgtt   4680
cacagccaaa ccactgacat tcctccatgt cctcacggct ggatttctct ctggaaagga   4740
ttttcattca tcatgttcac aagtgcaggt tctgagggca ccgggcaagc actggcctcc   4800
cctggctcct gcctggaaga attccgagcc agcccatttc tagaatgtca tggaagagga   4860
acgtgcaact actattcaaa ttcctacagt ttctggctgg cttcattaaa cccagaaaga   4920
atgttcagaa agcctattcc atcaactgtg aaagctgggg aattagaaaa aataataagt   4980
cgctgtcagg tgtgcatgaa gaaaagacac tga                                5013
```

<210> 12
<211> 1670
<212> PRT
<213> Homo sapiens

<400> 12

```
Met Ser Ala Arg Thr Ala Pro Arg Pro Gln Val Leu Leu Leu Pro Leu
1               5                   10                  15

Leu Leu Val Leu Leu Ala Ala Ala Pro Ala Ala Ser Lys Gly Cys Val
            20                  25                  30

Cys Lys Asp Lys Gly Gln Cys Phe Cys Asp Gly Ala Lys Gly Glu Lys
        35                  40                  45

Gly Glu Lys Gly Phe Pro Gly Pro Pro Gly Ser Pro Gly Gln Lys Gly
    50                  55                  60
```

Phe Thr Gly Pro Glu Gly Leu Pro Gly Pro Gln Gly Pro Lys Gly Phe
65                  70                  75                  80

Pro Gly Leu Pro Gly Leu Thr Gly Ser Lys Gly Val Arg Gly Ile Ser
                85                  90                  95

Gly Leu Pro Gly Phe Ser Gly Ser Pro Gly Leu Pro Gly Thr Pro Gly
            100                 105                 110

Asn Thr Gly Pro Tyr Gly Leu Val Gly Val Pro Gly Cys Ser Gly Ser
        115                 120                 125

Lys Gly Glu Gln Gly Phe Pro Gly Leu Pro Gly Thr Leu Gly Tyr Pro
    130                 135                 140

Gly Ile Pro Gly Ala Ala Gly Leu Lys Gly Gln Lys Gly Ala Pro Ala
145                 150                 155                 160

Lys Glu Glu Asp Ile Glu Leu Asp Ala Lys Gly Asp Pro Gly Leu Pro
                165                 170                 175

Gly Ala Pro Gly Pro Gln Gly Leu Pro Gly Pro Pro Gly Phe Pro Gly
            180                 185                 190

Pro Val Gly Pro Pro Gly Pro Pro Gly Phe Phe Gly Phe Pro Gly Ala
        195                 200                 205

Met Gly Pro Arg Gly Pro Lys Gly His Met Gly Glu Arg Val Ile Gly
    210                 215                 220

His Lys Gly Glu Arg Gly Val Lys Gly Leu Thr Gly Pro Pro Gly Pro
225                 230                 235                 240

Pro Gly Thr Val Ile Val Thr Leu Thr Gly Pro Asp Asn Arg Thr Asp
                245                 250                 255

Leu Lys Gly Glu Lys Gly Asp Lys Gly Ala Met Gly Glu Pro Gly Pro
            260                 265                 270

Pro Gly Pro Ser Gly Leu Pro Gly Glu Ser Tyr Gly Ser Glu Lys Gly
        275                 280                 285

Ala Pro Gly Asp Pro Gly Leu Gln Gly Lys Pro Gly Lys Asp Gly Val
    290                 295                 300

Pro Gly Phe Pro Gly Ser Glu Gly Val Lys Gly Asn Arg Gly Phe Pro

305 310 315 320

Gly Leu Met Gly Glu Asp Gly Ile Lys Gly Gln Lys Gly Asp Ile Gly
325 330 335

Pro Pro Gly Phe Arg Gly Pro Thr Glu Tyr Tyr Asp Thr Tyr Gln Glu
340 345 350

Lys Gly Asp Glu Gly Thr Pro Gly Pro Pro Gly Pro Arg Gly Ala Arg
355 360 365

Gly Pro Gln Gly Pro Ser Gly Pro Pro Gly Val Pro Gly Ser Pro Gly
370 375 380

Ser Ser Arg Pro Gly Leu Arg Gly Ala Pro Gly Trp Pro Gly Leu Lys
385 390 395 400

Gly Ser Lys Gly Glu Arg Gly Arg Pro Gly Lys Asp Ala Met Gly Thr
405 410 415

Pro Gly Ser Pro Gly Cys Ala Gly Ser Pro Gly Leu Pro Gly Ser Pro
420 425 430

Gly Pro Pro Gly Pro Pro Gly Asp Ile Val Phe Arg Lys Gly Pro Pro
435 440 445

Gly Asp His Gly Leu Pro Gly Tyr Leu Gly Ser Pro Gly Ile Pro Gly
450 455 460

Val Asp Gly Pro Lys Gly Glu Pro Gly Leu Leu Cys Thr Gln Cys Pro
465 470 475 480

Tyr Ile Pro Gly Pro Pro Gly Leu Pro Gly Leu Pro Gly Leu His Gly
485 490 495

Val Lys Gly Ile Pro Gly Arg Gln Gly Ala Ala Gly Leu Lys Gly Ser
500 505 510

Pro Gly Ser Pro Gly Asn Thr Gly Leu Pro Gly Phe Pro Gly Phe Pro
515 520 525

Gly Ala Gln Gly Asp Pro Gly Leu Lys Gly Glu Lys Gly Glu Thr Leu
530 535 540

Gln Pro Glu Gly Gln Val Gly Val Pro Gly Asp Pro Gly Leu Arg Gly
545 550 555 560

```
Gln Pro Gly Arg Lys Gly Leu Asp Gly Ile Pro Gly Thr Pro Gly Val
                565                 570                 575

Lys Gly Leu Pro Gly Pro Lys Gly Glu Leu Ala Leu Ser Gly Glu Lys
            580                 585                 590

Gly Asp Gln Gly Pro Pro Gly Asp Pro Gly Ser Pro Gly Ser Pro Gly
        595                 600                 605

Pro Ala Gly Pro Ala Gly Pro Pro Gly Tyr Gly Pro Gln Gly Glu Pro
    610                 615                 620

Gly Leu Gln Gly Thr Gln Gly Val Pro Gly Ala Pro Gly Pro Pro Gly
625                 630                 635                 640

Glu Ala Gly Pro Arg Gly Glu Leu Ser Val Ser Thr Pro Val Pro Gly
                645                 650                 655

Pro Pro Gly Pro Pro Gly Pro Pro Gly His Pro Gly Pro Gln Gly Pro
            660                 665                 670

Pro Gly Ile Pro Gly Ser Leu Gly Lys Cys Gly Asp Pro Gly Leu Pro
        675                 680                 685

Gly Pro Asp Gly Glu Pro Gly Ile Pro Gly Ile Gly Phe Pro Gly Pro
    690                 695                 700

Pro Gly Pro Lys Gly Asp Gln Gly Phe Pro Gly Thr Lys Gly Ser Leu
705                 710                 715                 720

Gly Cys Pro Gly Lys Met Gly Glu Pro Gly Leu Pro Gly Lys Pro Gly
                725                 730                 735

Leu Pro Gly Ala Lys Gly Glu Pro Ala Val Ala Met Pro Gly Gly Pro
            740                 745                 750

Gly Thr Pro Gly Phe Pro Gly Glu Arg Gly Asn Ser Gly Glu His Gly
        755                 760                 765

Glu Ile Gly Leu Pro Gly Leu Pro Gly Leu Pro Gly Thr Pro Gly Asn
    770                 775                 780

Glu Gly Leu Asp Gly Pro Arg Gly Asp Pro Gly Gln Pro Gly Pro Pro
785                 790                 795                 800

Gly Glu Gln Gly Pro Pro Gly Arg Cys Ile Glu Gly Pro Arg Gly Ala
                805                 810                 815
```

```
Gln Gly Leu Pro Gly Leu Asn Gly Leu Lys Gly Gln Gln Gly Arg Arg
            820                 825                 830

Gly Lys Thr Gly Pro Lys Gly Asp Pro Gly Ile Pro Gly Leu Asp Arg
        835                 840                 845

Ser Gly Phe Pro Gly Glu Thr Gly Ser Pro Gly Ile Pro Gly His Gln
    850                 855                 860

Gly Glu Met Gly Pro Leu Gly Gln Arg Gly Tyr Pro Gly Asn Pro Gly
865                 870                 875                 880

Ile Leu Gly Pro Pro Gly Glu Asp Gly Val Ile Gly Met Met Gly Phe
                885                 890                 895

Pro Gly Ala Ile Gly Pro Pro Gly Pro Pro Gly Asn Pro Gly Thr Pro
            900                 905                 910

Gly Gln Arg Gly Ser Pro Gly Ile Pro Gly Val Lys Gly Gln Arg Gly
        915                 920                 925

Thr Pro Gly Ala Lys Gly Glu Gln Gly Asp Lys Gly Asn Pro Gly Pro
    930                 935                 940

Ser Glu Ile Ser His Val Ile Gly Asp Lys Gly Glu Pro Gly Leu Lys
945                 950                 955                 960

Gly Phe Ala Gly Asn Pro Gly Glu Lys Gly Asn Arg Gly Val Pro Gly
                965                 970                 975

Met Pro Gly Leu Lys Gly Leu Lys Gly Leu Pro Gly Pro Ala Gly Pro
            980                 985                 990

Pro Gly Pro Arg Gly Asp Leu Gly  Ser Thr Gly Asn Pro  Gly Glu Pro
        995                  1000                1005

Gly Leu  Arg Gly Ile Pro Gly  Ser Met Gly Asn Met  Gly Met Pro
    1010                 1015                1020

Gly Ser  Lys Gly Lys Arg Gly  Thr Leu Gly Phe Pro  Gly Arg Ala
    1025                 1030                1035

Gly Arg  Pro Gly Leu Pro Gly  Ile His Gly Leu Gln  Gly Asp Lys
    1040                 1045                1050

Gly Glu  Pro Gly Tyr Ser Glu  Gly Thr Arg Pro Gly  Pro Pro Gly
    1055                 1060                1065
```

```
Pro Thr Gly Asp Pro Gly Leu Pro Gly Asp Met Gly Lys Lys Gly
    1070                1075                1080

Glu Met Gly Gln Pro Gly Pro Pro Gly His Leu Gly Pro Ala Gly
    1085                1090                1095

Pro Glu Gly Ala Pro Gly Ser Pro Gly Ser Pro Gly Leu Pro Gly
    1100                1105                1110

Lys Pro Gly Pro His Gly Asp Leu Gly Phe Lys Gly Ile Lys Gly
    1115                1120                1125

Leu Leu Gly Pro Pro Gly Ile Arg Gly Pro Pro Gly Leu Pro Gly
    1130                1135                1140

Phe Pro Gly Ser Pro Gly Pro Met Gly Ile Arg Gly Asp Gln Gly
    1145                1150                1155

Arg Asp Gly Ile Pro Gly Pro Ala Gly Glu Lys Gly Glu Thr Gly
    1160                1165                1170

Leu Leu Arg Ala Pro Pro Gly Pro Arg Gly Asn Pro Gly Ala Gln
    1175                1180                1185

Gly Ala Lys Gly Asp Arg Gly Ala Pro Gly Phe Pro Gly Leu Pro
    1190                1195                1200

Gly Arg Lys Gly Ala Met Gly Asp Ala Gly Pro Arg Gly Pro Thr
    1205                1210                1215

Gly Ile Glu Gly Phe Pro Gly Pro Pro Gly Leu Pro Gly Ala Ile
    1220                1225                1230

Ile Pro Gly Gln Thr Gly Asn Arg Gly Pro Pro Gly Ser Arg Gly
    1235                1240                1245

Ser Pro Gly Ala Pro Gly Pro Pro Gly Pro Pro Gly Ser His Val
    1250                1255                1260

Ile Gly Ile Lys Gly Asp Lys Gly Ser Met Gly His Pro Gly Pro
    1265                1270                1275

Lys Gly Pro Pro Gly Thr Ala Gly Asp Met Gly Pro Pro Gly Arg
    1280                1285                1290

Leu Gly Ala Pro Gly Thr Pro Gly Leu Pro Gly Pro Arg Gly Asp
```

1295 1300 1305

Pro Gly Phe Gln Gly Phe Pro Gly Val Lys Gly Glu Lys Gly Asn
1310 1315 1320

Pro Gly Phe Leu Gly Ser Ile Gly Pro Pro Gly Pro Ile Gly Pro
1325 1330 1335

Lys Gly Pro Pro Gly Val Arg Gly Asp Pro Gly Thr Leu Lys Ile
1340 1345 1350

Ile Ser Leu Pro Gly Ser Pro Gly Pro Pro Gly Thr Pro Gly Glu
1355 1360 1365

Pro Gly Met Gln Gly Glu Pro Gly Pro Pro Gly Pro Pro Gly Asn
1370 1375 1380

Leu Gly Pro Cys Gly Pro Arg Gly Lys Pro Gly Lys Asp Gly Lys
1385 1390 1395

Pro Gly Thr Pro Gly Pro Ala Gly Glu Lys Gly Asn Lys Gly Ser
1400 1405 1410

Lys Gly Glu Pro Gly Pro Ala Gly Ser Asp Gly Leu Pro Gly Leu
1415 1420 1425

Lys Gly Lys Arg Gly Asp Ser Gly Ser Pro Ala Thr Trp Thr Thr
1430 1435 1440

Arg Gly Phe Val Phe Thr Arg His Ser Gln Thr Thr Ala Ile Pro
1445 1450 1455

Ser Cys Pro Glu Gly Thr Val Pro Leu Tyr Ser Gly Phe Ser Phe
1460 1465 1470

Leu Phe Val Gln Gly Asn Gln Arg Ala His Gly Gln Asp Leu Gly
1475 1480 1485

Thr Leu Gly Ser Cys Leu Gln Arg Phe Thr Thr Met Pro Phe Leu
1490 1495 1500

Phe Cys Asn Val Asn Asp Val Cys Asn Phe Ala Ser Arg Asn Asp
1505 1510 1515

Tyr Ser Tyr Trp Leu Ser Thr Pro Ala Leu Met Pro Met Asn Met
1520 1525 1530

```
Ala Pro Ile Thr Gly Arg Ala Leu Glu Pro Tyr Ile Ser Arg Cys
    1535                1540                1545

Thr Val Cys Glu Gly Pro Ala Ile Ala Ile Ala Val His Ser Gln
    1550                1555                1560

Thr Thr Asp Ile Pro Pro Cys Pro His Gly Trp Ile Ser Leu Trp
    1565                1570                1575

Lys Gly Phe Ser Phe Ile Met Phe Thr Ser Ala Gly Ser Glu Gly
    1580                1585                1590

Thr Gly Gln Ala Leu Ala Ser Pro Gly Ser Cys Leu Glu Glu Phe
    1595                1600                1605

Arg Ala Ser Pro Phe Leu Glu Cys His Gly Arg Gly Thr Cys Asn
    1610                1615                1620

Tyr Tyr Ser Asn Ser Tyr Ser Phe Trp Leu Ala Ser Leu Asn Pro
    1625                1630                1635

Glu Arg Met Phe Arg Lys Pro Ile Pro Ser Thr Val Lys Ala Gly
    1640                1645                1650

Glu Leu Glu Lys Ile Ile Ser Arg Cys Gln Val Cys Met Lys Lys
    1655                1660                1665

Arg His
    1670
```

<210> 13
<211> 2781
<212> DNA
<213> Homo sapiens

<400> 13

```
atggatatgt ttcctctcac ctgggttttc ttagccctct acttttcaag acaccaagtg      60
agaggccaac cagacccacc gtgcggaggt cgtttgaatt ccaaagatgc tggctatatc     120
acctctcccg gttacccccca ggactacccc tcccaccaga actgcgagtg gattgtttac    180
gccccccgaac ccaaccagaa gattgtcctc aacttcaacc ctcactttga aatcgagaag    240
cacgactgca agtatgactt tatcgagatt cgggatgggg acagtgaatc cgcagacctc     300
ctgggcaaac actgtgggaa catcgccccg cccaccatca tctcctcggg ctccatgctc     360
tacatcaagt tcacctccga ctacgcccgg caggggggcag gcttctctct gcgctacgag    420
atcttcaaga caggctctga agattgctca aaaaacttca caagccccaa cgggaccatc     480
gaatctcctg ggtttcctga gaagtatcca cacaacttgg actgcacctt taccatcctg     540
```

gccaaaccca agatggagat catcctgcag ttcctgatct ttgacctgga gcatgaccct 600
ttgcaggtgg gagaggggga ctgcaagtac gattggctgg acatctggga tggcattcca 660
catgttggcc ccctgattgg caagtactgt gggaccaaaa caccctctga acttcgttca 720
tcgacgggga tcctctccct gacctttcac acggacatgg cggtggccaa ggatggcttc 780
tctgcgcgtt actacctggt ccaccaagag ccactagaga actttcagtg caatgttcct 840
ctgggcatgg agtctggccg gattgctaat gaacagatca gtgcctcatc tacctactct 900
gatgggaggt ggacccctca acaaagccgg ctccatggtg atgacaatgg ctggaccccc 960
aacttggatt ccaacaagga gtatctccag gtggacctgc gctttttaac catgctcacg 1020
gccatcgcaa cacagggagc gatttccagg gaaacacaga atggctacta tgtcaaatcc 1080
tacaagctgg aagtcagcac taatggagag gactggatgg tgtaccggca tggcaaaaac 1140
cacaaggtat ttcaagccaa caacgatgca actgaggtgg ttctgaacaa gctccacgct 1200
ccactgctga caaggtttgt tagaatccgc cctcagacct ggcactcagg tatcgccctc 1260
cggctggagc tcttcggctg ccgggtcaca gatgctccct gctccaacat gctggggatg 1320
ctctcaggcc tcattgcaga ctcccagatc tccgcctctt ccacccagga atacctctgg 1380
agccccagtg cagcccgcct ggtcagcagc cgctcgggct ggttccctcg aatccctcag 1440
gcccagcccg gtgaggagtg gcttcaggta gatctgggaa cacccaagac agtgaaaggt 1500
gtcatcatcc agggagcccg cggaggagac agtatcactg ctgtggaagc cagagcattt 1560
gtgcgcaagt tcaaagtctc ctacagccta aacggcaagg actgggaata cattcaggac 1620
cccaggaccc agcagccaaa gctgttcgaa gggaacatgc actatgacac ccctgacatc 1680
cgaaggtttg accccattcc ggcacagtat gtgcgggtat acccggagag gtggtcgccg 1740
gcggggattg ggatgcggct ggaggtgctg ggctgtgact ggacagactc caagcccacg 1800
gtagagacgc tgggacccac tgtgaagagc gaagagacaa ccacccccta ccccaccgaa 1860
gaggaggcca cagagtgtgg ggagaactgc agctttgagg atgacaaaga tttgcagctc 1920
ccttcgggat tcaattgcaa cttcgatttc ctcgaggagc cctgtggttg gatgtatgac 1980
catgccaagt ggctccggac cacctgggcc agcagctcca gcccaaacga ccggacgttt 2040
ccagatgaca ggaatttctt gcggctgcag agtgacagcc agagagaggg ccagtatgcc 2100
cggctcatca gcccccctgt ccacctgccc cgaagcccgg tgtgcatgga gttccagtac 2160
caggccacgg gcggccgcgg ggtggcgctg caggtggtgc gggaagccag ccaggagagc 2220
aagttgctgt gggtcatccg tgaggaccag ggcggcgagt ggaagcacgg gcggatcatc 2280
ctgcccagct acgacatgga gtaccagatt gtgttcgagg gagtgatagg gaaaggacgt 2340
tccggagaga ttgccattga tgacattcgg ataagcactg atgtcccact ggagaactgc 2400

atggaaccca tctcggcttt tgcagtggac atcccagaaa tacatgagag agaaggatat 2460

gaagatgaaa ttgatgatga atacgaggtg gactggagca attcttcttc tgcaacctca 2520

gggtctggcg ccccctcgac cgacaaagaa aagagctggc tgtacaccct ggatcccatc 2580

ctcatcacca tcatcgccat gagctcactg ggcgtcctcc tgggggccac ctgtgcaggc 2640

ctcctgctct actgcacctg ttcctactcg ggcctgagct cccgaagctg caccacactg 2700

gagaactaca acttcgagct ctacgatggc cttaagcaca aggtcaagat gaaccaccaa 2760

aagtgctgct ccgaggcatg a 2781

<210> 14
<211> 926
<212> PRT
<213> Homo sapiens

<400> 14

```
Met Asp Met Phe Pro Leu Thr Trp Val Phe Leu Ala Leu Tyr Phe Ser
1               5                   10                  15

Arg His Gln Val Arg Gly Gln Pro Asp Pro Pro Cys Gly Gly Arg Leu
            20                  25                  30

Asn Ser Lys Asp Ala Gly Tyr Ile Thr Ser Pro Gly Tyr Pro Gln Asp
        35                  40                  45

Tyr Pro Ser His Gln Asn Cys Glu Trp Ile Val Tyr Ala Pro Glu Pro
    50                  55                  60

Asn Gln Lys Ile Val Leu Asn Phe Asn Pro His Phe Glu Ile Glu Lys
65                  70                  75                  80

His Asp Cys Lys Tyr Asp Phe Ile Glu Ile Arg Asp Gly Asp Ser Glu
                85                  90                  95

Ser Ala Asp Leu Leu Gly Lys His Cys Gly Asn Ile Ala Pro Pro Thr
            100                 105                 110

Ile Ile Ser Ser Gly Ser Met Leu Tyr Ile Lys Phe Thr Ser Asp Tyr
        115                 120                 125

Ala Arg Gln Gly Ala Gly Phe Ser Leu Arg Tyr Glu Ile Phe Lys Thr
    130                 135                 140

Gly Ser Glu Asp Cys Ser Lys Asn Phe Thr Ser Pro Asn Gly Thr Ile
145                 150                 155                 160

Glu Ser Pro Gly Phe Pro Glu Lys Tyr Pro His Asn Leu Asp Cys Thr
```

165 170 175

Phe Thr Ile Leu Ala Lys Pro Lys Met Glu Ile Ile Leu Gln Phe Leu
180 185 190

Ile Phe Asp Leu Glu His Asp Pro Leu Gln Val Gly Glu Gly Asp Cys
195 200 205

Lys Tyr Asp Trp Leu Asp Ile Trp Asp Gly Ile Pro His Val Gly Pro
210 215 220

Leu Ile Gly Lys Tyr Cys Gly Thr Lys Thr Pro Ser Glu Leu Arg Ser
225 230 235 240

Ser Thr Gly Ile Leu Ser Leu Thr Phe His Thr Asp Met Ala Val Ala
245 250 255

Lys Asp Gly Phe Ser Ala Arg Tyr Tyr Leu Val His Gln Glu Pro Leu
260 265 270

Glu Asn Phe Gln Cys Asn Val Pro Leu Gly Met Glu Ser Gly Arg Ile
275 280 285

Ala Asn Glu Gln Ile Ser Ala Ser Ser Thr Tyr Ser Asp Gly Arg Trp
290 295 300

Thr Pro Gln Gln Ser Arg Leu His Gly Asp Asp Asn Gly Trp Thr Pro
305 310 315 320

Asn Leu Asp Ser Asn Lys Glu Tyr Leu Gln Val Asp Leu Arg Phe Leu
325 330 335

Thr Met Leu Thr Ala Ile Ala Thr Gln Gly Ala Ile Ser Arg Glu Thr
340 345 350

Gln Asn Gly Tyr Tyr Val Lys Ser Tyr Lys Leu Glu Val Ser Thr Asn
355 360 365

Gly Glu Asp Trp Met Val Tyr Arg His Gly Lys Asn His Lys Val Phe
370 375 380

Gln Ala Asn Asn Asp Ala Thr Glu Val Val Leu Asn Lys Leu His Ala
385 390 395 400

Pro Leu Leu Thr Arg Phe Val Arg Ile Arg Pro Gln Thr Trp His Ser
405 410 415

```
Gly Ile Ala Leu Arg Leu Glu Leu Phe Gly Cys Arg Val Thr Asp Ala
            420                 425                 430

Pro Cys Ser Asn Met Leu Gly Met Leu Ser Gly Leu Ile Ala Asp Ser
        435                 440                 445

Gln Ile Ser Ala Ser Ser Thr Gln Glu Tyr Leu Trp Ser Pro Ser Ala
    450                 455                 460

Ala Arg Leu Val Ser Ser Arg Ser Gly Trp Phe Pro Arg Ile Pro Gln
465                 470                 475                 480

Ala Gln Pro Gly Glu Glu Trp Leu Gln Val Asp Leu Gly Thr Pro Lys
                485                 490                 495

Thr Val Lys Gly Val Ile Ile Gln Gly Ala Arg Gly Gly Asp Ser Ile
            500                 505                 510

Thr Ala Val Glu Ala Arg Ala Phe Val Arg Lys Phe Lys Val Ser Tyr
        515                 520                 525

Ser Leu Asn Gly Lys Asp Trp Glu Tyr Ile Gln Asp Pro Arg Thr Gln
    530                 535                 540

Gln Pro Lys Leu Phe Glu Gly Asn Met His Tyr Asp Thr Pro Asp Ile
545                 550                 555                 560

Arg Arg Phe Asp Pro Ile Pro Ala Gln Tyr Val Arg Val Tyr Pro Glu
                565                 570                 575

Arg Trp Ser Pro Ala Gly Ile Gly Met Arg Leu Glu Val Leu Gly Cys
            580                 585                 590

Asp Trp Thr Asp Ser Lys Pro Thr Val Glu Thr Leu Gly Pro Thr Val
        595                 600                 605

Lys Ser Glu Glu Thr Thr Thr Pro Tyr Pro Thr Glu Glu Glu Ala Thr
    610                 615                 620

Glu Cys Gly Glu Asn Cys Ser Phe Glu Asp Asp Lys Asp Leu Gln Leu
625                 630                 635                 640

Pro Ser Gly Phe Asn Cys Asn Phe Asp Phe Leu Glu Glu Pro Cys Gly
                645                 650                 655

Trp Met Tyr Asp His Ala Lys Trp Leu Arg Thr Thr Trp Ala Ser Ser
            660                 665                 670
```

```
Ser Ser Pro Asn Asp Arg Thr Phe Pro Asp Asp Arg Asn Phe Leu Arg
        675                 680                 685

Leu Gln Ser Asp Ser Gln Arg Glu Gly Gln Tyr Ala Arg Leu Ile Ser
    690                 695                 700

Pro Pro Val His Leu Pro Arg Ser Pro Val Cys Met Glu Phe Gln Tyr
705                 710                 715                 720

Gln Ala Thr Gly Gly Arg Gly Val Ala Leu Gln Val Val Arg Glu Ala
                725                 730                 735

Ser Gln Glu Ser Lys Leu Leu Trp Val Ile Arg Glu Asp Gln Gly Gly
            740                 745                 750

Glu Trp Lys His Gly Arg Ile Ile Leu Pro Ser Tyr Asp Met Glu Tyr
        755                 760                 765

Gln Ile Val Phe Glu Gly Val Ile Gly Lys Gly Arg Ser Gly Glu Ile
    770                 775                 780

Ala Ile Asp Asp Ile Arg Ile Ser Thr Asp Val Pro Leu Glu Asn Cys
785                 790                 795                 800

Met Glu Pro Ile Ser Ala Phe Ala Val Asp Ile Pro Glu Ile His Glu
                805                 810                 815

Arg Glu Gly Tyr Glu Asp Glu Ile Asp Asp Glu Tyr Glu Val Asp Trp
            820                 825                 830

Ser Asn Ser Ser Ser Ala Thr Ser Gly Ser Gly Ala Pro Ser Thr Asp
        835                 840                 845

Lys Glu Lys Ser Trp Leu Tyr Thr Leu Asp Pro Ile Leu Ile Thr Ile
    850                 855                 860

Ile Ala Met Ser Ser Leu Gly Val Leu Leu Gly Ala Thr Cys Ala Gly
865                 870                 875                 880

Leu Leu Leu Tyr Cys Thr Cys Ser Tyr Ser Gly Leu Ser Ser Arg Ser
                885                 890                 895

Cys Thr Thr Leu Glu Asn Tyr Asn Phe Glu Leu Tyr Asp Gly Leu Lys
            900                 905                 910

His Lys Val Lys Met Asn His Gln Lys Cys Cys Ser Glu Ala
        915                 920                 925
```

<210> 15
<211> 1173
<212> DNA
<213> Homo sapiens

<400> 15

```
atggcgcaaa ggtacgacga tctaccccat tacgggggca tggatggagt aggcatcccc      60
tccacgatgt atggggaccc gcatgcagcc aggtccatgc agccggtcca ccacctgaac     120
cacgggcctc ctctgcactc gcatcagtac ccgcacacag ctcataccaa cgccatggcc     180
cccagcatgg gctcctctgt caatgacgct ttaaagagag ataaagatgc catttatgga     240
cacccccctct tccctctctt agcactgatt tttgagaaat gtgaattagc tacttgtacc    300
ccccgcgagc cgggggtggc gggcggggac gtctgctcgt cagagtcatt caatgaagat     360
atagccgtgt tcgccaaaca gattcgcgca gaaaaacctc tattttcttc taatccagaa     420
ctggataact tgatgattca agccatacaa gtattaaggt ttcatctatt ggaattagag     480
aaggtacacg aattatgtga caatttctgc caccggtata ttagctgttt gaaagggaaa     540
atgcctatcg atttggtgat agacgataga gaaggaggat caaaatcaga cagtgaagat     600
ataacaagat cagcaaatct aactgaccag ccctcttgga acagagatca tgatgacacg     660
gcatctactc gttcaggagg aaccccaggc ccttccagcg gtggccacac gtcacacagt     720
ggggacaaca gcagtgagca aggtgatggc ttggacaaca gtgtagcttc ccccagcaca     780
ggtgacgatg atgaccctga taaggacaaa aagcgtcaca aaaagcgtgg catctttccc     840
aaagtagcca caaatatcat gagggcgtgg ctgttccagc atctaacaca cccttaccct     900
tctgaagaac agaaaaagca gttggcacaa gacacgggac tcaccatcct tcaagtgaac     960
aattggttta ttaatgcccg gagaagaata gtgcagccca tgatagacca gtccaaccga    1020
gcagtaagtc aaggaacacc ttataatcct gatggacagc ccatgggagg tttcgtaatg    1080
gacggtcagc aacatatggg aattagagca ccaggaccta tgagtggaat gggcatgaat    1140
atgggcatgg aggggcagtg gcactacatg taa                                 1173
```

<210> 16
<211> 390
<212> PRT
<213> Homo sapiens

<400> 16

```
Met Ala Gln Arg Tyr Asp Asp Leu Pro His Tyr Gly Gly Met Asp Gly
1               5                   10                  15

Val Gly Ile Pro Ser Thr Met Tyr Gly Asp Pro His Ala Ala Arg Ser
            20                  25                  30
```

```
Met Gln Pro Val His His Leu Asn His Gly Pro Pro Leu His Ser His
        35                  40                  45

Gln Tyr Pro His Thr Ala His Thr Asn Ala Met Ala Pro Ser Met Gly
    50                  55                  60

Ser Ser Val Asn Asp Ala Leu Lys Arg Asp Lys Asp Ala Ile Tyr Gly
65                  70                  75                  80

His Pro Leu Phe Pro Leu Leu Ala Leu Ile Phe Glu Lys Cys Glu Leu
                85                  90                  95

Ala Thr Cys Thr Pro Arg Glu Pro Gly Val Ala Gly Gly Asp Val Cys
            100                 105                 110

Ser Ser Glu Ser Phe Asn Glu Asp Ile Ala Val Phe Ala Lys Gln Ile
        115                 120                 125

Arg Ala Glu Lys Pro Leu Phe Ser Ser Asn Pro Glu Leu Asp Asn Leu
    130                 135                 140

Met Ile Gln Ala Ile Gln Val Leu Arg Phe His Leu Leu Glu Leu Glu
145                 150                 155                 160

Lys Val His Glu Leu Cys Asp Asn Phe Cys His Arg Tyr Ile Ser Cys
                165                 170                 175

Leu Lys Gly Lys Met Pro Ile Asp Leu Val Ile Asp Asp Arg Glu Gly
            180                 185                 190

Gly Ser Lys Ser Asp Ser Glu Asp Ile Thr Arg Ser Ala Asn Leu Thr
        195                 200                 205

Asp Gln Pro Ser Trp Asn Arg Asp His Asp Asp Thr Ala Ser Thr Arg
    210                 215                 220

Ser Gly Gly Thr Pro Gly Pro Ser Ser Gly Gly His Thr Ser His Ser
225                 230                 235                 240

Gly Asp Asn Ser Ser Glu Gln Gly Asp Gly Leu Asp Asn Ser Val Ala
                245                 250                 255

Ser Pro Ser Thr Gly Asp Asp Asp Asp Pro Asp Lys Asp Lys Lys Arg
            260                 265                 270

His Lys Lys Arg Gly Ile Phe Pro Lys Val Ala Thr Asn Ile Met Arg
        275                 280                 285
```

```
Ala Trp Leu Phe Gln His Leu Thr His Pro Tyr Pro Ser Glu Glu Gln
    290                 295                 300

Lys Lys Gln Leu Ala Gln Asp Thr Gly Leu Thr Ile Leu Gln Val Asn
305                 310                 315                 320

Asn Trp Phe Ile Asn Ala Arg Arg Arg Ile Val Gln Pro Met Ile Asp
                325                 330                 335

Gln Ser Asn Arg Ala Val Ser Gln Gly Thr Pro Tyr Asn Pro Asp Gly
            340                 345                 350

Gln Pro Met Gly Gly Phe Val Met Asp Gly Gln Gln His Met Gly Ile
        355                 360                 365

Arg Ala Pro Gly Pro Met Ser Gly Met Gly Met Asn Met Gly Met Glu
    370                 375                 380

Gly Gln Trp His Tyr Met
385                 390
```

<210> 17
<211> 2097
<212> DNA
<213> Homo sapiens

<400> 17

```
atgctgagcc caaagatcag gcaggccagg agggcccgct ccaaaagcct agtgatgggg     60
gagcagagcc ggagccctgg gcggatgccg tgccctcaca ggctgggccc cgtgctgaag    120
gcgggctggc tgaagaagca gaggagcatc atgaagaact ggcagcagcg ctggtttgtg    180
ctgcgtgggg atcagctttt ctactacaag gacaaagatg agatcaagcc ccagggattt    240
atttctctac aagggacaca ggtgactgaa cttcctcctg gccccgagga cccagggaag    300
cacctctttg agatcagccc aggtggtgcc ggggagcggg agaaggtgcc ggccaacccc    360
gaggcgctcc tgctcatggc cagctcccag cgtgacatgg aggactgggt gcaggccatc    420
cgccgagtca tctgggcccc gctgggcgga gggatctttg ggcagcgcct agaggaaaca    480
gtccaccacg agcggaagta tggcccccgc ctggcgcccc tgctggtgga gcagtgtgtg    540
gacttcatcc gggagcgcgg gctcactgag gaggggctgt tccgcatgcc aggccaggcc    600
aacctggtga gggacctgca ggattccttc gactgtgggg agaagccact gtttgacagc    660
acaacagacg tgcacacggt ggcctccctg ctgaagctgt acctgcggga gctccccgag    720
cccgtggtcc ccttcgccag gtacgaggac ttcctcagct gcgcccagct gctcaccaag    780
gacgaggggg agggcactct ggagttggct aaacaagtga gcaaccttcc tcaggcaaat    840
```

```
tacaacctgc tcagatacat ctgcaagttt ctggatgaag ttcaggcata ctcaaatgtc    900
aacaagatga gtgtccagaa tctggcaacc gtttttggac ctaacattct gcggccacag    960
gtagaggacc cagtaaccat catggaaggc acttccctcg tccagcacct gatgaccgtc   1020
ctcatccgca aacacagcca gctcttcacg gcaccggtcc cggaagggcc cacctccccg   1080
cgcgggggcc tgcaatgcgc agtggggtgg ggctccgagg aggtcaccag ggacagccaa   1140
ggagagcccg gcggccccgg cctgcccgcg cacaggacct cttccctgga cggggcggcc   1200
gtggcggtgc tctccagaac agcccccacg ggccggggga gccggtgcag ccctgggaag   1260
aaggtgcaga ccctgcccag ttggaagtcc tccttccggc agccgaggtc cctatcggga   1320
agcccgaagg ggggcggctc atccctggag gtgcccatca tctcctccgg cgggaactgg   1380
cttatgaacg ggctgtcctc cctgcgcgga caccgccggg cctcgtcggg agaccggctc   1440
aaggactcgg gctccgtgca gagactctcc acctacgaca atgtgcccgc gccgggcctg   1500
gtccccggca tacccagcgt ggccagtatg gcgtggtccg ggcctcgtc cagcgagtcg   1560
tcggtggggg gctcactcag cagctgcacg gcctgccgcg ccagcgactc gtctgcccgc   1620
agttccctgc acaccgactg ggccctggag ccctccccgc tccccagcag cagcgaggac   1680
cccaagtccc tggacctgga ccacagcatg gacgaggcgg gcgcgggtgc cagcaacagc   1740
gagcccagcg agccggacag ccccacccgg gaacacgcgc gccgctccga ggccttacag   1800
gggctggtca ctgagctcag ggccgagctg tgccgccagc ggactgagta cgagaggagt   1860
gtgaaaagaa tcgaagaagg gagtgctgac ctgagaaaac gaatgtcccg gttagaagaa   1920
gaactggacc aggaaaagaa aaaatacatc atgctggaaa taaagctgcg gaactctgaa   1980
cgggcgcggg aggatgcgga gaggaggaac cagctgttgc agagggaaat ggaggagttt   2040
ttttcgaccc taggaagctt gactgttggg gcaaaaggtg ccagggcccc aaagtaa      2097
```

<210> 18
<211> 698
<212> PRT
<213> Homo sapiens

<400> 18

```
Met Leu Ser Pro Lys Ile Arg Gln Ala Arg Arg Ala Arg Ser Lys Ser
1               5                   10                  15

Leu Val Met Gly Glu Gln Ser Arg Ser Pro Gly Arg Met Pro Cys Pro
            20                  25                  30

His Arg Leu Gly Pro Val Leu Lys Ala Gly Trp Leu Lys Lys Gln Arg
        35                  40                  45
```

```
Ser Ile Met Lys Asn Trp Gln Gln Arg Trp Phe Val Leu Arg Gly Asp
    50                  55                  60

Gln Leu Phe Tyr Tyr Lys Asp Lys Asp Glu Ile Lys Pro Gln Gly Phe
65                  70                  75                  80

Ile Ser Leu Gln Gly Thr Gln Val Thr Glu Leu Pro Pro Gly Pro Glu
                85                  90                  95

Asp Pro Gly Lys His Leu Phe Glu Ile Ser Pro Gly Gly Ala Gly Glu
            100                 105                 110

Arg Glu Lys Val Pro Ala Asn Pro Glu Ala Leu Leu Leu Met Ala Ser
        115                 120                 125

Ser Gln Arg Asp Met Glu Asp Trp Val Gln Ala Ile Arg Arg Val Ile
    130                 135                 140

Trp Ala Pro Leu Gly Gly Gly Ile Phe Gly Gln Arg Leu Glu Glu Thr
145                 150                 155                 160

Val His His Glu Arg Lys Tyr Gly Pro Arg Leu Ala Pro Leu Leu Val
                165                 170                 175

Glu Gln Cys Val Asp Phe Ile Arg Glu Arg Gly Leu Thr Glu Glu Gly
            180                 185                 190

Leu Phe Arg Met Pro Gly Gln Ala Asn Leu Val Arg Asp Leu Gln Asp
        195                 200                 205

Ser Phe Asp Cys Gly Glu Lys Pro Leu Phe Asp Ser Thr Thr Asp Val
    210                 215                 220

His Thr Val Ala Ser Leu Leu Lys Leu Tyr Leu Arg Glu Leu Pro Glu
225                 230                 235                 240

Pro Val Val Pro Phe Ala Arg Tyr Glu Asp Phe Leu Ser Cys Ala Gln
                245                 250                 255

Leu Leu Thr Lys Asp Glu Gly Glu Gly Thr Leu Glu Leu Ala Lys Gln
            260                 265                 270

Val Ser Asn Leu Pro Gln Ala Asn Tyr Asn Leu Leu Arg Tyr Ile Cys
        275                 280                 285

Lys Phe Leu Asp Glu Val Gln Ala Tyr Ser Asn Val Asn Lys Met Ser
    290                 295                 300
```

Val Gln Asn Leu Ala Thr Val Phe Gly Pro Asn Ile Leu Arg Pro Gln
305 310 315 320

Val Glu Asp Pro Val Thr Ile Met Glu Gly Thr Ser Leu Val Gln His
325 330 335

Leu Met Thr Val Leu Ile Arg Lys His Ser Gln Leu Phe Thr Ala Pro
340 345 350

Val Pro Glu Gly Pro Thr Ser Pro Arg Gly Gly Leu Gln Cys Ala Val
355 360 365

Gly Trp Gly Ser Glu Glu Val Thr Arg Asp Ser Gln Gly Glu Pro Gly
370 375 380

Gly Pro Gly Leu Pro Ala His Arg Thr Ser Ser Leu Asp Gly Ala Ala
385 390 395 400

Val Ala Val Leu Ser Arg Thr Ala Pro Thr Gly Pro Gly Ser Arg Cys
405 410 415

Ser Pro Gly Lys Lys Val Gln Thr Leu Pro Ser Trp Lys Ser Ser Phe
420 425 430

Arg Gln Pro Arg Ser Leu Ser Gly Ser Pro Lys Gly Gly Gly Ser Ser
435 440 445

Leu Glu Val Pro Ile Ile Ser Ser Gly Gly Asn Trp Leu Met Asn Gly
450 455 460

Leu Ser Ser Leu Arg Gly His Arg Arg Ala Ser Ser Gly Asp Arg Leu
465 470 475 480

Lys Asp Ser Gly Ser Val Gln Arg Leu Ser Thr Tyr Asp Asn Val Pro
485 490 495

Ala Pro Gly Leu Val Pro Gly Ile Pro Ser Val Ala Ser Met Ala Trp
500 505 510

Ser Gly Ala Ser Ser Ser Glu Ser Ser Val Gly Gly Ser Leu Ser Ser
515 520 525

Cys Thr Ala Cys Arg Ala Ser Asp Ser Ser Ala Arg Ser Ser Leu His
530 535 540

Thr Asp Trp Ala Leu Glu Pro Ser Pro Leu Pro Ser Ser Ser Glu Asp
545 550 555 560

Pro Lys Ser Leu Asp Leu Asp His Ser Met Asp Glu Ala Gly Ala Gly
565 570 575

Ala Ser Asn Ser Glu Pro Ser Glu Pro Asp Ser Pro Thr Arg Glu His
580 585 590

Ala Arg Arg Ser Glu Ala Leu Gln Gly Leu Val Thr Glu Leu Arg Ala
595 600 605

Glu Leu Cys Arg Gln Arg Thr Glu Tyr Glu Arg Ser Val Lys Arg Ile
610 615 620

Glu Glu Gly Ser Ala Asp Leu Arg Lys Arg Met Ser Arg Leu Glu Glu
625 630 635 640

Glu Leu Asp Gln Glu Lys Lys Lys Tyr Ile Met Leu Glu Ile Lys Leu
645 650 655

Arg Asn Ser Glu Arg Ala Arg Glu Asp Ala Glu Arg Arg Asn Gln Leu
660 665 670

Leu Gln Arg Glu Met Glu Glu Phe Phe Ser Thr Leu Gly Ser Leu Thr
675 680 685

Val Gly Ala Lys Gly Ala Arg Ala Pro Lys
690 695

<210> 19
<211> 19
<212> DNA
<213> Artificial

<220>
<223> Synthetic DNA

<400> 19
agtcgcctgt caccatttc 19

<210> 20
<211> 20
<212> DNA
<213> Artificial

<220>
<223> Synthetic DNA

<400> 20
actacggaca ttttcgcatc 20

<210> 21
<211> 22
<212> DNA

<213> Artificial

<220>
<223> Synthetic DNA

<400> 21
gtttgattgc tgcatatttc ag 22

<210> 22
<211> 23
<212> DNA
<213> Artificial

<220>
<223> Synthetic DNA

<400> 22
ggcttagaaa tcttttctaa atg 23

<210> 23
<211> 21
<212> DNA
<213> Artificial

<220>
<223> Synthetic DNA

<400> 23
aatgacatga ttactactct a 21

<210> 24
<211> 21
<212> DNA
<213> Artificial

<220>
<223> Synthetic DNA

<400> 24
ttaatcggtt taggaataca a 21

<210> 25
<211> 22
<212> DNA
<213> Artificial

<220>
<223> Synthetic DNA

<400> 25
cattataaag attcaggcaa tg 22

<210> 26
<211> 21
<212> DNA
<213> Artificial

<220>

<223> Synthetic DNA

<400> 26
gacagtaaga tacagtctat c 21

<210> 27
<211> 22
<212> DNA
<213> Artificial

<220>
<223> Synthetic DNA

<400> 27
acctgttaag tttgtatgca ac 22

<210> 28
<211> 20
<212> DNA
<213> Artificial

<220>
<223> Synthetic DNA

<400> 28
tccaggaaga ggaaaggaaa 20

<210> 29
<211> 23
<212> DNA
<213> Artificial

<220>
<223> Synthetic DNA

<400> 29
catagcaatt tagtgaaata act 23

<210> 30
<211> 22
<212> DNA
<213> Artificial

<220>
<223> Synthetic DNA

<400> 30
gatatggtta agaaaactgt tc 22

<210> 31
<211> 21
<212> DNA
<213> Artificial

<220>
<223> Synthetic DNA

<400> 31

tgacagtttg acagttaaag g 21

<210> 32
<211> 21
<212> DNA
<213> Artificial

<220>
<223> Synthetic DNA

<400> 32
ggatatttct cccaatgaaa g 21

<210> 33
<211> 21
<212> DNA
<213> Artificial

<220>
<223> Synthetic DNA

<400> 33
ctcagattgc cttataatag t 21

<210> 34
<211> 21
<212> DNA
<213> Artificial

<220>
<223> Synthetic DNA

<400> 34
tcatgttact gctacgtaaa c 21

<210> 35
<211> 20
<212> DNA
<213> Artificial

<220>
<223> Synthetic DNA

<400> 35
aaggcctctt aaagatcatg 20

<210> 36
<211> 21
<212> DNA
<213> Artificial

<220>
<223> Synthetic DNA

<400> 36
ttttcatggt gttttatccc t 21

<210> 37

<211> 23
<212> DNA
<213> Artificial

<220>
<223> Synthetic DNA

<400> 37
tctgccatct ctctcctcct ttt 23

<210> 38
<211> 24
<212> DNA
<213> Artificial

<220>
<223> Synthetic DNA

<400> 38
tctgacacaa tgtcctattg ccat 24

<210> 39
<211> 24
<212> DNA
<213> Artificial

<220>
<223> Synthetic DNA

<400> 39
gccccggctc tcggttataa gatg 24

<210> 40
<211> 20
<212> DNA
<213> Artificial

<220>
<223> Synthetic DNA

<400> 40
ccgttcccca gagattccaa 20

<210> 41
<211> 20
<212> DNA
<213> Artificial

<220>
<223> Synthetic DNA

<400> 41
tgccattccg gaggaggtgt 20

<210> 42
<211> 20
<212> DNA
<213> Artificial

<220>
<223> Synthetic DNA

<400> 42
gcccatcagg aatctcccaa 20

<210> 43
<211> 21
<212> DNA
<213> Artificial

<220>
<223> Synthetic DNA

<400> 43
atctggatca tccccttccg c 21

<210> 44
<211> 26
<212> DNA
<213> Artificial

<220>
<223> Synthetic DNA

<400> 44
cccggaacag aaagtaaagc ctctag 26

<210> 45
<211> 1497
<212> DNA
<213> Homo sapiens

<400> 45

atgacagttt tcctttcctt tgctttcctc gctgccattc tgactcacat agggtgcagc 60
aatcagcgcc gaagtccaga aaacagtggg agaagatata accggattca acatgggcaa 120
tgtgcctaca ctttcattct tccagaacac gatggcaact gtcgtgagag tacgacagac 180
cagtacaaca caaacgctct gcagagagat gctccacacg tggaaccgga tttctcttcc 240
cagaaacttc aacatctgga acatgtgatg gaaaattata ctcagtggct gcaaaaactt 300
gagaattaca ttgtggaaaa catgaagtcg gagatggccc agatacagca gaatgcagtt 360
cagaaccaca cggctaccat gctggagata ggaaccagcc tcctctctca gactgcagag 420
cagaccagaa agctgacaga tgttgagacc caggtactaa atcaaacttc tcgacttgag 480
atacagctgc tggagaattc attatccacc tacaagctag agaagcaact tcttcaacag 540
acaaatgaaa tcttgaagat ccatgaaaaa aacagtttat tagaacataa aatcttagaa 600
atggaaggaa aacacaagga agagttggac accttaaagg aagagaaaga gaaccttcaa 660

```
ggcttggtta ctcgtcaaac atatataatc caggagctgg aaaagcaatt aaacagagct     720
accaccaaca acagtgtcct tcagaagcag caactggagc tgatggacac agtccacaac     780
cttgtcaatc tttgcactaa agaaggtgtt ttactaaagg gaggaaaaag agaggaagag     840
aaaccattta gagactgtgc agatgtatat caagctggtt ttaataaaag tggaatctac     900
actatttata ttaataatat gccagaaccc aaaaaggtgt tttgcaatat ggatgtcaat     960
gggggaggtt ggactgtaat acaacatcgt gaagatggaa gtctagattt ccaaagaggc    1020
tggaaggaat ataaaatggg ttttggaaat ccctccggtg aatattggct ggggaatgag    1080
tttatttttg ccattaccag tcagaggcag tacatgctaa gaattgagtt aatggactgg    1140
gaagggaacc gagcctattc acagtatgac agattccaca taggaaatga aaagcaaaac    1200
tataggttgt atttaaaagg tcacactggg acagcaggaa aacagagcag cctgatctta    1260
cacggtgctg atttcagcac taaagatgct gataatgaca actgtatgtg caaatgtgcc    1320
ctcatgttaa caggaggatg gtggtttgat gcttgtggcc cctccaatct aaatggaatg    1380
ttctatactg cgggacaaaa ccatggaaaa ctgaatggga taaagtggca ctacttcaaa    1440
gggcccagtt actccttacg ttccacaact atgatgattc gacctttaga tttttga       1497
```

<210> 46
<211> 498
<212> PRT
<213> Homo sapiens

<400> 46

```
Met Thr Val Phe Leu Ser Phe Ala Phe Leu Ala Ala Ile Leu Thr His
1               5                   10                  15

Ile Gly Cys Ser Asn Gln Arg Arg Ser Pro Glu Asn Ser Gly Arg Arg
            20                  25                  30

Tyr Asn Arg Ile Gln His Gly Gln Cys Ala Tyr Thr Phe Ile Leu Pro
        35                  40                  45

Glu His Asp Gly Asn Cys Arg Glu Ser Thr Thr Asp Gln Tyr Asn Thr
    50                  55                  60

Asn Ala Leu Gln Arg Asp Ala Pro His Val Glu Pro Asp Phe Ser Ser
65                  70                  75                  80

Gln Lys Leu Gln His Leu Glu His Val Met Glu Asn Tyr Thr Gln Trp
                85                  90                  95

Leu Gln Lys Leu Glu Asn Tyr Ile Val Glu Asn Met Lys Ser Glu Met
            100                 105                 110
```

Ala Gln Ile Gln Gln Asn Ala Val Gln Asn His Thr Ala Thr Met Leu
115 120 125

Glu Ile Gly Thr Ser Leu Leu Ser Gln Thr Ala Glu Gln Thr Arg Lys
130 135 140

Leu Thr Asp Val Glu Thr Gln Val Leu Asn Gln Thr Ser Arg Leu Glu
145 150 155 160

Ile Gln Leu Leu Glu Asn Ser Leu Ser Thr Tyr Lys Leu Glu Lys Gln
165 170 175

Leu Leu Gln Gln Thr Asn Glu Ile Leu Lys Ile His Glu Lys Asn Ser
180 185 190

Leu Leu Glu His Lys Ile Leu Glu Met Glu Gly Lys His Lys Glu Glu
195 200 205

Leu Asp Thr Leu Lys Glu Glu Lys Glu Asn Leu Gln Gly Leu Val Thr
210 215 220

Arg Gln Thr Tyr Ile Ile Gln Glu Leu Glu Lys Gln Leu Asn Arg Ala
225 230 235 240

Thr Thr Asn Asn Ser Val Leu Gln Lys Gln Gln Leu Glu Leu Met Asp
245 250 255

Thr Val His Asn Leu Val Asn Leu Cys Thr Lys Glu Gly Val Leu Leu
260 265 270

Lys Gly Gly Lys Arg Glu Glu Glu Lys Pro Phe Arg Asp Cys Ala Asp
275 280 285

Val Tyr Gln Ala Gly Phe Asn Lys Ser Gly Ile Tyr Thr Ile Tyr Ile
290 295 300

Asn Asn Met Pro Glu Pro Lys Lys Val Phe Cys Asn Met Asp Val Asn
305 310 315 320

Gly Gly Gly Trp Thr Val Ile Gln His Arg Glu Asp Gly Ser Leu Asp
325 330 335

Phe Gln Arg Gly Trp Lys Glu Tyr Lys Met Gly Phe Gly Asn Pro Ser
340 345 350

Gly Glu Tyr Trp Leu Gly Asn Glu Phe Ile Phe Ala Ile Thr Ser Gln
355 360 365

```
Arg Gln Tyr Met Leu Arg Ile Glu Leu Met Asp Trp Glu Gly Asn Arg
    370                 375                 380

Ala Tyr Ser Gln Tyr Asp Arg Phe His Ile Gly Asn Glu Lys Gln Asn
385                 390                 395                 400

Tyr Arg Leu Tyr Leu Lys Gly His Thr Gly Thr Ala Gly Lys Gln Ser
                405                 410                 415

Ser Leu Ile Leu His Gly Ala Asp Phe Ser Thr Lys Asp Ala Asp Asn
            420                 425                 430

Asp Asn Cys Met Cys Lys Cys Ala Leu Met Leu Thr Gly Gly Trp Trp
        435                 440                 445

Phe Asp Ala Cys Gly Pro Ser Asn Leu Asn Gly Met Phe Tyr Thr Ala
    450                 455                 460

Gly Gln Asn His Gly Lys Leu Asn Gly Ile Lys Trp His Tyr Phe Lys
465                 470                 475                 480

Gly Pro Ser Tyr Ser Leu Arg Ser Thr Thr Met Met Ile Arg Pro Leu
                485                 490                 495

Asp Phe
```

<210> 47
<211> 1335
<212> DNA
<213> Homo sapiens

<400> 47

```
atgtggcaga ttgttttctt tactctgagc tgtgatcttg tcttggccgc agcctataac     60
aactttcgga agagcatgga cagcatagga aagaagcaat atcaggtcca gcatgggtcc    120
tgcagctaca ctttcctcct gccagagatg gacaactgcc gctcttcctc cagcccctac    180
gtgtccaatg ctgtgcagag ggacgcgccg ctcgaatacg atgactcggt gcagaggctg    240
caagtgctgg agaacatcat ggaaaacaac actcagtggc taatgaaggt attaaatcag    300
accacgagac ttgaacttca gctcttggaa cactccctct cgacaaacaa attggaaaaa    360
cagattttgg accagaccag tgaaataaac aaattgcaag ataagaacag tttcctagaa    420
aagaaggtgc tagctatgga agacaagcac atcatccaac tacagtcaat aaaagaagag    480
aaagatcagc tacaggtgtt agtatccaag caaaattcca tcattgaaga actagaaaaa    540
aaaatagtga ctgccacggt gaataattca gttcttcaga agcagcaaca tgatctcatg    600
```

```
gagacagtta ataacttact gactatgatg tccacatcaa actcagctaa ggaccccact     660

gttgctaaag aagaacaaat cagcttcaga gactgtgctg aagtattcaa atcaggacac     720

accacgaatg gcatctacac gttaacattc cctaattcta cagaagagat caaggcctac     780

tgtgacatgg aagctggagg aggcgggtgg acaattattc agcgacgtga ggatggcagc     840

gttgattttc agaggacttg gaaagaatat aaagtgggat ttggtaaccc ttcaggagaa     900

tattggctgg gaaatgagtt tgtttcgcaa ctgactaatc agcaacgcta tgtgcttaaa     960

atacacctta aagactggga agggaatgag gcttactcat tgtatgaaca tttctatctc    1020

tcaagtgaag aactcaatta taggattcac cttaaaggac ttacagggac agccggcaaa    1080

ataagcagca tcagccaacc aggaaatgat tttagcacaa aggatggaga caacgacaaa    1140

tgtatttgca aatgttcaca aatgctaaca ggaggctggt ggtttgatgc atgtggtcct    1200

tccaacttga acggaatgta ctatccacag aggcagaaca caaataagtt caacggcatt    1260

aaatggtact actggaaagg ctcaggctat tcgctcaagg ccacaaccat gatgatccga    1320

ccagcagatt tctaa                                                      1335
```

<210> 48
<211> 444
<212> PRT
<213> Homo sapiens

<400> 48

```
Met Trp Gln Ile Val Phe Phe Thr Leu Ser Cys Asp Leu Val Leu Ala
1               5                   10                  15

Ala Ala Tyr Asn Asn Phe Arg Lys Ser Met Asp Ser Ile Gly Lys Lys
            20                  25                  30

Gln Tyr Gln Val Gln His Gly Ser Cys Ser Tyr Thr Phe Leu Leu Pro
        35                  40                  45

Glu Met Asp Asn Cys Arg Ser Ser Ser Ser Pro Tyr Val Ser Asn Ala
    50                  55                  60

Val Gln Arg Asp Ala Pro Leu Glu Tyr Asp Asp Ser Val Gln Arg Leu
65                  70                  75                  80

Gln Val Leu Glu Asn Ile Met Glu Asn Asn Thr Gln Trp Leu Met Lys
                85                  90                  95

Val Leu Asn Gln Thr Thr Arg Leu Glu Leu Gln Leu Leu Glu His Ser
            100                 105                 110
```

```
Leu Ser Thr Asn Lys Leu Glu Lys Gln Ile Leu Asp Gln Thr Ser Glu
        115                 120                 125

Ile Asn Lys Leu Gln Asp Lys Asn Ser Phe Leu Glu Lys Lys Val Leu
    130                 135                 140

Ala Met Glu Asp Lys His Ile Ile Gln Leu Gln Ser Ile Lys Glu Glu
145                 150                 155                 160

Lys Asp Gln Leu Gln Val Leu Val Ser Lys Gln Asn Ser Ile Ile Glu
                165                 170                 175

Glu Leu Glu Lys Lys Ile Val Thr Ala Thr Val Asn Asn Ser Val Leu
            180                 185                 190

Gln Lys Gln Gln His Asp Leu Met Glu Thr Val Asn Asn Leu Leu Thr
        195                 200                 205

Met Met Ser Thr Ser Asn Ser Ala Lys Asp Pro Thr Val Ala Lys Glu
    210                 215                 220

Glu Gln Ile Ser Phe Arg Asp Cys Ala Glu Val Phe Lys Ser Gly His
225                 230                 235                 240

Thr Thr Asn Gly Ile Tyr Thr Leu Thr Phe Pro Asn Ser Thr Glu Glu
                245                 250                 255

Ile Lys Ala Tyr Cys Asp Met Glu Ala Gly Gly Gly Gly Trp Thr Ile
            260                 265                 270

Ile Gln Arg Arg Glu Asp Gly Ser Val Asp Phe Gln Arg Thr Trp Lys
        275                 280                 285

Glu Tyr Lys Val Gly Phe Gly Asn Pro Ser Gly Glu Tyr Trp Leu Gly
    290                 295                 300

Asn Glu Phe Val Ser Gln Leu Thr Asn Gln Gln Arg Tyr Val Leu Lys
305                 310                 315                 320

Ile His Leu Lys Asp Trp Glu Gly Asn Glu Ala Tyr Ser Leu Tyr Glu
                325                 330                 335

His Phe Tyr Leu Ser Ser Glu Glu Leu Asn Tyr Arg Ile His Leu Lys
            340                 345                 350

Gly Leu Thr Gly Thr Ala Gly Lys Ile Ser Ser Ile Ser Gln Pro Gly
        355                 360                 365
```

Asn Asp Phe Ser Thr Lys Asp Gly Asp Asn Asp Lys Cys Ile Cys Lys
    370                 375                 380

Cys Ser Gln Met Leu Thr Gly Gly Trp Trp Phe Asp Ala Cys Gly Pro
385                 390                 395                 400

Ser Asn Leu Asn Gly Met Tyr Tyr Pro Gln Arg Gln Asn Thr Asn Lys
                405                 410                 415

Phe Asn Gly Ile Lys Trp Tyr Tyr Trp Lys Gly Ser Gly Tyr Ser Leu
            420                 425                 430

Lys Ala Thr Thr Met Met Ile Arg Pro Ala Asp Phe
        435                 440

<210> 49
<211> 1854
<212> DNA
<213> Homo sapiens

<400> 49

```
atggctgaag caaagaccca ctggcttgga gcagccctgt ctcttatccc tttaattttc      60
ctcatctctg ggggctgaagc agcttcattt cagagaaacc agctgcttca gaaagaacca    120
gacctcaggt tggaaaatgt ccaaaagttt cccagtcctg aaatgatcag ggctttggag    180
tacatagaaa acctccgaca acaagctcat aaggaagaaa gcagcccaga ttataatccc    240
taccaaggtg tctctgtccc ccttcagcaa aaagaaaatg gcgatgaaag ccacttgccc    300
gagagggatt cactgagtga agaagactgg atgagaataa tactcgaagc tttgagacag    360
gctgaaaatg agcctcagtc tgcaccaaaa gaaaataagc cctatgcctt gaattcagaa    420
aagaactttc caatggacat gagtgatgat tatgagacac agcagtggcc agaaagaaag    480
cttaagcaca tgcaattccc tcctatgtat gaagagaatt ccagggataa cccctttaaa    540
cgcacaaatg aaatagtgga ggaacaatat actcctcaaa gccttgctac attggaatct    600
gtcttccaag agctggggaa actgacagga ccaaacaacc agaaacgtga gaggatggat    660
gaggagcaaa aactttatac ggatgatgaa gatgatatct acaaggctaa taacattgcc    720
tatgaagatg tggtcggggg agaagactgg aacccagtag aggagaaaat agagagtcaa    780
acccaggaag aggtgagaga cagcaaagag aatatagaaa aaaatgaaca aatcaacgat    840
gagatgaaac gctcagggca gcttggcatc caggaagaag atcttcggaa agagagtaaa    900
gaccaactct cagatgatgt ctccaaagta attgcctatt tgaaaaggtt agtaaatgct    960
gcaggaagtg ggaggttaca gaatgggcaa aatggggaaa gggccaccag gctttttgag   1020
aaacctcttg attctcagtc tatttatcag ctgattgaaa tctcaaggaa tttacagata   1080
```

```
cccccagaag acttaattga gatgctcaaa actggggaga agccgaatgg atcagtggaa   1140
ccggagcggg agcttgacct tcctgttgac ctagatgaca tctcagaggc tgacttagac   1200
catccagacc tgttccaaaa taggatgctc tccaagagtg gctaccctaa aacacctggt   1260
cgtgctggga ctgaggccct accagacggg ctcagtgttg aggatatttt aaatctttta   1320
gggatggaga gtgcagcaaa tcagaaaacg tcgtattttc ccaatccata taaccaggag   1380
aaagttctgc caaggctccc ttatggtgct ggaagatcta gatcgaacca gcttcccaaa   1440
gctgcctgga ttccacatgt tgaaaacaga cagatggcat atgaaaacct gaacgacaag   1500
gatcaagaat taggtgagta cttggccagg atgctagtta aataccctga gatcattaat   1560
tcaaaccaag tgaagcgagt tcctggtcaa ggctcatctg aagatgacct gcaggaagag   1620
gaacaaattg agcaggccat caaagagcat ttgaatcaag gcagctctca ggagactgac   1680
aagctggccc cggtgagcaa aaggttccct gtggggcccc cgaagaatga tgatacccca   1740
aataggcagt actgggatga agatctgtta atgaaagtgc tggaatacct caaccaagaa   1800
aaggcagaaa agggaaggga gcatattgct aagagagcaa tggaaaatat gtaa         1854
```

<210> 50
<211> 617
<212> PRT
<213> Homo sapiens

<400> 50

```
Met Ala Glu Ala Lys Thr His Trp Leu Gly Ala Ala Leu Ser Leu Ile
1               5                   10                  15

Pro Leu Ile Phe Leu Ile Ser Gly Ala Glu Ala Ala Ser Phe Gln Arg
            20                  25                  30

Asn Gln Leu Leu Gln Lys Glu Pro Asp Leu Arg Leu Glu Asn Val Gln
        35                  40                  45

Lys Phe Pro Ser Pro Glu Met Ile Arg Ala Leu Glu Tyr Ile Glu Asn
    50                  55                  60

Leu Arg Gln Gln Ala His Lys Glu Glu Ser Ser Pro Asp Tyr Asn Pro
65                  70                  75                  80

Tyr Gln Gly Val Ser Val Pro Leu Gln Gln Lys Glu Asn Gly Asp Glu
                85                  90                  95

Ser His Leu Pro Glu Arg Asp Ser Leu Ser Glu Glu Asp Trp Met Arg
            100                 105                 110

Ile Ile Leu Glu Ala Leu Arg Gln Ala Glu Asn Glu Pro Gln Ser Ala
```

```
        115                 120                 125

Pro Lys Glu Asn Lys Pro Tyr Ala Leu Asn Ser Glu Lys Asn Phe Pro
    130                 135                 140

Met Asp Met Ser Asp Asp Tyr Glu Thr Gln Gln Trp Pro Glu Arg Lys
145                 150                 155                 160

Leu Lys His Met Gln Phe Pro Pro Met Tyr Glu Glu Asn Ser Arg Asp
                165                 170                 175

Asn Pro Phe Lys Arg Thr Asn Glu Ile Val Glu Glu Gln Tyr Thr Pro
            180                 185                 190

Gln Ser Leu Ala Thr Leu Glu Ser Val Phe Gln Glu Leu Gly Lys Leu
        195                 200                 205

Thr Gly Pro Asn Asn Gln Lys Arg Glu Arg Met Asp Glu Glu Gln Lys
    210                 215                 220

Leu Tyr Thr Asp Asp Glu Asp Asp Ile Tyr Lys Ala Asn Asn Ile Ala
225                 230                 235                 240

Tyr Glu Asp Val Val Gly Gly Glu Asp Trp Asn Pro Val Glu Glu Lys
                245                 250                 255

Ile Glu Ser Gln Thr Gln Glu Glu Val Arg Asp Ser Lys Glu Asn Ile
            260                 265                 270

Glu Lys Asn Glu Gln Ile Asn Asp Glu Met Lys Arg Ser Gly Gln Leu
        275                 280                 285

Gly Ile Gln Glu Glu Asp Leu Arg Lys Glu Ser Lys Asp Gln Leu Ser
    290                 295                 300

Asp Asp Val Ser Lys Val Ile Ala Tyr Leu Lys Arg Leu Val Asn Ala
305                 310                 315                 320

Ala Gly Ser Gly Arg Leu Gln Asn Gly Gln Asn Gly Glu Arg Ala Thr
                325                 330                 335

Arg Leu Phe Glu Lys Pro Leu Asp Ser Gln Ser Ile Tyr Gln Leu Ile
            340                 345                 350

Glu Ile Ser Arg Asn Leu Gln Ile Pro Pro Glu Asp Leu Ile Glu Met
        355                 360                 365
```

Leu Lys Thr Gly Glu Lys Pro Asn Gly Ser Val Glu Pro Glu Arg Glu
370 375 380

Leu Asp Leu Pro Val Asp Leu Asp Asp Ile Ser Glu Ala Asp Leu Asp
385 390 395 400

His Pro Asp Leu Phe Gln Asn Arg Met Leu Ser Lys Ser Gly Tyr Pro
405 410 415

Lys Thr Pro Gly Arg Ala Gly Thr Glu Ala Leu Pro Asp Gly Leu Ser
420 425 430

Val Glu Asp Ile Leu Asn Leu Leu Gly Met Glu Ser Ala Ala Asn Gln
435 440 445

Lys Thr Ser Tyr Phe Pro Asn Pro Tyr Asn Gln Glu Lys Val Leu Pro
450 455 460

Arg Leu Pro Tyr Gly Ala Gly Arg Ser Arg Ser Asn Gln Leu Pro Lys
465 470 475 480

Ala Ala Trp Ile Pro His Val Glu Asn Arg Gln Met Ala Tyr Glu Asn
485 490 495

Leu Asn Asp Lys Asp Gln Glu Leu Gly Glu Tyr Leu Ala Arg Met Leu
500 505 510

Val Lys Tyr Pro Glu Ile Ile Asn Ser Asn Gln Val Lys Arg Val Pro
515 520 525

Gly Gln Gly Ser Ser Glu Asp Asp Leu Gln Glu Glu Glu Gln Ile Glu
530 535 540

Gln Ala Ile Lys Glu His Leu Asn Gln Gly Ser Ser Gln Glu Thr Asp
545 550 555 560

Lys Leu Ala Pro Val Ser Lys Arg Phe Pro Val Gly Pro Pro Lys Asn
565 570 575

Asp Asp Thr Pro Asn Arg Gln Tyr Trp Asp Glu Asp Leu Leu Met Lys
580 585 590

Val Leu Glu Tyr Leu Asn Gln Glu Lys Ala Glu Lys Gly Arg Glu His
595 600 605

Ile Ala Lys Arg Ala Met Glu Asn Met
610 615

<210> 51
<211> 627
<212> DNA
<213> Homo sapiens

<400> 51

```
atggctccct taggtgaagt tgggaactat ttcggtgtgc aggatgcggt accgtttggg      60
aatgtgcccg tgttgccggt ggacagcccg gttttgttaa gtgaccacct gggtcagtcc     120
gaagcagggg ggctccccag gggacccgca gtcacggact tggatcattt aaaggggatt     180
ctcaggcgga ggcagctata ctgcaggact ggatttcact tagaaatctt ccccaatggt     240
actatccagg gaaccaggaa agaccacagc cgatttggca ttctggaatt tatcagtata     300
gcagtgggcc tggtcagcat tcgaggcgtg gacagtggac tctacctcgg gatgaatgag     360
aagggggagc tgtatggatc agaaaaacta acccaagagt gtgtattcag agaacagttc     420
gaagaaaact ggtataatac gtactcatca aacctatata agcacgtgga cactggaagg     480
cgatactatg ttgcattaaa taaagatggg accccgagag aagggactag gactaaacgg     540
caccagaaat tcacacattt tttacctaga ccagtggacc ccgacaaagt acctgaactg     600
tataaggata ttctaagcca aagttga                                         627
```

<210> 52
<211> 208
<212> PRT
<213> Homo sapiens

<400> 52

```
Met Ala Pro Leu Gly Glu Val Gly Asn Tyr Phe Gly Val Gln Asp Ala
1               5                   10                  15

Val Pro Phe Gly Asn Val Pro Val Leu Pro Val Asp Ser Pro Val Leu
            20                  25                  30

Leu Ser Asp His Leu Gly Gln Ser Glu Ala Gly Gly Leu Pro Arg Gly
        35                  40                  45

Pro Ala Val Thr Asp Leu Asp His Leu Lys Gly Ile Leu Arg Arg Arg
    50                  55                  60

Gln Leu Tyr Cys Arg Thr Gly Phe His Leu Glu Ile Phe Pro Asn Gly
65                  70                  75                  80

Thr Ile Gln Gly Thr Arg Lys Asp His Ser Arg Phe Gly Ile Leu Glu
                85                  90                  95

Phe Ile Ser Ile Ala Val Gly Leu Val Ser Ile Arg Gly Val Asp Ser
            100                 105                 110

Gly Leu Tyr Leu Gly Met Asn Glu Lys Gly Glu Leu Tyr Gly Ser Glu
        115                 120                 125

Lys Leu Thr Gln Glu Cys Val Phe Arg Glu Gln Phe Glu Glu Asn Trp
    130                 135                 140

Tyr Asn Thr Tyr Ser Ser Asn Leu Tyr Lys His Val Asp Thr Gly Arg
145                 150                 155                 160

Arg Tyr Tyr Val Ala Leu Asn Lys Asp Gly Thr Pro Arg Glu Gly Thr
                165                 170                 175

Arg Thr Lys Arg His Gln Lys Phe Thr His Phe Leu Pro Arg Pro Val
            180                 185                 190

Asp Pro Asp Lys Val Pro Glu Leu Tyr Lys Asp Ile Leu Ser Gln Ser
        195                 200                 205
```

<210> 53
<211> 1902
<212> DNA
<213> Homo sapiens

<400> 53

```
atggagcctg cacccgcccg atctccgagg ccccagcagg accccgcccg gccccaggag     60
cccaccatgc ctccccccga gaccccctct gaaggccgcc agcccagccc cagccccagc    120
cctacagagc gagcccccgc ttcggaggag gagttccagt ttctgcgctg ccagcaatgc    180
caggcggaag ccaagtgccc gaagctgctg ccttgtctgc acacgctgtg ctcaggatgc    240
ctggaggcgt cgggcatgca gtgccccatc tgccaggcgc cctggcccct aggtgcagac    300
acacccgccc tggataacgt ctttttcgag agtctgcagc ggcgcctgtc ggtgtaccgg    360
cagattgtgg atgcgcaggc tgtgtgcacc cgctgcaaag agtcggccga cttctggtgc    420
tttgagtgcg agcagctcct ctgcgccaag tgcttcgagg cacaccagtg gttcctcaag    480
cacgaggccc ggcccctagc agagctgcgc aaccagtcgg tgcgtgagtt cctggacggc    540
acccgcaaga ccaacaacat cttctgctcc aaccccaacc accgcacccc tacgctgacc    600
agcatctact gccgaggatg ttccaagccg ctgtgctgct cgtgcgcgct ccttgacagc    660
agccacagtg agctcaagtg cgacatcagc gcagagatcc agcagcgaca ggaggagctg    720
gacgccatga cgcaggcgct gcaggagcag gatagtgcct ttggcgcggt tcacgcgcag    780
atgcacgcgg ccgtcggcca gctgggccgc gcgcgtgccg agaccgagga gctgatccgc    840
gagcgcgtgc gccaggtggt agctcacgtg cgggctcagg agcgcgagct gctggaggct    900
gtggacgcgc ggtaccagcg cgactacgag gagatggcca gtcggctggg ccgcctggat    960
```

```
gctgtgctgc agcgcatccg cacgggcagc gcgctggtgc agaggatgaa gtgctacgcc   1020
tcggaccagg aggtgctgga catgcacggt ttcctgcgcc aggcgctctg ccgcctgcgc   1080
caggaggagc cccagagcct gcaagctgcc gtgcgcaccg atggcttcga cgagttcaag   1140
gtgcgcctgc aggacctcag ctcttgcatc acccagggga aagatgcagc tgtatccaag   1200
aaagccagcc cagaggctgc cagcactccc agggacccta ttgacgttga cctgcccgag   1260
gaggcagaga gagtgaaggc ccaggttcag gccctggggc tggctgaagc ccagcctatg   1320
gctgtggtac agtcagtgcc cggggcacac cccgtgccag tgtacgcctt ctccatcaaa   1380
ggcccttcct atggagagga tgtctccaat acaacgacag cccagaagag gaagtgcagc   1440
cagacccagt gccccaggaa ggtcatcaag atggagtctg aggaggggaa ggaggcaagg   1500
ttggctcgga gctccccgga gcagcccagg cccagcacct ccaaggcagt ctcaccaccc   1560
cacctggatg gaccgcctag ccccaggagc cccgtcatag gaagtgaggt cttcctgccc   1620
aacagcaacc acgtggccag tggcgccggg gaggcagagg aacgcgttgt ggtgatcagc   1680
agctcggaag actcagatgc cgaaaactcg tcctcccgag agctggatga cagcagcagt   1740
gagtccagtg acctccagct ggaaggcccc agcaccctca gggtcctgga cgagaacctt   1800
gctgaccccc aagcagaaga cagacctctg gttttctttg acctcaagat tgacaatgaa   1860
agtgggttct cctggggcta cccccacccc tttctaattt ag                      1902
```

<210> 54
<211> 633
<212> PRT
<213> Homo sapiens

<400> 54

```
Met Glu Pro Ala Pro Ala Arg Ser Pro Arg Pro Gln Gln Asp Pro Ala
1               5                   10                  15

Arg Pro Gln Glu Pro Thr Met Pro Pro Pro Glu Thr Pro Ser Glu Gly
            20                  25                  30

Arg Gln Pro Ser Pro Ser Pro Ser Pro Thr Glu Arg Ala Pro Ala Ser
        35                  40                  45

Glu Glu Glu Phe Gln Phe Leu Arg Cys Gln Gln Cys Gln Ala Glu Ala
    50                  55                  60

Lys Cys Pro Lys Leu Leu Pro Cys Leu His Thr Leu Cys Ser Gly Cys
65                  70                  75                  80

Leu Glu Ala Ser Gly Met Gln Cys Pro Ile Cys Gln Ala Pro Trp Pro
                85                  90                  95
```

```
Leu Gly Ala Asp Thr Pro Ala Leu Asp Asn Val Phe Phe Glu Ser Leu
            100                 105                 110

Gln Arg Arg Leu Ser Val Tyr Arg Gln Ile Val Asp Ala Gln Ala Val
        115                 120                 125

Cys Thr Arg Cys Lys Glu Ser Ala Asp Phe Trp Cys Phe Glu Cys Glu
    130                 135                 140

Gln Leu Leu Cys Ala Lys Cys Phe Glu Ala His Gln Trp Phe Leu Lys
145                 150                 155                 160

His Glu Ala Arg Pro Leu Ala Glu Leu Arg Asn Gln Ser Val Arg Glu
                165                 170                 175

Phe Leu Asp Gly Thr Arg Lys Thr Asn Asn Ile Phe Cys Ser Asn Pro
            180                 185                 190

Asn His Arg Thr Pro Thr Leu Thr Ser Ile Tyr Cys Arg Gly Cys Ser
        195                 200                 205

Lys Pro Leu Cys Cys Ser Cys Ala Leu Leu Asp Ser Ser His Ser Glu
    210                 215                 220

Leu Lys Cys Asp Ile Ser Ala Glu Ile Gln Gln Arg Gln Glu Glu Leu
225                 230                 235                 240

Asp Ala Met Thr Gln Ala Leu Gln Glu Gln Asp Ser Ala Phe Gly Ala
                245                 250                 255

Val His Ala Gln Met His Ala Ala Val Gly Gln Leu Gly Arg Ala Arg
            260                 265                 270

Ala Glu Thr Glu Glu Leu Ile Arg Glu Arg Val Arg Gln Val Val Ala
        275                 280                 285

His Val Arg Ala Gln Glu Arg Glu Leu Leu Glu Ala Val Asp Ala Arg
    290                 295                 300

Tyr Gln Arg Asp Tyr Glu Glu Met Ala Ser Arg Leu Gly Arg Leu Asp
305                 310                 315                 320

Ala Val Leu Gln Arg Ile Arg Thr Gly Ser Ala Leu Val Gln Arg Met
                325                 330                 335

Lys Cys Tyr Ala Ser Asp Gln Glu Val Leu Asp Met His Gly Phe Leu
```

```
            340                 345                 350

Arg Gln Ala Leu Cys Arg Leu Arg Gln Glu Glu Pro Gln Ser Leu Gln
        355                 360                 365

Ala Ala Val Arg Thr Asp Gly Phe Asp Glu Phe Lys Val Arg Leu Gln
    370                 375                 380

Asp Leu Ser Ser Cys Ile Thr Gln Gly Lys Asp Ala Ala Val Ser Lys
385                 390                 395                 400

Lys Ala Ser Pro Glu Ala Ala Ser Thr Pro Arg Asp Pro Ile Asp Val
                405                 410                 415

Asp Leu Pro Glu Glu Ala Glu Arg Val Lys Ala Gln Val Gln Ala Leu
            420                 425                 430

Gly Leu Ala Glu Ala Gln Pro Met Ala Val Val Gln Ser Val Pro Gly
        435                 440                 445

Ala His Pro Val Pro Val Tyr Ala Phe Ser Ile Lys Gly Pro Ser Tyr
    450                 455                 460

Gly Glu Asp Val Ser Asn Thr Thr Thr Ala Gln Lys Arg Lys Cys Ser
465                 470                 475                 480

Gln Thr Gln Cys Pro Arg Lys Val Ile Lys Met Glu Ser Glu Glu Gly
                485                 490                 495

Lys Glu Ala Arg Leu Ala Arg Ser Ser Pro Glu Gln Pro Arg Pro Ser
            500                 505                 510

Thr Ser Lys Ala Val Ser Pro Pro His Leu Asp Gly Pro Pro Ser Pro
        515                 520                 525

Arg Ser Pro Val Ile Gly Ser Glu Val Phe Leu Pro Asn Ser Asn His
    530                 535                 540

Val Ala Ser Gly Ala Gly Glu Ala Glu Glu Arg Val Val Val Ile Ser
545                 550                 555                 560

Ser Ser Glu Asp Ser Asp Ala Glu Asn Ser Ser Ser Arg Glu Leu Asp
                565                 570                 575

Asp Ser Ser Ser Glu Ser Ser Asp Leu Gln Leu Glu Gly Pro Ser Thr
            580                 585                 590
```

```
Leu Arg Val Leu Asp Glu Asn Leu Ala Asp Pro Gln Ala Glu Asp Arg
        595                 600                 605

Pro Leu Val Phe Phe Asp Leu Lys Ile Asp Asn Glu Ser Gly Phe Ser
    610                 615                 620

Trp Gly Tyr Pro His Pro Phe Leu Ile
625                 630
```

<210> 55
<211> 2421
<212> DNA
<213> Homo sapiens

<400> 55

```
atgggcgccc ctgcctgcgc cctcgcgctc tgcgtggccg tggccatcgt ggccggcgcc     60
tcctcggagt ccttggggac ggagcagcgc gtcgtggggc gagcggcaga agtcccgggc    120
ccagagcccg gccagcagga gcagttggtc ttcggcagcg gggatgctgt ggagctgagc    180
tgtcccccgc ccgggggtgg tcccatgggg cccactgtct gggtcaagga tggcacaggg    240
ctggtgccct cggagcgtgt cctggtgggg ccccagcggc tgcaggtgct gaatgcctcc    300
cacgaggact ccggggccta cagctgccgg cagcggctca cgcagcgcgt actgtgccac    360
ttcagtgtgc gggtgacaga cgctccatcc tcgggagatg acgaagacgg ggaggacgag    420
gctgaggaca caggtgtgga cacaggggcc ccttactgga cacggcccga gcggatggac    480
aagaagctgc tggccgtgcc ggccgccaac accgtccgct tccgctgccc agccgctggc    540
aaccccactc cctccatctc ctggctgaag aacggcaggg agttccgcgg cgagcaccgc    600
attggaggca tcaagctgcg gcatcagcag tggagcctgg tcatggaaag cgtggtgccc    660
tcggaccgcg gcaactacac ctgcgtcgtg gagaacaagt ttggcagcat ccggcagacg    720
tacacgctgg acgtgctgga gcgctccccg caccggccca tcctgcaggc ggggctgccg    780
gccaaccaga cggcggtgct gggcagcgac gtggagttcc actgcaaggt gtacagtgac    840
gcacagcccc acatccagtg gctcaagcac gtggaggtga atggcagcaa ggtgggcccg    900
gacggcacac cctacgttac cgtgctcaag acggcgggcg ctaacaccac cgacaaggag    960
ctagaggttc tctccttgca caacgtcacc tttgaggacg ccggggagta cacctgcctg   1020
gcgggcaatt ctattgggtt ttctcatcac tctgcgtggc tggtggtgct gccagccgag   1080
gaggagctgg tggaggctga cgaggcgggc agtgtgtatg caggcatcct cagctacggg   1140
gtgggcttct tcctgttcat cctggtggtg gcggctgtga cgctctgccg cctgcgcagc   1200
ccccccaaga aaggcctggg ctcccccacc gtgcacaaga tctcccgctt cccgctcaag   1260
cgacaggtgt ccctggagtc caacgcgtcc atgagctcca acacaccact ggtgcgcatc   1320
gcaaggctgt cctcagggga gggccccacg ctggccaatg tctccgagct cgagctgcct   1380
```

```
gccgacccca aatgggagct gtctcgggcc cggctgaccc tgggcaagcc ccttggggag   1440
ggctgcttcg gccaggtggt catggcggag gccatcggca ttgacaagga ccgggccgcc   1500
aagcctgtca ccgtagccgt gaagatgctg aaagacgatg ccactgacaa ggacctgtcg   1560
gacctggtgt ctgagatgga gatgatgaag atgatcggga aacacaaaaa catcatcaac   1620
ctgctgggcg cctgcacgca gggcgggccc ctgtacgtgc tggtggagta cgcggccaag   1680
ggtaacctgc gggagtttct gcgggcgcgg cggcccccgg gcctggacta ctccttcgac   1740
acctgcaagc cgcccgagga gcagctcacc ttcaaggacc tggtgtcctg tgcctaccag   1800
gtggcccggg gcatggagta cttggcctcc cagaagtgca tccacaggga cctggctgcc   1860
cgcaatgtgc tggtgaccga ggacaacgtg atgaagatcg cagacttcgg gctggcccgg   1920
gacgtgcaca acctcgacta ctacaagaag acgaccaacg gccggctgcc cgtgaagtgg   1980
atggcgcctg aggccttgtt tgaccgagtc tacactcacc agagtgacgt ctggtccttt   2040
ggggtcctgc tctgggagat cttcacgctg gggggctccc cgtaccccgg catccctgtg   2100
gaggagctct tcaagctgct gaaggagggc caccgcatgg acaagcccgc caactgcaca   2160
cacgacctgt acatgatcat gcgggagtgc tggcatgccg cgccctccca gaggcccacc   2220
ttcaagcagc tggtggagga cctggaccgt gtccttaccg tgacgtccac cgacgagtac   2280
ctggacctgt cggcgccttt cgagcagtac tccccgggtg gccaggacac ccccagctcc   2340
agctcctcag gggacgactc cgtgtttgcc cacgacctgc tgcccccggc cccacccagc   2400
agtgggggct cgcggacgtg a                                             2421
```

<210> 56
<211> 806
<212> PRT
<213> Homo sapiens

<400> 56

```
Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80
```

```
Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
```

325 330 335

Tyr Thr Cys Leu Ala Gly Asn Ser Ile Gly Phe Ser His His Ser Ala
340 345 350

Trp Leu Val Val Leu Pro Ala Glu Glu Glu Leu Val Glu Ala Asp Glu
355 360 365

Ala Gly Ser Val Tyr Ala Gly Ile Leu Ser Tyr Gly Val Gly Phe Phe
370 375 380

Leu Phe Ile Leu Val Val Ala Ala Val Thr Leu Cys Arg Leu Arg Ser
385 390 395 400

Pro Pro Lys Lys Gly Leu Gly Ser Pro Thr Val His Lys Ile Ser Arg
405 410 415

Phe Pro Leu Lys Arg Gln Val Ser Leu Glu Ser Asn Ala Ser Met Ser
420 425 430

Ser Asn Thr Pro Leu Val Arg Ile Ala Arg Leu Ser Ser Gly Glu Gly
435 440 445

Pro Thr Leu Ala Asn Val Ser Glu Leu Glu Leu Pro Ala Asp Pro Lys
450 455 460

Trp Glu Leu Ser Arg Ala Arg Leu Thr Leu Gly Lys Pro Leu Gly Glu
465 470 475 480

Gly Cys Phe Gly Gln Val Val Met Ala Glu Ala Ile Gly Ile Asp Lys
485 490 495

Asp Arg Ala Ala Lys Pro Val Thr Val Ala Val Lys Met Leu Lys Asp
500 505 510

Asp Ala Thr Asp Lys Asp Leu Ser Asp Leu Val Ser Glu Met Glu Met
515 520 525

Met Lys Met Ile Gly Lys His Lys Asn Ile Ile Asn Leu Leu Gly Ala
530 535 540

Cys Thr Gln Gly Gly Pro Leu Tyr Val Leu Val Glu Tyr Ala Ala Lys
545 550 555 560

Gly Asn Leu Arg Glu Phe Leu Arg Ala Arg Arg Pro Pro Gly Leu Asp
565 570 575

```
Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Glu Tyr Leu Asp Leu Ser Ala Pro Phe Glu
        755                 760                 765

Gln Tyr Ser Pro Gly Gly Gln Asp Thr Pro Ser Ser Ser Ser Ser Gly
    770                 775                 780

Asp Asp Ser Val Phe Ala His Asp Leu Leu Pro Pro Ala Pro Pro Ser
785                 790                 795                 800

Ser Gly Gly Ser Arg Thr
                805
```

<210> 57
<211> 2115

<212> DNA
<213> Homo sapiens

<400> 57

```
atggtcagct ggggtcgttt catctgcctg gtcgtggtca ccatggcaac cttgtccctg     60
gcccggccct ccttcagttt agttgaggat accacattag agccagaagg agcaccatac    120
tggaccaaca cagaaaagat ggaaaagcgg ctccatgctg tgcctgcggc caacactgtc    180
aagtttcgct gcccagccgg ggggaaccca atgccaacca tgcggtggct gaaaaacggg    240
aaggagttta agcaggagca tcgcattgga ggctacaagg tacgaaacca gcactggagc    300
ctcattatgg aaagtgtggt cccatctgac aagggaaatt atacctgtgt agtggagaat    360
gaatacgggt ccatcaatca cacgtaccac ctggatgttg tggagcgatc gcctcaccgg    420
cccatcctcc aagccggact gccggcaaat gcctccacag tggtcggagg agacgtagag    480
tttgtctgca aggtttacag tgatgcccag ccccacatcc agtggatcaa gcacgtggaa    540
aagaacggca gtaaatacgg gcccgacggg ctgccctacc tcaaggttct caaggccgcc    600
ggtgttaaca ccacggacaa agagattgag gttctctata ttcggaatgt aacttttgag    660
gacgctgggg aatatacgtg cttggcgggt aattctattg ggatatcctt tcactctgca    720
tggttgacag ttctgccagc gcctggaaga gaaaaggaga ttacagcttc cccagactac    780
ctggagatag ccatttactg catagggggtc ttcttaatcg cctgtatggt ggtaacagtc    840
atcctgtgcc gaatgaagaa cacgaccaag aagccagact tcagcagcca gccggctgtg    900
cacaagctga ccaaacgtat ccccctgcgg agacaggttt cggctgagtc cagctcctcc    960
atgaactcca acaccccgct ggtgaggata acaacacgcc tctcttcaac ggcagacacc   1020
cccatgctgg caggggtctc cgagtatgaa cttccagagg acccaaaatg ggagtttcca   1080
agagataagc tgacactggg caagcccctg ggagaaggtt gctttgggca agtggtcatg   1140
gcggaagcag tgggaattga caaagacaag cccaaggagg cggtcaccgt ggccgtgaag   1200
atgttgaaag atgatgccac agagaaagac ctttctgatc tggtgtcaga gatggagatg   1260
atgaagatga ttgggaaaca caagaatatc ataaatcttc ttggagcctg cacacaggat   1320
gggcctctct atgtcatagt tgagtatgcc tctaaaggca acctccgaga atacctccga   1380
gcccggaggc cacccgggat ggagtactcc tatgacatta accgtgttcc tgaggagcag   1440
atgaccttca aggacttggt gtcatgcacc taccagctgg ccagaggcat ggagtacttg   1500
gcttcccaaa aatgtattca tcgagattta gcagccagaa atgttttggt aacagaaaac   1560
aatgtgatga aaatagcaga ctttggactc gccagagata tcaacaatat agactattac   1620
aaaaagacca ccaatgggcg gcttccagtc aagtggatgg ctccagaagc cctgtttgat   1680
agagtataca ctcatcagag tgatgtctgg tccttcgggg tgttaatgtg ggagatcttc   1740
actttagggg gctcgcccta cccagggatt cccgtggagg aactttttaa gctgctgaag   1800
```

gaaggacaca gaatggataa gccagccaac tgcaccaacg aactgtacat gatgatgagg 1860

gactgttggc atgcagtgcc ctcccagaga ccaacgttca agcagttggt agaagacttg 1920

gatcgaattc tcactctcac aaccaatgag gaatacttgg acctcagcca acctctcgaa 1980

cagtattcac ctagttaccc tgacacaaga agttcttgtt cttcaggaga tgattctgtt 2040

ttttctccag accccatgcc ttacgaacca tgccttcctc agtatccaca cataaacggc 2100

agtgttaaaa catga 2115

<210> 58
<211> 704
<212> PRT
<213> Homo sapiens

<400> 58

```
Met Val Ser Trp Gly Arg Phe Ile Cys Leu Val Val Val Thr Met Ala
1               5                   10                  15

Thr Leu Ser Leu Ala Arg Pro Ser Phe Ser Leu Val Glu Asp Thr Thr
            20                  25                  30

Leu Glu Pro Glu Gly Ala Pro Tyr Trp Thr Asn Thr Glu Lys Met Glu
        35                  40                  45

Lys Arg Leu His Ala Val Pro Ala Ala Asn Thr Val Lys Phe Arg Cys
    50                  55                  60

Pro Ala Gly Gly Asn Pro Met Pro Thr Met Arg Trp Leu Lys Asn Gly
65                  70                  75                  80

Lys Glu Phe Lys Gln Glu His Arg Ile Gly Gly Tyr Lys Val Arg Asn
                85                  90                  95

Gln His Trp Ser Leu Ile Met Glu Ser Val Val Pro Ser Asp Lys Gly
            100                 105                 110

Asn Tyr Thr Cys Val Val Glu Asn Glu Tyr Gly Ser Ile Asn His Thr
        115                 120                 125

Tyr His Leu Asp Val Val Glu Arg Ser Pro His Arg Pro Ile Leu Gln
    130                 135                 140

Ala Gly Leu Pro Ala Asn Ala Ser Thr Val Val Gly Gly Asp Val Glu
145                 150                 155                 160

Phe Val Cys Lys Val Tyr Ser Asp Ala Gln Pro His Ile Gln Trp Ile
                165                 170                 175
```

```
Lys His Val Glu Lys Asn Gly Ser Lys Tyr Gly Pro Asp Gly Leu Pro
            180                 185                 190

Tyr Leu Lys Val Leu Lys Ala Ala Gly Val Asn Thr Thr Asp Lys Glu
        195                 200                 205

Ile Glu Val Leu Tyr Ile Arg Asn Val Thr Phe Glu Asp Ala Gly Glu
    210                 215                 220

Tyr Thr Cys Leu Ala Gly Asn Ser Ile Gly Ile Ser Phe His Ser Ala
225                 230                 235                 240

Trp Leu Thr Val Leu Pro Ala Pro Gly Arg Glu Lys Glu Ile Thr Ala
                245                 250                 255

Ser Pro Asp Tyr Leu Glu Ile Ala Ile Tyr Cys Ile Gly Val Phe Leu
            260                 265                 270

Ile Ala Cys Met Val Val Thr Val Ile Leu Cys Arg Met Lys Asn Thr
        275                 280                 285

Thr Lys Lys Pro Asp Phe Ser Ser Gln Pro Ala Val His Lys Leu Thr
    290                 295                 300

Lys Arg Ile Pro Leu Arg Arg Gln Val Ser Ala Glu Ser Ser Ser Ser
305                 310                 315                 320

Met Asn Ser Asn Thr Pro Leu Val Arg Ile Thr Thr Arg Leu Ser Ser
                325                 330                 335

Thr Ala Asp Thr Pro Met Leu Ala Gly Val Ser Glu Tyr Glu Leu Pro
            340                 345                 350

Glu Asp Pro Lys Trp Glu Phe Pro Arg Asp Lys Leu Thr Leu Gly Lys
        355                 360                 365

Pro Leu Gly Glu Gly Cys Phe Gly Gln Val Val Met Ala Glu Ala Val
    370                 375                 380

Gly Ile Asp Lys Asp Lys Pro Lys Glu Ala Val Thr Val Ala Val Lys
385                 390                 395                 400

Met Leu Lys Asp Asp Ala Thr Glu Lys Asp Leu Ser Asp Leu Val Ser
                405                 410                 415

Glu Met Glu Met Met Lys Met Ile Gly Lys His Lys Asn Ile Ile Asn
            420                 425                 430
```

```
Leu Leu Gly Ala Cys Thr Gln Asp Gly Pro Leu Tyr Val Ile Val Glu
        435                 440                 445

Tyr Ala Ser Lys Gly Asn Leu Arg Glu Tyr Leu Arg Ala Arg Arg Pro
    450                 455                 460

Pro Gly Met Glu Tyr Ser Tyr Asp Ile Asn Arg Val Pro Glu Glu Gln
465                 470                 475                 480

Met Thr Phe Lys Asp Leu Val Ser Cys Thr Tyr Gln Leu Ala Arg Gly
                485                 490                 495

Met Glu Tyr Leu Ala Ser Gln Lys Cys Ile His Arg Asp Leu Ala Ala
            500                 505                 510

Arg Asn Val Leu Val Thr Glu Asn Asn Val Met Lys Ile Ala Asp Phe
        515                 520                 525

Gly Leu Ala Arg Asp Ile Asn Asn Ile Asp Tyr Tyr Lys Lys Thr Thr
    530                 535                 540

Asn Gly Arg Leu Pro Val Lys Trp Met Ala Pro Glu Ala Leu Phe Asp
545                 550                 555                 560

Arg Val Tyr Thr His Gln Ser Asp Val Trp Ser Phe Gly Val Leu Met
                565                 570                 575

Trp Glu Ile Phe Thr Leu Gly Gly Ser Pro Tyr Pro Gly Ile Pro Val
            580                 585                 590

Glu Glu Leu Phe Lys Leu Leu Lys Glu Gly His Arg Met Asp Lys Pro
        595                 600                 605

Ala Asn Cys Thr Asn Glu Leu Tyr Met Met Met Arg Asp Cys Trp His
    610                 615                 620

Ala Val Pro Ser Gln Arg Pro Thr Phe Lys Gln Leu Val Glu Asp Leu
625                 630                 635                 640

Asp Arg Ile Leu Thr Leu Thr Thr Asn Glu Glu Tyr Leu Asp Leu Ser
                645                 650                 655

Gln Pro Leu Glu Gln Tyr Ser Pro Ser Tyr Pro Asp Thr Arg Ser Ser
            660                 665                 670

Cys Ser Ser Gly Asp Asp Ser Val Phe Ser Pro Asp Pro Met Pro Tyr
```

675 680 685

Glu Pro Cys Leu Pro Gln Tyr Pro His Ile Asn Gly Ser Val Lys Thr
690 695 700

<210> 59
<211> 2463
<212> DNA
<213> Homo sapiens

<400> 59

atgtggagct ggaagtgcct cctcttctgg gctgtgctgg tcacagccac actctgcacc 60
gctaggccgt ccccgacctt gcctgaacaa gcccagccct ggggagcccc tgtggaagtg 120
gagtccttcc tggtccaccc cggtgacctg ctgcagcttc gctgtcggct gcgggacgat 180
gtgcagagca tcaactggct gcgggacggg gtgcagctgg cggaaagcaa ccgcacccgc 240
atcacagggg aggaggtgga ggtgcaggac tccgtgcccg cagactccgg cctctatgct 300
tgcgtaacca gcagcccctc gggcagtgac accacctact tctccgtcaa tgtttcagat 360
gctctcccct cctcggagga tgatgatgat gatgatgact cctcttcaga ggagaaagaa 420
acagataaca ccaaaccaaa ccgtatgccc gtagctccat attggacatc cccagaaaag 480
atggaaaaga aattgcatgc agtgccggct gccaagacag tgaagttcaa atgcccttcc 540
agtgggaccc caaaccccac actgcgctgg ttgaaaaatg gcaaagaatt caaacctgac 600
cacagaattg gaggctacaa ggtccgttat gccacctgga gcatcataat ggactctgtg 660
gtgccctctg acaagggcaa ctacacctgc attgtggaga atgagtacgg cagcatcaac 720
cacacatacc agctggatgt cgtggagcgg tcccctcacc ggcccatcct gcaagcaggg 780
ttgcccgcca acaaaacagt ggccctgggt agcaacgtgg agttcatgtg taaggtgtac 840
agtgacccgc agccgcacat ccagtggcta aagcacatcg aggtgaatgg gagcaagatt 900
ggcccagaca acctgcctta tgtccagatc ttgaagactg ctggagttaa taccaccgac 960
aaagagatgg aggtgcttca cttaagaaat gtctcctttg aggacgcagg ggagtatacg 1020
tgcttggcgg gtaactctat cggactctcc catcactctg catggttgac cgttctggaa 1080
gccctggaag agaggccggc agtgatgacc tcgcccctgt acctggagat catcatctat 1140
tgcacagggg ccttcctcat ctcctgcatg gtggggtcgg tcatcgtcta caagatgaag 1200
agtggtacca agaagagtga cttccacagc cagatggctg tgcacaagct ggccaagagc 1260
atccctctgc gcagacaggt gtctgctgac tccagtgcat ccatgaactc tggggttctt 1320
ctggttcggc catcacggct ctcctccagt gggactccca tgctagcagg ggtctctgag 1380
tatgagcttc ccgaagaccc tcgctgggag ctgcctcggg acagactggt cttaggcaaa 1440
cccctgggag agggctgctt tgggcaggtg gtgttggcag aggctatcgg gctggacaag 1500

gacaaaccca accgtgtgac caaagtggct gtgaagatgt tgaagtcgga cgcaacagag 1560

aaagacttgt cagacctgat ctcagaaatg gagatgatga agatgatcgg gaagcataag 1620

aatatcatca acctgctggg ggcctgcacg caggatggtc ccttgtatgt catcgtggag 1680

tatgcctcca agggcaacct gcgggagtac ctgcaggccc ggaggccccc agggctggaa 1740

tactgctaca accccagcca caacccagag gagcagctct cctccaagga cctggtgtcc 1800

tgcgcctacc aggtggcccg aggcatggag tatctggcct ccaagaagtg catacaccga 1860

gacctggcag ccaggaatgt cctggtgaca gaggacaatg tgatgaagat agcagacttt 1920

ggcctcgcac gggacattca ccacatcgac tactataaaa agacaaccaa cggccgactg 1980

cctgtgaagt ggatggcacc cgaggcatta tttgaccgga tctacaccca ccagagtgat 2040

gtgtggtctt tcggggtgct cctgtgggag atcttcactc tgggcggctc cccatacccc 2100

ggtgtgcctg tggaggaact tttcaagctg ctgaaggagg gtcaccgcat ggacaagccc 2160

agtaactgca ccaacgagct gtacatgatg atgcgggact gctggcatgc agtgccctca 2220

cagagaccca ccttcaagca gctggtggaa gacctggacc gcatcgtggc cttgacctcc 2280

aaccaggagt acctggacct gtccatgccc ctggaccagt actcccccag ctttcccgac 2340

acccggagct ctacgtgctc ctcaggggag gattccgtct tctctcatga gccgctgccc 2400

gaggagccct gcctgccccg acacccagcc cagcttgcca atggcggact caaacgccgc 2460

tga 2463

<210> 60
<211> 820
<212> PRT
<213> Homo sapiens

<400> 60

```
Met Trp Ser Trp Lys Cys Leu Leu Phe Trp Ala Val Leu Val Thr Ala
1               5                   10                  15

Thr Leu Cys Thr Ala Arg Pro Ser Pro Thr Leu Pro Glu Gln Ala Gln
            20                  25                  30

Pro Trp Gly Ala Pro Val Glu Val Glu Ser Phe Leu Val His Pro Gly
        35                  40                  45

Asp Leu Leu Gln Leu Arg Cys Arg Leu Arg Asp Asp Val Gln Ser Ile
    50                  55                  60

Asn Trp Leu Arg Asp Gly Val Gln Leu Ala Glu Ser Asn Arg Thr Arg
65                  70                  75                  80

Ile Thr Gly Glu Glu Val Glu Val Gln Asp Ser Val Pro Ala Asp Ser
```

85 90 95

Gly Leu Tyr Ala Cys Val Thr Ser Ser Pro Ser Gly Ser Asp Thr Thr
100 105 110

Tyr Phe Ser Val Asn Val Ser Asp Ala Leu Pro Ser Ser Glu Asp Asp
115 120 125

Asp Asp Asp Asp Asp Ser Ser Ser Glu Glu Lys Glu Thr Asp Asn Thr
130 135 140

Lys Pro Asn Arg Met Pro Val Ala Pro Tyr Trp Thr Ser Pro Glu Lys
145 150 155 160

Met Glu Lys Lys Leu His Ala Val Pro Ala Ala Lys Thr Val Lys Phe
165 170 175

Lys Cys Pro Ser Ser Gly Thr Pro Asn Pro Thr Leu Arg Trp Leu Lys
180 185 190

Asn Gly Lys Glu Phe Lys Pro Asp His Arg Ile Gly Gly Tyr Lys Val
195 200 205

Arg Tyr Ala Thr Trp Ser Ile Ile Met Asp Ser Val Val Pro Ser Asp
210 215 220

Lys Gly Asn Tyr Thr Cys Ile Val Glu Asn Glu Tyr Gly Ser Ile Asn
225 230 235 240

His Thr Tyr Gln Leu Asp Val Val Glu Arg Ser Pro His Arg Pro Ile
245 250 255

Leu Gln Ala Gly Leu Pro Ala Asn Lys Thr Val Ala Leu Gly Ser Asn
260 265 270

Val Glu Phe Met Cys Lys Val Tyr Ser Asp Pro Gln Pro His Ile Gln
275 280 285

Trp Leu Lys His Ile Glu Val Asn Gly Ser Lys Ile Gly Pro Asp Asn
290 295 300

Leu Pro Tyr Val Gln Ile Leu Lys Thr Ala Gly Val Asn Thr Thr Asp
305 310 315 320

Lys Glu Met Glu Val Leu His Leu Arg Asn Val Ser Phe Glu Asp Ala
325 330 335

```
Gly Glu Tyr Thr Cys Leu Ala Gly Asn Ser Ile Gly Leu Ser His His
            340                 345                 350

Ser Ala Trp Leu Thr Val Leu Glu Ala Leu Glu Glu Arg Pro Ala Val
        355                 360                 365

Met Thr Ser Pro Leu Tyr Leu Glu Ile Ile Ile Tyr Cys Thr Gly Ala
    370                 375                 380

Phe Leu Ile Ser Cys Met Val Gly Ser Val Ile Val Tyr Lys Met Lys
385                 390                 395                 400

Ser Gly Thr Lys Lys Ser Asp Phe His Ser Gln Met Ala Val His Lys
                405                 410                 415

Leu Ala Lys Ser Ile Pro Leu Arg Arg Gln Val Ser Ala Asp Ser Ser
            420                 425                 430

Ala Ser Met Asn Ser Gly Val Leu Leu Val Arg Pro Ser Arg Leu Ser
        435                 440                 445

Ser Ser Gly Thr Pro Met Leu Ala Gly Val Ser Glu Tyr Glu Leu Pro
    450                 455                 460

Glu Asp Pro Arg Trp Glu Leu Pro Arg Asp Arg Leu Val Leu Gly Lys
465                 470                 475                 480

Pro Leu Gly Glu Gly Cys Phe Gly Gln Val Val Leu Ala Glu Ala Ile
                485                 490                 495

Gly Leu Asp Lys Asp Lys Pro Asn Arg Val Thr Lys Val Ala Val Lys
            500                 505                 510

Met Leu Lys Ser Asp Ala Thr Glu Lys Asp Leu Ser Asp Leu Ile Ser
        515                 520                 525

Glu Met Glu Met Met Lys Met Ile Gly Lys His Lys Asn Ile Ile Asn
    530                 535                 540

Leu Leu Gly Ala Cys Thr Gln Asp Gly Pro Leu Tyr Val Ile Val Glu
545                 550                 555                 560

Tyr Ala Ser Lys Gly Asn Leu Arg Glu Tyr Leu Gln Ala Arg Arg Pro
                565                 570                 575

Pro Gly Leu Glu Tyr Cys Tyr Asn Pro Ser His Asn Pro Glu Glu Gln
            580                 585                 590
```

```
Leu Ser Ser Lys Asp Leu Val Ser Cys Ala Tyr Gln Val Ala Arg Gly
          595                 600                 605

Met Glu Tyr Leu Ala Ser Lys Lys Cys Ile His Arg Asp Leu Ala Ala
    610                 615                 620

Arg Asn Val Leu Val Thr Glu Asp Asn Val Met Lys Ile Ala Asp Phe
625                 630                 635                 640

Gly Leu Ala Arg Asp Ile His His Ile Asp Tyr Tyr Lys Lys Thr Thr
                645                 650                 655

Asn Gly Arg Leu Pro Val Lys Trp Met Ala Pro Glu Ala Leu Phe Asp
            660                 665                 670

Arg Ile Tyr Thr His Gln Ser Asp Val Trp Ser Phe Gly Val Leu Leu
        675                 680                 685

Trp Glu Ile Phe Thr Leu Gly Gly Ser Pro Tyr Pro Gly Val Pro Val
    690                 695                 700

Glu Glu Leu Phe Lys Leu Leu Lys Glu Gly His Arg Met Asp Lys Pro
705                 710                 715                 720

Ser Asn Cys Thr Asn Glu Leu Tyr Met Met Met Arg Asp Cys Trp His
                725                 730                 735

Ala Val Pro Ser Gln Arg Pro Thr Phe Lys Gln Leu Val Glu Asp Leu
            740                 745                 750

Asp Arg Ile Val Ala Leu Thr Ser Asn Gln Glu Tyr Leu Asp Leu Ser
        755                 760                 765

Met Pro Leu Asp Gln Tyr Ser Pro Ser Phe Pro Asp Thr Arg Ser Ser
    770                 775                 780

Thr Cys Ser Ser Gly Glu Asp Ser Val Phe Ser His Glu Pro Leu Pro
785                 790                 795                 800

Glu Glu Pro Cys Leu Pro Arg His Pro Ala Gln Leu Ala Asn Gly Gly
                805                 810                 815

Leu Lys Arg Arg
            820
```

<210> 61
<211> 2409

<212> DNA
<213> Homo sapiens

<400> 61

atgcggctgc tgctggccct gttgggggtc ctgctgagtg tgcctgggcc tccagtcttg 60
tccctggagg cctctgagga agtggagctt gagccctgcc tggctcccag cctggagcag 120
caagagcagg agctgacagt agcccttggg cagcctgtgc gtctgtgctg tgggcgggct 180
gagcgtggtg gccactggta caaggagggc agtcgcctgg cacctgctgg ccgtgtacgg 240
ggctggaggg gccgcctaga gattgccagc ttcctacctg aggatgctgg ccgctacctc 300
tgcctggcac gaggctccat gatcgtcctg cagaatctca ccttgattac aggtgactcc 360
ttgacctcca gcaacgatga tgaggacccc aagtcccata gggacccctc gaataggcac 420
agttaccccc agcaagcacc ctactggaca cacccccagc gcatggagaa gaaactgcat 480
gcagtacctg cggggaacac cgtcaagttc cgctgtccag ctgcaggcaa ccccacgccc 540
accatccgct ggcttaagga tggacaggcc tttcatgggg agaaccgcat tggaggcatt 600
cggctgcgcc atcagcactg gagtctcgtg atggagagcg tggtgccctc ggaccgcggc 660
acatacacct gcctggtaga gaacgctgtg ggcagcatcc gctataacta cctgctagat 720
gtgctggagc ggtccccgca ccggcccatc ctgcaggccg ggctcccggc caacaccaca 780
gccgtggtgg gcagcgacgt ggagctgctg tgcaaggtgt acagcgatgc ccagccccac 840
atccagtggc tgaagcacat cgtcatcaac ggcagcagct tcggagccga cggtttcccc 900
tatgtgcaag tcctaaagac tgcagacatc aatagctcag aggtggaggt cctgtacctg 960
cggaacgtgt cagccgagga cgcaggcgag tacacctgcc tcgcaggcaa ttccatcggc 1020
ctctcctacc agtctgcctg gctcacggtg ctgccagagg aggaccccac atggaccgca 1080
gcagcgcccg aggccaggta tacggacatc atcctgtacg cgtcgggctc cctggccttg 1140
gctgtgctcc tgctgctggc cgggctgtat cgagggcagg cgctccacgg ccggcacccc 1200
cgcccgcccg ccactgtgca gaagctctcc cgcttccctc tggcccgaca gttctccctg 1260
gagtcaggct cttccggcaa gtcaagctca tccctggtac gaggcgtgcg tctctcctcc 1320
agcggccccg ccttgctcgc cggcctcgtg agtctagatc tacctctcga cccactatgg 1380
gagttccccc gggacaggct ggtgcttggg aagcccctag gcgagggctg ctttggccag 1440
gtagtacgtg cagaggcctt tggcatggac cctgcccggc ctgaccaagc cagcactgtg 1500
gccgtcaaga tgctcaaaga caacgcctct gacaaggacc tggccgacct ggtctcggag 1560
atggaggtga tgaagctgat cggccgacac aagaacatca tcaacctgct tggtgtctgc 1620
acccaggaag ggcccctgta cgtgatcgtg gagtgcgccg ccaagggaaa cctgcgggag 1680
ttcctgcggg cccggcgccc cccaggcccc gacctcagcc ccgacggtcc tcggagcagt 1740
gaggggccgc tctccttccc agtcctggtc tcctgcgcct accaggtggc ccgaggcatg 1800

cagtatctgg agtcccggaa gtgtatccac cgggacctgg ctgcccgcaa tgtgctggtg 1860

actgaggaca atgtgatgaa gattgctgac tttgggctgg cccgcggcgt ccaccacatt 1920

gactactata agaaaaccag caacggccgc ctgcctgtga agtggatggc gcccgaggcc 1980

ttgtttgacc gggtgtacac acaccagagt gacgtgtggt cttttgggat cctgctatgg 2040

gagatcttca ccctcggggg ctccccgtat cctggcatcc cggtggagga gctgttctcg 2100

ctgctgcggg agggacatcg gatggaccga cccccacact gcccccccaga gctgtacggg 2160

ctgatgcgtg agtgctggca cgcagcgccc tcccagaggc ctaccttcaa gcagctggtg 2220

gaggcgctgg acaaggtcct gctggccgtc tctgaggagt acctcgacct ccgcctgacc 2280

ttcggaccct attcccctc tggtggggac gccagcagca cctgctcctc cagcgattct 2340

gtcttcagcc acgacccccct gccattggga tccagctcct tccccttcgg gtctggggtg 2400

cagacatga 2409

<210> 62
<211> 802
<212> PRT
<213> Homo sapiens

<400> 62

Met Arg Leu Leu Leu Ala Leu Leu Gly Val Leu Leu Ser Val Pro Gly
1 5 10 15

Pro Pro Val Leu Ser Leu Glu Ala Ser Glu Glu Val Glu Leu Glu Pro
20 25 30

Cys Leu Ala Pro Ser Leu Glu Gln Gln Glu Gln Glu Leu Thr Val Ala
35 40 45

Leu Gly Gln Pro Val Arg Leu Cys Cys Gly Arg Ala Glu Arg Gly Gly
50 55 60

His Trp Tyr Lys Glu Gly Ser Arg Leu Ala Pro Ala Gly Arg Val Arg
65 70 75 80

Gly Trp Arg Gly Arg Leu Glu Ile Ala Ser Phe Leu Pro Glu Asp Ala
85 90 95

Gly Arg Tyr Leu Cys Leu Ala Arg Gly Ser Met Ile Val Leu Gln Asn
100 105 110

Leu Thr Leu Ile Thr Gly Asp Ser Leu Thr Ser Ser Asn Asp Asp Glu
115 120 125

Asp Pro Lys Ser His Arg Asp Pro Ser Asn Arg His Ser Tyr Pro Gln
    130                 135                 140

Gln Ala Pro Tyr Trp Thr His Pro Gln Arg Met Glu Lys Lys Leu His
145                 150                 155                 160

Ala Val Pro Ala Gly Asn Thr Val Lys Phe Arg Cys Pro Ala Ala Gly
                165                 170                 175

Asn Pro Thr Pro Thr Ile Arg Trp Leu Lys Asp Gly Gln Ala Phe His
            180                 185                 190

Gly Glu Asn Arg Ile Gly Gly Ile Arg Leu Arg His Gln His Trp Ser
        195                 200                 205

Leu Val Met Glu Ser Val Val Pro Ser Asp Arg Gly Thr Tyr Thr Cys
    210                 215                 220

Leu Val Glu Asn Ala Val Gly Ser Ile Arg Tyr Asn Tyr Leu Leu Asp
225                 230                 235                 240

Val Leu Glu Arg Ser Pro His Arg Pro Ile Leu Gln Ala Gly Leu Pro
                245                 250                 255

Ala Asn Thr Thr Ala Val Val Gly Ser Asp Val Glu Leu Leu Cys Lys
            260                 265                 270

Val Tyr Ser Asp Ala Gln Pro His Ile Gln Trp Leu Lys His Ile Val
        275                 280                 285

Ile Asn Gly Ser Ser Phe Gly Ala Asp Gly Phe Pro Tyr Val Gln Val
    290                 295                 300

Leu Lys Thr Ala Asp Ile Asn Ser Ser Glu Val Glu Val Leu Tyr Leu
305                 310                 315                 320

Arg Asn Val Ser Ala Glu Asp Ala Gly Glu Tyr Thr Cys Leu Ala Gly
                325                 330                 335

Asn Ser Ile Gly Leu Ser Tyr Gln Ser Ala Trp Leu Thr Val Leu Pro
            340                 345                 350

Glu Glu Asp Pro Thr Trp Thr Ala Ala Ala Pro Glu Ala Arg Tyr Thr
        355                 360                 365

Asp Ile Ile Leu Tyr Ala Ser Gly Ser Leu Ala Leu Ala Val Leu Leu
    370                 375                 380

```
Leu Leu Ala Gly Leu Tyr Arg Gly Gln Ala Leu His Gly Arg His Pro
385                 390                 395                 400

Arg Pro Pro Ala Thr Val Gln Lys Leu Ser Arg Phe Pro Leu Ala Arg
                405                 410                 415

Gln Phe Ser Leu Glu Ser Gly Ser Ser Gly Lys Ser Ser Ser Ser Leu
            420                 425                 430

Val Arg Gly Val Arg Leu Ser Ser Ser Gly Pro Ala Leu Leu Ala Gly
        435                 440                 445

Leu Val Ser Leu Asp Leu Pro Leu Asp Pro Leu Trp Glu Phe Pro Arg
    450                 455                 460

Asp Arg Leu Val Leu Gly Lys Pro Leu Gly Glu Gly Cys Phe Gly Gln
465                 470                 475                 480

Val Val Arg Ala Glu Ala Phe Gly Met Asp Pro Ala Arg Pro Asp Gln
                485                 490                 495

Ala Ser Thr Val Ala Val Lys Met Leu Lys Asp Asn Ala Ser Asp Lys
            500                 505                 510

Asp Leu Ala Asp Leu Val Ser Glu Met Glu Val Met Lys Leu Ile Gly
        515                 520                 525

Arg His Lys Asn Ile Ile Asn Leu Leu Gly Val Cys Thr Gln Glu Gly
    530                 535                 540

Pro Leu Tyr Val Ile Val Glu Cys Ala Ala Lys Gly Asn Leu Arg Glu
545                 550                 555                 560

Phe Leu Arg Ala Arg Arg Pro Pro Gly Pro Asp Leu Ser Pro Asp Gly
                565                 570                 575

Pro Arg Ser Ser Glu Gly Pro Leu Ser Phe Pro Val Leu Val Ser Cys
            580                 585                 590

Ala Tyr Gln Val Ala Arg Gly Met Gln Tyr Leu Glu Ser Arg Lys Cys
        595                 600                 605

Ile His Arg Asp Leu Ala Ala Arg Asn Val Leu Val Thr Glu Asp Asn
    610                 615                 620

Val Met Lys Ile Ala Asp Phe Gly Leu Ala Arg Gly Val His His Ile
625                 630                 635                 640
```

Asp Tyr Tyr Lys Lys Thr Ser Asn Gly Arg Leu Pro Val Lys Trp Met
645 650 655

Ala Pro Glu Ala Leu Phe Asp Arg Val Tyr Thr His Gln Ser Asp Val
660 665 670

Trp Ser Phe Gly Ile Leu Leu Trp Glu Ile Phe Thr Leu Gly Gly Ser
675 680 685

Pro Tyr Pro Gly Ile Pro Val Glu Glu Leu Phe Ser Leu Leu Arg Glu
690 695 700

Gly His Arg Met Asp Arg Pro Pro His Cys Pro Pro Glu Leu Tyr Gly
705 710 715 720

Leu Met Arg Glu Cys Trp His Ala Ala Pro Ser Gln Arg Pro Thr Phe
725 730 735

Lys Gln Leu Val Glu Ala Leu Asp Lys Val Leu Leu Ala Val Ser Glu
740 745 750

Glu Tyr Leu Asp Leu Arg Leu Thr Phe Gly Pro Tyr Ser Pro Ser Gly
755 760 765

Gly Asp Ala Ser Ser Thr Cys Ser Ser Ser Asp Ser Val Phe Ser His
770 775 780

Asp Pro Leu Pro Leu Gly Ser Ser Ser Phe Pro Phe Gly Ser Gly Val
785 790 795 800

Gln Thr

<210> 63
<211> 2064
<212> DNA
<213> Homo sapiens

<400> 63

atggtcagct actgggacac cggggtcctg ctgtgcgcgc tgctcagctg tctgcttctc 60

acaggatcta gttcaggttc aaaattaaaa gatcctgaac tgagtttaaa aggcacccag 120

cacatcatgc aagcaggcca gacactgcat ctccaatgca gggggggaagc agcccataaa 180

tggtctttgc ctgaaatggt gagtaaggaa agcgaaaggc tgagcataac taaatctgcc 240

tgtggaagaa atggcaaaca attctgcagt actttaacct tgaacacagc tcaagcaaac 300

cacactggct tctacagctg caaatatcta gctgtaccta cttcaaagaa gaaggaaaca 360

gaatctgcaa tctatatatt tattagtgat acaggtagac ctttcgtaga gatgtacagt 420

gaaatccccg aaattataca catgactgaa ggaagggagc tcgtcattcc ctgccgggtt 480

acgtcaccta acatcactgt tactttaaaa aagtttccac ttgacacttt gatccctgat 540

ggaaaacgca taatctggga cagtagaaag ggcttcatca tatcaaatgc aacgtacaaa 600

gaaatagggc ttctgacctg tgaagcaaca gtcaatgggc atttgtataa gacaaactat 660

ctcacacatc gacaaaccaa tacaatcata gatgtccaaa taagcacacc acgcccagtc 720

aaattactta gaggccatac tcttgtcctc aattgtactg ctaccactcc cttgaacacg 780

agagttcaaa tgacctggag ttaccctgat gaaaaaaata agagagcttc cgtaaggcga 840

cgaattgacc aaagcaattc ccatgccaac atattctaca gtgttcttac tattgacaaa 900

atgcagaaca aagacaaagg actttatact tgtcgtgtaa ggagtggacc atcattcaaa 960

tctgttaaca cctcagtgca tatatatgat aaagcattca tcactgtgaa acatcgaaaa 1020

cagcaggtgc ttgaaaccgt agctggcaag cggtcttacc ggctctctat gaaagtgaag 1080

gcatttccct cgccggaagt tgtatggtta aaagatgggt tacctgcgac tgagaaatct 1140

gctcgctatt tgactcgtgg ctactcgtta attatcaagg acgtaactga agaggatgca 1200

gggaattata caatcttgct gagcataaaa cagtcaaatg tgtttaaaaa cctcactgcc 1260

actctaattg tcaatgtgaa accccagatt tacgaaaagg ccgtgtcatc gtttccagac 1320

ccggctctct acccactggg cagcagacaa atcctgactt gtaccgcata tggtatccct 1380

caacctacaa tcaagtggtt ctggcacccc tgtaaccata atcattccga agcaaggtgt 1440

gacttttgtt ccaataatga agagtccttt atcctggatg ctgacagcaa catgggaaac 1500

agaattgaga gcatcactca gcgcatggca ataatagaag gaaagaataa gatggctagc 1560

accttggttg tggctgactc tagaatttct ggaatctaca tttgcatagc ttccaataaa 1620

gttgggactg tgggaagaaa cataagcttt tatatcacag atgtgccaaa tgggtttcat 1680

gttaacttgg aaaaaatgcc gacggaagga gaggacctga aactgtcttg cacagttaac 1740

aagttcttat acagagacgt tacttggatt ttactgcgga cagttaataa cagaacaatg 1800

cactacagta ttagcaagca aaaaatggcc atcactaagg agcactccat cactcttaat 1860

cttaccatca tgaatgtttc cctgcaagat tcaggcacct atgcctgcag agccaggaat 1920

gtatacacag gggaagaaat cctccagaag aaagaaatta caatcagagg tgagcactgc 1980

aacaaaaagg ctgttttctc tcggatctcc aaatttaaaa gcacaaggaa tgattgtacc 2040

acacaaagta atgtaaaaca ttaa 2064

<210> 64
<211> 687
<212> PRT

<213> Homo sapiens

<400> 64

```
Met Val Ser Tyr Trp Asp Thr Gly Val Leu Leu Cys Ala Leu Leu Ser
1               5                   10                  15

Cys Leu Leu Leu Thr Gly Ser Ser Ser Gly Ser Lys Leu Lys Asp Pro
            20                  25                  30

Glu Leu Ser Leu Lys Gly Thr Gln His Ile Met Gln Ala Gly Gln Thr
        35                  40                  45

Leu His Leu Gln Cys Arg Gly Glu Ala Ala His Lys Trp Ser Leu Pro
    50                  55                  60

Glu Met Val Ser Lys Glu Ser Glu Arg Leu Ser Ile Thr Lys Ser Ala
65                  70                  75                  80

Cys Gly Arg Asn Gly Lys Gln Phe Cys Ser Thr Leu Thr Leu Asn Thr
                85                  90                  95

Ala Gln Ala Asn His Thr Gly Phe Tyr Ser Cys Lys Tyr Leu Ala Val
            100                 105                 110

Pro Thr Ser Lys Lys Lys Glu Thr Glu Ser Ala Ile Tyr Ile Phe Ile
        115                 120                 125

Ser Asp Thr Gly Arg Pro Phe Val Glu Met Tyr Ser Glu Ile Pro Glu
    130                 135                 140

Ile Ile His Met Thr Glu Gly Arg Glu Leu Val Ile Pro Cys Arg Val
145                 150                 155                 160

Thr Ser Pro Asn Ile Thr Val Thr Leu Lys Lys Phe Pro Leu Asp Thr
                165                 170                 175

Leu Ile Pro Asp Gly Lys Arg Ile Ile Trp Asp Ser Arg Lys Gly Phe
            180                 185                 190

Ile Ile Ser Asn Ala Thr Tyr Lys Glu Ile Gly Leu Leu Thr Cys Glu
        195                 200                 205

Ala Thr Val Asn Gly His Leu Tyr Lys Thr Asn Tyr Leu Thr His Arg
    210                 215                 220

Gln Thr Asn Thr Ile Ile Asp Val Gln Ile Ser Thr Pro Arg Pro Val
225                 230                 235                 240
```

```
Lys Leu Leu Arg Gly His Thr Leu Val Leu Asn Cys Thr Ala Thr Thr
                245                 250                 255

Pro Leu Asn Thr Arg Val Gln Met Thr Trp Ser Tyr Pro Asp Glu Lys
            260                 265                 270

Asn Lys Arg Ala Ser Val Arg Arg Arg Ile Asp Gln Ser Asn Ser His
        275                 280                 285

Ala Asn Ile Phe Tyr Ser Val Leu Thr Ile Asp Lys Met Gln Asn Lys
    290                 295                 300

Asp Lys Gly Leu Tyr Thr Cys Arg Val Arg Ser Gly Pro Ser Phe Lys
305                 310                 315                 320

Ser Val Asn Thr Ser Val His Ile Tyr Asp Lys Ala Phe Ile Thr Val
                325                 330                 335

Lys His Arg Lys Gln Gln Val Leu Glu Thr Val Ala Gly Lys Arg Ser
            340                 345                 350

Tyr Arg Leu Ser Met Lys Val Lys Ala Phe Pro Ser Pro Glu Val Val
        355                 360                 365

Trp Leu Lys Asp Gly Leu Pro Ala Thr Glu Lys Ser Ala Arg Tyr Leu
    370                 375                 380

Thr Arg Gly Tyr Ser Leu Ile Ile Lys Asp Val Thr Glu Glu Asp Ala
385                 390                 395                 400

Gly Asn Tyr Thr Ile Leu Leu Ser Ile Lys Gln Ser Asn Val Phe Lys
                405                 410                 415

Asn Leu Thr Ala Thr Leu Ile Val Asn Val Lys Pro Gln Ile Tyr Glu
            420                 425                 430

Lys Ala Val Ser Ser Phe Pro Asp Pro Ala Leu Tyr Pro Leu Gly Ser
        435                 440                 445

Arg Gln Ile Leu Thr Cys Thr Ala Tyr Gly Ile Pro Gln Pro Thr Ile
    450                 455                 460

Lys Trp Phe Trp His Pro Cys Asn His Asn His Ser Glu Ala Arg Cys
465                 470                 475                 480

Asp Phe Cys Ser Asn Asn Glu Glu Ser Phe Ile Leu Asp Ala Asp Ser
                485                 490                 495
```

```
Asn Met Gly Asn Arg Ile Glu Ser Ile Thr Gln Arg Met Ala Ile Ile
                500                 505                 510

Glu Gly Lys Asn Lys Met Ala Ser Thr Leu Val Val Ala Asp Ser Arg
            515                 520                 525

Ile Ser Gly Ile Tyr Ile Cys Ile Ala Ser Asn Lys Val Gly Thr Val
    530                 535                 540

Gly Arg Asn Ile Ser Phe Tyr Ile Thr Asp Val Pro Asn Gly Phe His
545                 550                 555                 560

Val Asn Leu Glu Lys Met Pro Thr Glu Gly Glu Asp Leu Lys Leu Ser
                565                 570                 575

Cys Thr Val Asn Lys Phe Leu Tyr Arg Asp Val Thr Trp Ile Leu Leu
            580                 585                 590

Arg Thr Val Asn Asn Arg Thr Met His Tyr Ser Ile Ser Lys Gln Lys
        595                 600                 605

Met Ala Ile Thr Lys Glu His Ser Ile Thr Leu Asn Leu Thr Ile Met
    610                 615                 620

Asn Val Ser Leu Gln Asp Ser Gly Thr Tyr Ala Cys Arg Ala Arg Asn
625                 630                 635                 640

Val Tyr Thr Gly Glu Glu Ile Leu Gln Lys Lys Glu Ile Thr Ile Arg
                645                 650                 655

Gly Glu His Cys Asn Lys Lys Ala Val Phe Ser Arg Ile Ser Lys Phe
            660                 665                 670

Lys Ser Thr Arg Asn Asp Cys Thr Thr Gln Ser Asn Val Lys His
        675                 680                 685
```